# EUROPEAN PATENT SPECIFICATION

(11) **EP 1 893 210 B1**
(45) Date of publication and mention of the grant of the patent: **02.10.2019**
(21) Application number: 06771588.8
(22) Date of filing: 26.05.2006
(51) Int. Cl.: A61K 31/495, C07D 295/15, A61P 35/00, C12Q 1/37, G01N 33/574

(54) **SELECTIVE APOPTOTIC INDUCTION IN CANCER CELLS INCLUDING ACTIVATION OF PROCASPASE-3**
SELEKTIVE APOPTOTISCHE INDUKTION IN KREBSZELLEN MIT AKTIVIERUNG VON PROCASPASE-3
INDUCTION APOPTOTIQUE SELECTIVE DANS DES CELLULES CANCEREUSES INCLUANT UNE ACTIVATION DE LA PROCASPASE-3

(30) Priority: 26.05.2005 US 684807 P; 28.03.2006 US 743878 P; 25.05.2006 US 420425
(43) Date of publication of application: 05.03.2008
(60) Divisional application: 13166145.6
(73) Proprietor: THE BOARD OF TRUSTEES OF THE UNIVERSITY OF ILLINOIS, Urbana, IL 61801 (US)
(72) Inventor: HERGENROTHER, Paul J., Champaign, Illinois 61822 (US); PUTT, Karson S., Urbana, Illinois 61801 (US); CHEN, Grace W., Walnut Creek, CA 94598 (US); PEARSON, Jennifer M., Bloomington, Illinois 61704 (US)
(74) Representative: Fairbairn, Angus Chisholm
(86) International application number: PCT/US2006/020910
(87) International publication number: WO 2006/128173

(56) References cited:
- WO-A1-02/083114
- BUCKLEY C D ET AL: "RGD peptides induce apoptosis by direct caspase-3 activation" NATURE, NATURE PUBLISHING GROUP, LONDON, GB LNKD- DOI:10.1038/17409, vol. 397, 11 February 1999 (1999-02-11), pages 534-539, XP003000981 ISSN: 0028-0836
- SINGH AJITA V ET AL: "Sulforaphane induces caspase-mediated apoptosis in cultured PC-3 human prostate cancer cells and retards growth of PC-3 xenografts in vivo." CARCINOGENESIS (OXFORD), vol. 25, no. 1, January 2004 (2004-01), pages 83-90, XP002591973 ISSN: 0143-3334
- PUTT K. ET AL.: 'Small-molecule activation of procaspase-3 to caspase-3 as a personalized anticancer strategy' NATURE CHEMICAL BIOLOGY vol. 2, no. 10, 2006, pages 543 - 550, XP003013055
- DATABASE REGISTRY [Online] CHEMICAL ABSTRACTS SERVICE, COLUMBUS, OHIO, US; 19 January 2001 (2001-01-19), retrieved from STN Database accession no. RN 315183-21-2
- DATABASE REGISTRY [Online] CHEMICAL ABSTRACTS SERVICE, COLUMBUS, OHIO, US; 19 January 2001 (2001-01-19), retrieved from STN Database accession no. RN 315183-08-5

### SEQUENCE LISTING

<110> The Board of Trustees of the University of Illinois
<120> Selective Apoptotic Induction in Cancer Cells Including Activation of Procaspase-3
<130> EPP98656
<140> PCT/US2006/020910 (EP 06771588.8)
   <141> 2006-05-26
<150> US 60/684,807
   <151> 2005-05-26
<150> US 60/743,878
   <151> 2006-03-28
<150> US 11/420,425
   <151> 2006-05-25
<160> 27
<170> PatentIn version 3.3
<210> 1
   <211> 834
   <212> DNA
   <213> Homo sapiens
<220>
   <221> CDS
   <222> (1)..(834)
   <223> Procaspase-3; with amino acid DDD wild-type safety catch sequence (ACCESSION Number NM_004346)
<400> 1
   atg gag aac act gaa aac tca gtg gat tca aaa tcc att aaa aat ttg        48
   Met Glu Asn Thr Glu Asn Ser Val Asp Ser Lys Ser Ile Lys Asn Leu
   1               5                   10                  15
   gaa cca aag atc ata cat gga agc gaa tca atg gac tct gga ata tcc        96
   Glu Pro Lys Ile Ile His Gly Ser Glu Ser Met Asp Ser Gly Ile Ser
               20                  25                  30
   ctg gac aac agt tat aaa atg gat tat cct gag atg ggt tta tgt ata       144
   Leu Asp Asn Ser Tyr Lys Met Asp Tyr Pro Glu Met Gly Leu Cys Ile
           35                  40                  45
   ata att aat aat aag aat ttt cat aaa agc act gga atg aca tct cgg       192
   Ile Ile Asn Asn Lys Asn Phe His Lys Ser Thr Gly Met Thr Ser Arg
       50                  55                  60
   tct ggt aca gat gtc gat gca gca aac ctc agg gaa aca ttc aga aac       240
   Ser Gly Thr Asp Val Asp Ala Ala Asn Leu Arg Glu Thr Phe Arg Asn
   65                  70                  75                  80
   ttg aaa tat gaa gtc agg aat aaa aat gat ctt aca cgt gaa gaa att       288
   Leu Lys Tyr Glu Val Arg Asn Lys Asn Asp Leu Thr Arg Glu Glu Ile
                   85                  90                  95
   gtg gaa ttg atg cgt gat gtt tct aaa gaa gat cac agc aaa agg agc      336
   Val Glu Leu Met Arg Asp Val Ser Lys Glu Asp His Ser Lys Arg Ser
               100                 105                 110
   agt ttt gtt tgt gtg ctt ctg agc cat ggt gaa gaa gga ata att ttt      384
   Ser Phe Val Cys Val Leu Leu Ser His Gly Glu Glu Gly Ile Ile Phe
           115                 120                 125
   gga aca aat gga cct gtt gac ctg aaa aaa ata aca aac ttt ttc aga      432
   Gly Thr Asn Gly Pro Val Asp Leu Lys Lys Ile Thr Asn Phe Phe Arg
       130                 135                 140
   ggg gat cgt tgt aga agt cta act gga aaa ccc aaa ctt ttc att att      480
   Gly Asp Arg Cys Arg Ser Leu Thr Gly Lys Pro Lys Leu Phe Ile Ile
   145                 150                 155                 160
   cag gcc tgc cgt ggt aca gaa ctg gac tgt ggc att gag aca gac agt      528
   Gln Ala Cys Arg Gly Thr Glu Leu Asp Cys Gly Ile Glu Thr Asp Ser
                   165                 170                 175
   ggt gtt gat gat gac atg gcg tgt cat aaa ata cca gtg gag gcc gac      576
   Gly Val Asp Asp Asp Met Ala Cys His Lys Ile Pro Val Glu Ala Asp
               180                 185                 190
   ttc ttg tat gca tac tcc aca gca cct ggt tat tat tct tgg cga aat      624
   Phe Leu Tyr Ala Tyr Ser Thr Ala Pro Gly Tyr Tyr Ser Trp Arg Asn
           195                 200                 205
   tca aag gat ggc tcc tgg ttc atc cag tcg ctt tgt gcc atg ctg aaa      672
   Ser Lys Asp Gly Ser Trp Phe Ile Gln Ser Leu Cys Ala Met Leu Lys
       210                 215                 220
   cag tat gcc gac aag ctt gaa ttt atg cac att ctt acc cgg gtt aac      720
   Gln Tyr Ala Asp Lys Leu Glu Phe Met His Ile Leu Thr Arg Val Asn
   225                 230                 235                 240
   cga aag gtg gca aca gaa ttt gag tcc ttt tcc ttt gac gct act ttt      768
   Arg Lys Val Ala Thr Glu Phe Glu Ser Phe Ser Phe Asp Ala Thr Phe
                   245                 250                 255
   cat gca aag aaa cag att cca tgt att gtt tcc atg ctc aca aaa gaa      816
   His Ala Lys Lys Gln Ile Pro Cys Ile Val Ser Met Leu Thr Lys Glu
               260                 265                 270
   ctc tat ttt tat cac taa                                              834
   Leu Tyr Phe Tyr His
           275
<210> 2
   <211> 277
   <212> PRT
   <213> Homo sapiens
<400> 2
   Met Glu Asn Thr Glu Asn Ser Val Asp Ser Lys Ser Ile Lys Asn Leu
   1               5                   10                  15
   Glu Pro Lys Ile Ile His Gly Ser Glu Ser Met Asp Ser Gly Ile Ser
               20                  25                  30
   Leu Asp Asn Ser Tyr Lys Met Asp Tyr Pro Glu Met Gly Leu Cys Ile
           35                  40                  45
   Ile Ile Asn Asn Lys Asn Phe His Lys Ser Thr Gly Met Thr Ser Arg
       50                  55                  60
   Ser Gly Thr Asp Val Asp Ala Ala Asn Leu Arg Glu Thr Phe Arg Asn
   65                  70                  75                  80
   Leu Lys Tyr Glu Val Arg Asn Lys Asn Asp Leu Thr Arg Glu Glu Ile
                   85                  90                  95
   Val Glu Leu Met Arg Asp Val Ser Lys Glu Asp His Ser Lys Arg Ser
               100                 105                 110
   Ser Phe Val Cys Val Leu Leu Ser His Gly Glu Glu Gly Ile Ile Phe
           115                 120                 125
   Gly Thr Asn Gly Pro Val Asp Leu Lys Lys Ile Thr Asn Phe Phe Arg
       130                 135                 140
   Gly Asp Arg Cys Arg Ser Leu Thr Gly Lys Pro Lys Leu Phe Ile Ile
   145                 150                 155                 160
   Gln Ala Cys Arg Gly Thr Glu Leu Asp Cys Gly Ile Glu Thr Asp Ser
                   165                 170                 175
   Gly Val Asp Asp Asp Met Ala Cys His Lys Ile Pro Val Glu Ala Asp
               180                 185                 190
   Phe Leu Tyr Ala Tyr Ser Thr Ala Pro Gly Tyr Tyr Ser Trp Arg Asn
           195                 200                 205
   Ser Lys Asp Gly Ser Trp Phe Ile Gln Ser Leu Cys Ala Met Leu Lys
       210                 215                 220
   Gln Tyr Ala Asp Lys Leu Glu Phe Met His Ile Leu Thr Arg Val Asn
   225                 230                 235                 240
   Arg Lys Val Ala Thr Glu Phe Glu Ser Phe Ser Phe Asp Ala Thr Phe
                   245                 250                 255
   His Ala Lys Lys Gln Ile Pro Cys Ile Val Ser Met Leu Thr Lys Glu
               260                 265                 270
   Leu Tyr Phe Tyr His
           275
<210> 3
   <211> 1326
   <212> DNA
   <213> Homo sapiens
<220>
   <221> CDS
   <222> (50)..(880)
   <223> Procaspase-3; with amino acid DDD to ADD safety catch mutant sequence
<220>
   <221> misc_feature
   <222> (934)..(934)
   <223> n is a, c, g, t or u
<220>
   <221> misc_feature
   <222> (939)..(939)
   <223> n is a, c, g, t or u
<220>
   <221> misc_feature
   <222> (954)..(954)
   <223> n is a, c, g, t or u
<220>
   <221> misc_feature
   <222> (982)..(982)
   <223> n is a, c, g, t or u
<220>
   <221> misc_feature
   <222> (988)..(988)
   <223> n is a, c, g, t or u
<220>
   <221> misc_feature
   <222> (1000)..(1000)
   <223> n is a, c, g, t or u
<220>
   <221> misc_feature
   <222> (1024)..(1024)
   <223> n is a, c, g, t or u
<220>
   <221> misc_feature
   <222> (1036)..(1036)
   <223> n is a, c, g, t or u
<220>
   <221> misc_feature
   <222> (1045)..(1045)
   <223> n is a, c, g, t or u
<220>
   <221> misc_feature
   <222> (1051)..(1051)
   <223> n is a, c, g, t or u
<220>
   <221> misc_feature
   <222> (1053)..(1053)
   <223> n is a, c, g, t or u
<220>
   <221> misc_feature
   <222> (1063)..(1063)
   <223> n is a, c, g, t or u
<220>
   <221> misc_feature
   <222> (1071)..(1071)
   <223> n is a, c, g, t or u
<220>
   <221> misc_feature
   <222> (1075)..(1075)
   <223> n is a, c, g, t or u
<220>
   <221> misc_feature
   <222> (1079)..(1079)
   <223> n is a, c, g, t or u
<220>
   <221> misc_feature
   <222> (1086)..(1086)
   <223> n is a, c, g, t or u
<220>
   <221> misc_feature
   <222> (1093)..(1093)
   <223> n is a, c, g, t or u
<220>
   <221> misc_feature
   <222> (1095)..(1095)
   <223> n is a, c, g, t or u
<220>
   <221> misc_feature
   <222> (1100)..(1100)
   <223> n is a, c, g, t or u
<220>
   <221> misc_feature
   <222> (1104)..(1104)
   <223> n is a, c, g, t or u
<220>
   <221> misc_feature
   <222> (1106)..(1106)
   <223> n is a, c, g, t or u
<220>
   <221> misc_feature
   <222> (1125)..(1125)
   <223> n is a, c, g, t or u
<220>
   <221> misc_feature
   <222> (1140)..(1140)
   <223> n is a, c, g, t or u
<220>
   <221> misc_feature
   <222> (1146)..(1146)
   <223> n is a, c, g, t or u
<220>
   <221> misc_feature
   <222> (1149)..(1150)
   <223> n is a, c, g, t or u
<220>
   <221> misc_feature
   <222> (1155)..(1155)
   <223> n is a, c, g, t or u
<220>
   <221> misc_feature
   <222> (1157)..(1157)
   <223> n is a, c, g, t or u
<220>
   <221> misc_feature
   <222> (1159)..(1159)
   <223> n is a, c, g, t or u
<220>
   <221> misc_feature
   <222> (1190)..(1190)
   <223> n is a, c, g, t or u
<220>
   <221> misc_feature
   <222> (1199)..(1200)
   <223> n is a, c, g, t or u
<220>
   <221> misc_feature
   <222> (1209)..(1209)
   <223> n is a, c, g, t or u
<220>
   <221> misc_feature
   <222> (1212)..(1212)
   <223> n is a, c, g, t or u
<220>
   <221> misc_feature
   <222> (1223)..(1224)
   <223> n is a, c, g, t or u
<220>
   <221> misc_feature
   <222> (1233)..(1233)
   <223> n is a, c, g, t or u
<220>
   <221> misc_feature
   <222> (1235)..(1235)
   <223> n is a, c, g, t or u
<220>
   <221> misc_feature
   <222> (1238)..(1238)
   <223> n is a, c, g, t or u
<220>
   <221> misc_feature
   <222> (1241)..(1241)
   <223> n is a, c, g, t or u
<220>
   <221> misc_feature
   <222> (1246)..(1246)
   <223> n is a, c, g, t or u
<220>
   <221> misc_feature
   <222> (1256)..(1256)
   <223> n is a, c, g, t or u
<220>
   <221> misc_feature
   <222> (1259)..(1259)
   <223> n is a, c, g, t or u
<220>
   <221> misc_feature
   <222> (1262)..(1262)
   <223> n is a, c, g, t or u
<220>
   <221> misc_feature
   <222> (1273)..(1273)
   <223> n is a, c, g, t or u
<220>
   <221> misc_feature
   <222> (1276)..(1276)
   <223> n is a, c, g, t or u
<220>
   <221> misc_feature
   <222> (1282)..(1282)
   <223> n is a, c, g, t or u
<220>
   <221> misc_feature
   <222> (1285)..(1285)
   <223> n is a, c, g, t or u
<220>
   <221> misc_feature
   <222> (1288)..(1288)
   <223> n is a, c, g, t or u
<220>
   <221> misc_feature
   <222> (1294)..(1294)
   <223> n is a, c, g, t or u
<220>
   <221> misc_feature
   <222> (1299)..(1299)
   <223> n is a, c, g, t or u
<220>
   <221> misc_feature
   <222> (1302)..(1302)
   <223> n is a, c, g, t or u
<220>
   <221> misc_feature
   <222> (1306)..(1306)
   <223> n is a, c, g, t or u
<400> 3
   gtacattccc tctgaataat tttgtttact ttaagaagga gatatacat atg gag aac      58
                                                         Met Glu Asn
                                                         1
   act gaa aac tca gtg gat tca aaa tcc att aaa aat ttg gaa cca aag       106
   Thr Glu Asn Ser Val Asp Ser Lys Ser Ile Lys Asn Leu Glu Pro Lys
       5                   10                  15
   atc ata cat gga agc gaa tca atg gac tct gga ata tcc ctg gac aac       154
   Ile Ile His Gly Ser Glu Ser Met Asp Ser Gly Ile Ser Leu Asp Asn
   20                  25                  30                  35
   agt tat aaa atg gat tat cct gag atg ggt tta tgt ata ata att aat       202
   Ser Tyr Lys Met Asp Tyr Pro Glu Met Gly Leu Cys Ile Ile Ile Asn
                   40                  45                  50
   aat aag aat ttt cat aaa agc act gga atg aca tct cgg tct ggt aca       250
   Asn Lys Asn Phe His Lys Ser Thr Gly Met Thr Ser Arg Ser Gly Thr
               55                  60                  65
   gat gtc gat gca gca aac ctc agg gaa aca ttc aga aac ttg aaa tat       298
   Asp Val Asp Ala Ala Asn Leu Arg Glu Thr Phe Arg Asn Leu Lys Tyr
           70                  75                  80
   gaa gtc agg aat aaa aat gat ctt aca cgt gaa gaa att gtg gaa ttg       346
   Glu Val Arg Asn Lys Asn Asp Leu Thr Arg Glu Glu Ile Val Glu Leu
       85                  90                  95
   atg cgt gat gtt tct aaa gaa gat cac agc aaa agg agc agt ttt gtt       394
   Met Arg Asp Val Ser Lys Glu Asp His Ser Lys Arg Ser Ser Phe Val
   100                 105                 110                 115
   tgt gtg ctt ctg agc cat ggt gaa gaa gga ata att ttt gga aca aat       442
   Cys Val Leu Leu Ser His Gly Glu Glu Gly Ile Ile Phe Gly Thr Asn
                   120                 125                 130
   gga cct gtt gac ctg aaa aaa ata aca aac ttt ttc aga ggg gat cgt       490
   Gly Pro Val Asp Leu Lys Lys Ile Thr Asn Phe Phe Arg Gly Asp Arg
               135                 140                 145
   tgt aga agt cta act gga aaa ccc aaa ctt ttc att att cag gcc tgc       538
   Cys Arg Ser Leu Thr Gly Lys Pro Lys Leu Phe Ile Ile Gln Ala Cys
           150                 155                 160
   cgt ggt aca gaa ctg gac tgt ggc att gag aca gac agt ggt gtt gcg       586
   Arg Gly Thr Glu Leu Asp Cys Gly Ile Glu Thr Asp Ser Gly Val Ala
       165                 170                 175
   gat gac atg gcg tgt cat aaa ata cca gtg gag gcc gac ttc ttg tat       634
   Asp Asp Met Ala Cys His Lys Ile Pro Val Glu Ala Asp Phe Leu Tyr
   180                 185                 190                 195
   gca tac tcc aca gca cct ggt tat tat tct tgg cga aat tca aag gat      682
   Ala Tyr Ser Thr Ala Pro Gly Tyr Tyr Ser Trp Arg Asn Ser Lys Asp
                   200                 205                 210
   ggc tcc tgg ttc atc cag tcg ctt tgt gcc atg ctg aaa cag tat gcc      730
   Gly Ser Trp Phe Ile Gln Ser Leu Cys Ala Met Leu Lys Gln Tyr Ala
               215                 220                 225
   gac aag ctt gaa ttt atg cac att ctt acc cgg gtt aac cga aag gtg      778
   Asp Lys Leu Glu Phe Met His Ile Leu Thr Arg Val Asn Arg Lys Val
           230                 235                 240
   gca aca gaa ttt gag tcc ttt tcc ttt gac gct act ttt cat gca aag      826
   Ala Thr Glu Phe Glu Ser Phe Ser Phe Asp Ala Thr Phe His Ala Lys
       245                 250                 255
   aaa cag att cca tgt att gtt tcc atg ctc aca aaa gaa ctc tat ttt      874
   Lys Gln Ile Pro Cys Ile Val Ser Met Leu Thr Lys Glu Leu Tyr Phe
   260                 265                 270                 275
   tat cac ctcgagcacc accaccacca ccactgagat ccggctgcta caagccgaaa      930
   Tyr His
   gganctgant tggctgctgc cccnctgacc atactacata ccccctgggg cnctaacngg    990
   tctggggggn tttttgctga aggagacttt tccngatggc aatggnaccc cctgnccgcc    1050
   ntnacccggc ggngggggtt ncccnacgng acctancttg ccngncctan cccncncttc    1110
   cttttccttc ttccnccgtt ccggttcccn cagctnaann ggggncntng gtccattggc    1170
   ttcgcccccc caaactgttn gggggtccnn ggccccccna angtttccct tanngacccc    1230
   ttnanggnct ntcccngacc cccccnccnt tnttttaagg tcnccncccg gnaanggnta    1290
   aatnccttna ancccntggg ttgggggccc tttttt                              1326
<210> 4
   <211> 277
   <212> PRT
   <213> Homo sapiens
<400> 4
   Met Glu Asn Thr Glu Asn Ser Val Asp Ser Lys Ser Ile Lys Asn Leu
   1               5                   10                  15
   Glu Pro Lys Ile Ile His Gly Ser Glu Ser Met Asp Ser Gly Ile Ser
               20                  25                  30
   Leu Asp Asn Ser Tyr Lys Met Asp Tyr Pro Glu Met Gly Leu Cys Ile
           35                  40                  45
   Ile Ile Asn Asn Lys Asn Phe His Lys Ser Thr Gly Met Thr Ser Arg
       50                  55                  60
   Ser Gly Thr Asp Val Asp Ala Ala Asn Leu Arg Glu Thr Phe Arg Asn
   65                  70                  75                  80
   Leu Lys Tyr Glu Val Arg Asn Lys Asn Asp Leu Thr Arg Glu Glu Ile
                   85                  90                  95
   Val Glu Leu Met Arg Asp Val Ser Lys Glu Asp His Ser Lys Arg Ser
               100                 105                 110
   Ser Phe Val Cys Val Leu Leu Ser His Gly Glu Glu Gly Ile Ile Phe
           115                 120                 125
   Gly Thr Asn Gly Pro Val Asp Leu Lys Lys Ile Thr Asn Phe Phe Arg
       130                 135                 140
   Gly Asp Arg Cys Arg Ser Leu Thr Gly Lys Pro Lys Leu Phe Ile Ile
   145                 150                 155                 160
   Gln Ala Cys Arg Gly Thr Glu Leu Asp Cys Gly Ile Glu Thr Asp Ser
                   165                 170                 175
   Gly Val Ala Asp Asp Met Ala Cys His Lys Ile Pro Val Glu Ala Asp
               180                 185                 190
   Phe Leu Tyr Ala Tyr Ser Thr Ala Pro Gly Tyr Tyr Ser Trp Arg Asn
           195                 200                 205
   Ser Lys Asp Gly Ser Trp Phe Ile Gln Ser Leu Cys Ala Met Leu Lys
       210                 215                 220
   Gln Tyr Ala Asp Lys Leu Glu Phe Met His Ile Leu Thr Arg Val Asn
   225                 230                 235                 240
   Arg Lys Val Ala Thr Glu Phe Glu Ser Phe Ser Phe Asp Ala Thr Phe
                   245                 250                 255
   His Ala Lys Lys Gln Ile Pro Cys Ile Val Ser Met Leu Thr Lys Glu
               260                 265                 270
   Leu Tyr Phe Tyr His
           275
<210> 5
   <211> 1398
   <212> DNA
   <213> Homo sapiens
<220>
   <221> CDS
   <222> (51)..(881)
   <223> Procaspase-3; with amino acid DDD to DAD safety catch mutant sequence
<220>
   <221> misc_feature
   <222> (923)..(923)
   <223> n is a, c, g, t or u
<220>
   <221> misc_feature
   <222> (933)..(933)
   <223> n is a, c, g, t or u
<220>
   <221> misc_feature
   <222> (942)..(942)
   <223> n is a, c, g, t or u
<220>
   <221> misc_feature
   <222> (963)..(963)
   <223> n is a, c, g, t or u
<220>
   <221> misc_feature
   <222> (978)..(978)
   <223> n is a, c, g, t or u
<220>
   <221> misc_feature
   <222> (991)..(991)
   <223> n is a, c, g, t or u
<220>
   <221> misc_feature
   <222> (994)..(994)
   <223> n is a, c, g, t or u
<220>
   <221> misc_feature
   <222> (1022)..(1022)
   <223> n is a, c, g, t or u
<220>
   <221> misc_feature
   <222> (1031)..(1031)
   <223> n is a, c, g, t or u
<220>
   <221> misc_feature
   <222> (1033)..(1033)
   <223> n is a, c, g, t or u
<220>
   <221> misc_feature
   <222> (1045)..(1045)
   <223> n is a, c, g, t or u
<220>
   <221> misc_feature
   <222> (1047)..(1047)
   <223> n is a, c, g, t or u
<220>
   <221> misc_feature
   <222> (1061)..(1061)
   <223> n is a, c, g, t or u
<220>
   <221> misc_feature
   <222> (1067)..(1067)
   <223> n is a, c, g, t or u
<220>
   <221> misc_feature
   <222> (1069)..(1069)
   <223> n is a, c, g, t or u
<220>
   <221> misc_feature
   <222> (1076)..(1076)
   <223> n is a, c, g, t or u
<220>
   <221> misc_feature
   <222> (1091)..(1091)
   <223> n is a, c, g, t or u
<220>
   <221> misc_feature
   <222> (1095)..(1095)
   <223> n is a, c, g, t or u
<220>
   <221> misc_feature
   <222> (1098)..(1098)
   <223> n is a, c, g, t or u
<220>
   <221> misc_feature
   <222> (1102)..(1102)
   <223> n is a, c, g, t or u
<220>
   <221> misc_feature
   <222> (1105)..(1105)
   <223> n is a, c, g, t or u
<220>
   <221> misc_feature
   <222> (1112)..(1112)
   <223> n is a, c, g, t or u
<220>
   <221> misc_feature
   <222> (1116)..(1116)
   <223> n is a, c, g, t or u
<220>
   <221> misc_feature
   <222> (1122)..(1123)
   <223> n is a, c, g, t or u
<220>
   <221> misc_feature
   <222> (1126)..(1126)
   <223> n is a, c, g, t or u
<220>
   <221> misc_feature
   <222> (1129)..(1129)
   <223> n is a, c, g, t or u
<220>
   <221> misc_feature
   <222> (1134)..(1134)
   <223> n is a, c, g, t or u
<220>
   <221> misc_feature
   <222> (1139)..(1139)
   <223> n is a, c, g, t or u
<220>
   <221> misc_feature
   <222> (1144)..(1144)
   <223> n is a, c, g, t or u
<220>
   <221> misc_feature
   <222> (1148)..(1148)
   <223> n is a, c, g, t or u
<220>
   <221> misc_feature
   <222> (1150)..(1150)
   <223> n is a, c, g, t or u
<220>
   <221> misc_feature
   <222> (1158)..(1158)
   <223> n is a, c, g, t or u
<220>
   <221> misc_feature
   <222> (1175)..(1175)
   <223> n is a, c, g, t or u
<220>
   <221> misc_feature
   <222> (1180)..(1180)
   <223> n is a, c, g, t or u
<220>
   <221> misc_feature
   <222> (1190)..(1190)
   <223> n is a, c, g, t or u
<220>
   <221> misc_feature
   <222> (1196)..(1196)
   <223> n is a, c, g, t or u
<220>
   <221> misc_feature
   <222> (1200)..(1200)
   <223> n is a, c, g, t or u
<220>
   <221> misc_feature
   <222> (1202)..(1202)
   <223> n is a, c, g, t or u
<220>
   <221> misc_feature
   <222> (1206)..(1207)
   <223> n is a, c, g, t or u
<220>
   <221> misc_feature
   <222> (1215)..(1215)
   <223> n is a, c, g, t or u
<220>
   <221> misc_feature
   <222> (1224)..(1224)
   <223> n is a, c, g, t or u
<220>
   <221> misc_feature
   <222> (1231)..(1231)
   <223> n is a, c, g, t or u
<220>
   <221> misc_feature
   <222> (1238)..(1238)
   <223> n is a, c, g, t or u
<220>
   <221> misc_feature
   <222> (1245)..(1245)
   <223> n is a, c, g, t or u
<220>
   <221> misc_feature
   <222> (1249)..(1249)
   <223> n is a, c, g, t or u
<220>
   <221> misc_feature
   <222> (1273)..(1273)
   <223> n is a, c, g, t or u
<220>
   <221> misc_feature
   <222> (1276)..(1276)
   <223> n is a, c, g, t or u
<220>
   <221> misc_feature
   <222> (1284)..(1284)
   <223> n is a, c, g, t or u
<220>
   <221> misc_feature
   <222> (1299)..(1299)
   <223> n is a, c, g, t or u
<220>
   <221> misc_feature
   <222> (1309)..(1309)
   <223> n is a, c, g, t or u
<220>
   <221> misc_feature
   <222> (1312)..(1312)
   <223> n is a, c, g, t or u
<220>
   <221> misc_feature
   <222> (1319)..(1319)
   <223> n is a, c, g, t or u
<220>
   <221> misc_feature
   <222> (1321)..(1321)
   <223> n is a, c, g, t or u
<220>
   <221> misc_feature
   <222> (1325)..(1325)
   <223> n is a, c, g, t or u
<220>
   <221> misc_feature
   <222> (1328)..(1328)
   <223> n is a, c, g, t or u
<220>
   <221> misc_feature
   <222> (1335)..(1335)
   <223> n is a, c, g, t or u
<220>
   <221> misc_feature
   <222> (1337)..(1337)
   <223> n is a, c, g, t or u
<220>
   <221> misc_feature
   <222> (1345)..(1345)
   <223> n is a, c, g, t or u
<220>
   <221> misc_feature
   <222> (1348)..(1348)
   <223> n is a, c, g, t or u
<220>
   <221> misc_feature
   <222> (1355)..(1355)
   <223> n is a, c, g, t or u
<220>
   <221> misc_feature
   <222> (1358)..(1358)
   <223> n is a, c, g, t or u
<220>
   <221> misc_feature
   <222> (1364)..(1364)
   <223> n is a, c, g, t or u
<220>
   <221> misc_feature
   <222> (1369)..(1369)
   <223> n is a, c, g, t or u
<220>
   <221> misc_feature
   <222> (1376)..(1376)
   <223> n is a, c, g, t or u
<220>
   <221> misc_feature
   <222> (1379)..(1379)
   <223> n is a, c, g, t or u
<220>
   <221> misc_feature
   <222> (1387)..(1387)
   <223> n is a, c, g, t or u
<220>
   <221> misc_feature
   <222> (1390)..(1390)
   <223> n is a, c, g, t or u
<400> 5
   cgtacattcc ctctgaataa ttttgtttac tttaagaagg agatatacat atg gag       56
                                                          Met Glu
                                                          1
   aac act gaa aac tca gtg gat tca aaa tcc att aaa aat ttg gaa cca      104
   Asn Thr Glu Asn Ser Val Asp Ser Lys Ser Ile Lys Asn Leu Glu Pro
           5                   10                  15
   aag atc ata cat gga agc gaa tca atg gac tct gga ata tcc ctg gac      152
   Lys Ile Ile His Gly Ser Glu Ser Met Asp Ser Gly Ile Ser Leu Asp
       20                  25                  30
   aac agt tat aaa atg gat tat cct gag atg ggt tta tgt ata ata att      200
   Asn Ser Tyr Lys Met Asp Tyr Pro Glu Met Gly Leu Cys Ile Ile Ile
   35                  40                  45                  50
   aat aat aag aat ttt cat aaa agc act gga atg aca tct cgg tct ggt      248
   Asn Asn Lys Asn Phe His Lys Ser Thr Gly Met Thr Ser Arg Ser Gly
                   55                  60                  65
   aca gat gtc gat gca gca aac ctc agg gaa aca ttc aga aac ttg aaa      296
   Thr Asp Val Asp Ala Ala Asn Leu Arg Glu Thr Phe Arg Asn Leu Lys
               70                  75                  80
   tat gaa gtc agg aat aaa aat gat ctt aca cgt gaa gaa att gtg gaa      344
   Tyr Glu Val Arg Asn Lys Asn Asp Leu Thr Arg Glu Glu Ile Val Glu
           85                  90                  95
   ttg atg cgt gat gtt tct aaa gaa gat cac agc aaa agg agc agt ttt      392
   Leu Met Arg Asp Val Ser Lys Glu Asp His Ser Lys Arg Ser Ser Phe
       100                 105                 110
   gtt tgt gtg ctt ctg agc cat ggt gaa gaa gga ata att ttt gga aca      440
   Val Cys Val Leu Leu Ser His Gly Glu Glu Gly Ile Ile Phe Gly Thr
   115                 120                 125                 130
   aat gga cct gtt gac ctg aaa aaa ata aca aac ttt ttc aga ggg gat 488
   Asn Gly Pro Val Asp Leu Lys Lys Ile Thr Asn Phe Phe Arg Gly Asp
   135 140 145
   cgt tgt aga agt cta act gga aaa ccc aaa ctt ttc att att cag gcc 536
   Arg Cys Arg Ser Leu Thr Gly Lys Pro Lys Leu Phe Ile Ile Gln Ala
   150 155 160
   tgc cgt ggt aca gaa ctg gac tgt ggc att gag aca gac agt ggt gtt 584
   Cys Arg Gly Thr Glu Leu Asp Cys Gly Ile Glu Thr Asp Ser Gly Val
   165 170 175
   gat gct gac atg gcg tgt cat aaa ata cca gtg gag gcc gac ttc ttg 632
   Asp Ala Asp Met Ala Cys His Lys Ile Pro Val Glu Ala Asp Phe Leu
   180 185 190
   tat gca tac tcc aca gca cct ggt tat tat tct tgg cga aat tca aag 680
   Tyr Ala Tyr Ser Thr Ala Pro Gly Tyr Tyr Ser Trp Arg Asn Ser Lys
   195 200 205 210
   gat ggc tcc tgg ttc atc cag tcg ctt tgt gcc atg ctg aaa cag tat 728
   Asp Gly Ser Trp Phe Ile Gln Ser Leu Cys Ala Met Leu Lys Gln Tyr
   215 220 225
   gcc gac aag ctt gaa ttt atg cac att ctt acc cgg gtt aac cga aag 776
   Ala Asp Lys Leu Glu Phe Met His Ile Leu Thr Arg Val Asn Arg Lys
   230 235 240
   gtg gca aca gaa ttt gag tcc ttt tcc ttt gac gct act ttt cat gca 824
   Val Ala Thr Glu Phe Glu Ser Phe Ser Phe Asp Ala Thr Phe His Ala
   245 250 255
   aag aaa cag att cca tgt att gtt tcc atg ctc aca aaa gaa ctc tat 872
   Lys Lys Gln Ile Pro Cys Ile Val Ser Met Leu Thr Lys Glu Leu Tyr
   260 265 270
   ttt tat cac ctcgagcacc accaccacca ccactgagat ccggctgcta 921
   Phe Tyr His
   275
   cnaagcccga angaagctga nttggctgct gcccccgctg ancaataact agcatanccc 981
   cttggggccn ctnaacgggt ctggaggggt ttttgctgaa nggggacctn tntccggatt 1041
   ggcnanggga ccccccctgn accgcncntt aaccncgcgg ggggggggtn cccncanggg 1101
   nccnctacct ngccngcccc nnacnccncc ccnttccntt ctncctncnt tcccccngtt 1161
   cccgggtttc ccgnagccna aacgggggnc ccttnggtnc nattnngctt tccncccccc 1221
   ccnaaacttn taggggnggt cccngggncc ccccgaaagg tttccccttg cnggnccccc 1281
   ttnaaggact ttcccagnaa cccccccncg nccctttntn aggntcnccc cccngnaaag 1341
   ggtnaantcc gttnaanccc ttnggctngg gggcnccntt tttttnttnc ccccccc 1398
<210> 6
   <211> 277
   <212> PRT
   <213> Homo sapiens
<400> 6
   Met Glu Asn Thr Glu Asn Ser Val Asp Ser Lys Ser Ile Lys Asn Leu
   1               5                   10                  15
   Glu Pro Lys Ile Ile His Gly Ser Glu Ser Met Asp Ser Gly Ile Ser
               20                  25                  30
   Leu Asp Asn Ser Tyr Lys Met Asp Tyr Pro Glu Met Gly Leu Cys Ile
           35                  40                  45
   Ile Ile Asn Asn Lys Asn Phe His Lys Ser Thr Gly Met Thr Ser Arg
       50                  55                  60
   Ser Gly Thr Asp Val Asp Ala Ala Asn Leu Arg Glu Thr Phe Arg Asn
   65                  70                  75                  80
   Leu Lys Tyr Glu Val Arg Asn Lys Asn Asp Leu Thr Arg Glu Glu Ile
                   85                  90                  95
   Val Glu Leu Met Arg Asp Val Ser Lys Glu Asp His Ser Lys Arg Ser
               100                 105                 110
   Ser Phe Val Cys Val Leu Leu Ser His Gly Glu Glu Gly Ile Ile Phe
           115                 120                 125
   Gly Thr Asn Gly Pro Val Asp Leu Lys Lys Ile Thr Asn Phe Phe Arg
       130                 135                 140
   Gly Asp Arg Cys Arg Ser Leu Thr Gly Lys Pro Lys Leu Phe Ile Ile
   145                 150                 155                 160
   Gln Ala Cys Arg Gly Thr Glu Leu Asp Cys Gly Ile Glu Thr Asp Ser
                   165                 170                 175
   Gly Val Asp Ala Asp Met Ala Cys His Lys Ile Pro Val Glu Ala Asp
               180                 185                 190
   Phe Leu Tyr Ala Tyr Ser Thr Ala Pro Gly Tyr Tyr Ser Trp Arg Asn
           195                 200                 205
   Ser Lys Asp Gly Ser Trp Phe Ile Gln Ser Leu Cys Ala Met Leu Lys
       210                 215                 220
   Gln Tyr Ala Asp Lys Leu Glu Phe Met His Ile Leu Thr Arg Val Asn
   225                 230                 235                 240
   Arg Lys Val Ala Thr Glu Phe Glu Ser Phe Ser Phe Asp Ala Thr Phe
                   245                 250                 255
   His Ala Lys Lys Gln Ile Pro Cys Ile Val Ser Met Leu Thr Lys Glu
               260                 265                 270
   Leu Tyr Phe Tyr His
           275
<210> 7
   <211> 1316
   <212> DNA
   <213> Homo sapiens
<220>
   <221> CDS
   <222> (51)..(881)
   <223> Procaspase-3; with amino acid DDD to DAD safety catch mutant sequence
<220>
   <221> misc_feature
   <222> (914)..(914)
   <223> n is a, c, g, t or u
<220>
   <221> misc_feature
   <222> (942)..(942)
   <223> n is a, c, g, t or u
<220>
   <221> misc_feature
   <222> (958)..(958)
   <223> n is a, c, g, t or u
<220>
   <221> misc_feature
   <222> (982)..(982)
   <223> n is a, c, g, t or u
<220>
   <221> misc_feature
   <222> (986)..(986)
   <223> n is a, c, g, t or u
<220>
   <221> misc_feature
   <222> (988)..(988)
   <223> n is a, c, g, t or u
<220>
   <221> misc_feature
   <222> (1006)..(1006)
   <223> n is a, c, g, t or u
<220>
   <221> misc_feature
   <222> (1026)..(1026)
   <223> n is a, c, g, t or u
<220>
   <221> misc_feature
   <222> (1032)..(1032)
   <223> n is a, c, g, t or u
<220>
   <221> misc_feature
   <222> (1038)..(1038)
   <223> n is a, c, g, t or u
<220>
   <221> misc_feature
   <222> (1040)..(1041)
   <223> n is a, c, g, t or u
<220>
   <221> misc_feature
   <222> (1051)..(1051)
   <223> n is a, c, g, t or u
<220>
   <221> misc_feature
   <222> (1062)..(1062)
   <223> n is a, c, g, t or u
<220>
   <221> misc_feature
   <222> (1067)..(1067)
   <223> n is a, c, g, t or u
<220>
   <221> misc_feature
   <222> (1073)..(1073)
   <223> n is a, c, g, t or u
<220>
   <221> misc_feature
   <222> (1097)..(1097)
   <223> n is a, c, g, t or u
<220>
   <221> misc_feature
   <222> (1101)..(1101)
   <223> n is a, c, g, t or u
<220>
   <221> misc_feature
   <222> (1106)..(1106)
   <223> n is a, c, g, t or u
<220>
   <221> misc_feature
   <222> (1108)..(1108)
   <223> n is a, c, g, t or u
<220>
   <221> misc_feature
   <222> (1114)..(1114)
   <223> n is a, c, g, t or u
<220>
   <221> misc_feature
   <222> (1118)..(1118)
   <223> n is a, c, g, t or u
<220>
   <221> misc_feature
   <222> (1123)..(1123)
   <223> n is a, c, g, t or u
<220>
   <221> misc_feature
   <222> (1129)..(1129)
   <223> n is a, c, g, t or u
<220>
   <221> misc_feature
   <222> (1134)..(1134)
   <223> n is a, c, g, t or u
<220>
   <221> misc_feature
   <222> (1148)..(1149)
   <223> n is a, c, g, t or u
<220>
   <221> misc_feature
   <222> (1151)..(1151)
   <223> n is a, c, g, t or u
<220>
   <221> misc_feature
   <222> (1154)..(1154)
   <223> n is a, c, g, t or u
<220>
   <221> misc_feature
   <222> (1156)..(1157)
   <223> n is a, c, g, t or u
<220>
   <221> misc_feature
   <222> (1173)..(1173)
   <223> n is a, c, g, t or u
<220>
   <221> misc_feature
   <222> (1193)..(1193)
   <223> n is a, c, g, t or u
<220>
   <221> misc_feature
   <222> (1200)..(1200)
   <223> n is a, c, g, t or u
<220>
   <221> misc_feature
   <222> (1206)..(1206)
   <223> n is a, c, g, t or u
<220>
   <221> misc_feature
   <222> (1215)..(1215)
   <223> n is a, c, g, t or u
<220>
   <221> misc_feature
   <222> (1228)..(1230)
   <223> n is a, c, g, t or u
<220>
   <221> misc_feature
   <222> (1232)..(1232)
   <223> n is a, c, g, t or u
<220>
   <221> misc_feature
   <222> (1247)..(1247)
   <223> n is a, c, g, t or u
<220>
   <221> misc_feature
   <222> (1250)..(1250)
   <223> n is a, c, g, t or u
<220>
   <221> misc_feature
   <222> (1261)..(1261)
   <223> n is a, c, g, t or u
<220>
   <221> misc_feature
   <222> (1263)..(1264)
   <223> n is a, c, g, t or u
<220>
   <221> misc_feature
   <222> (1272)..(1272)
   <223> n is a, c, g, t or u
<220>
   <221> misc_feature
   <222> (1275)..(1275)
   <223> n is a, c, g, t or u
<220>
   <221> misc_feature
   <222> (1277)..(1277)
   <223> n is a, c, g, t or u
<220>
   <221> misc_feature
   <222> (1286)..(1286)
   <223> n is a, c, g, t or u
<220>
   <221> misc_feature
   <222> (1289)..(1289)
   <223> n is a, c, g, t or u
<220>
   <221> misc_feature
   <222> (1291)..(1292)
   <223> n is a, c, g, t or u
<220>
   <221> misc_feature
   <222> (1294)..(1294)
   <223> n is a, c, g, t or u
<220>
   <221> misc_feature
   <222> (1299)..(1299)
   <223> n is a, c, g, t or u
<220>
   <221> misc_feature
   <222> (1301)..(1301)
   <223> n is a, c, g, t or u
<220>
   <221> misc_feature
   <222> (1304)..(1304)
   <223> n is a, c, g, t or u
<220>
   <221> misc_feature
   <222> (1307)..(1307)
   <223> n is a, c, g, t or u
<220>
   <221> misc_feature
   <222> (1316)..(1316)
   <223> n is a, c, g, t or u
<400> 7
   cgtacattcc ctctgaataa ttttgtttac tttaagaagg agatatacat atg gag        56
                                                          Met Glu
                                                          1
   aac act gaa aac tca gtg gat tca aaa tcc att aaa aat ttg gaa cca      104
   Asn Thr Glu Asn Ser Val Asp Ser Lys Ser Ile Lys Asn Leu Glu Pro
           5                   10                  15
   aag atc ata cat gga agc gaa tca atg gac tct gga ata tcc ctg gac      152
   Lys Ile Ile His Gly Ser Glu Ser Met Asp Ser Gly Ile Ser Leu Asp
       20                  25                  30
   aac agt tat aaa atg gat tat cct gag atg ggt tta tgt ata ata att      200
   Asn Ser Tyr Lys Met Asp Tyr Pro Glu Met Gly Leu Cys Ile Ile Ile
   35                  40                  45                  50
   aat aat aag aat ttt cat aaa agc act gga atg aca tct cgg tct ggt      248
   Asn Asn Lys Asn Phe His Lys Ser Thr Gly Met Thr Ser Arg Ser Gly
                   55                  60                  65
   aca gat gtc gat gca gca aac ctc agg gaa aca ttc aga aac ttg aaa      296
   Thr Asp Val Asp Ala Ala Asn Leu Arg Glu Thr Phe Arg Asn Leu Lys
               70                  75                  80
   tat gaa gtc agg aat aaa aat gat ctt aca cgt gaa gaa att gtg gaa      344
   Tyr Glu Val Arg Asn Lys Asn Asp Leu Thr Arg Glu Glu Ile Val Glu
           85                  90                  95
   ttg atg cgt gat gtt tct aaa gaa gat cac agc aaa agg agc agt ttt      392
   Leu Met Arg Asp Val Ser Lys Glu Asp His Ser Lys Arg Ser Ser Phe
       100                 105                 110
   gtt tgt gtg ctt ctg agc cat ggt gaa gaa gga ata att ttt gga aca      440
   Val Cys Val Leu Leu Ser His Gly Glu Glu Gly Ile Ile Phe Gly Thr
   115                 120                 125                 130
   aat gga cct gtt gac ctg aaa aaa ata aca aac ttt ttc aga ggg gat      488
   Asn Gly Pro Val Asp Leu Lys Lys Ile Thr Asn Phe Phe Arg Gly Asp
               135                 140                 145
   cgt tgt aga agt cta act gga aaa ccc aaa ctt ttc att att cag gcc      536
   Arg Cys Arg Ser Leu Thr Gly Lys Pro Lys Leu Phe Ile Ile Gln Ala
           150                 155                 160
   tgc cgt ggt aca gaa ctg gac tgt ggc att gag aca gac agt ggt gtt      584
   Cys Arg Gly Thr Glu Leu Asp Cys Gly Ile Glu Thr Asp Ser Gly Val
       165                 170                 175
   gat gat gcc atg gcg tgt cat aaa ata cca gtg gag gcc gac ttc ttg      632
   Asp Asp Ala Met Ala Cys His Lys Ile Pro Val Glu Ala Asp Phe Leu
       180                 185                 190
   tat gca tac tcc aca gca cct ggt tat tat tct tgg cga aat tca aag      680
   Tyr Ala Tyr Ser Thr Ala Pro Gly Tyr Tyr Ser Trp Arg Asn Ser Lys
   195                 200                 205                 210
   gat ggc tcc tgg ttc atc cag tcg ctt tgt gcc atg ctg aaa cag tat      728
   Asp Gly Ser Trp Phe Ile Gln Ser Leu Cys Ala Met Leu Lys Gln Tyr
                   215                 220                 225
   gcc gac aag ctt gaa ttt atg cac att ctt acc cgg gtt aac cga aag      776
   Ala Asp Lys Leu Glu Phe Met His Ile Leu Thr Arg Val Asn Arg Lys
               230                 235                 240
   gtg gca aca gaa ttt gag tcc ttt tcc ttt gac gct act ttt cat gca      824
   Val Ala Thr Glu Phe Glu Ser Phe Ser Phe Asp Ala Thr Phe His Ala
           245                 250                 255
   aag aaa cag att cca tgt att gtt tcc atg ctc aca aaa gaa ctc tat      872
   Lys Lys Gln Ile Pro Cys Ile Val Ser Met Leu Thr Lys Glu Leu Tyr
       260                 265                 270
   ttt tat cac ctcgagcacc cccccaccac cactgagatc cgnctgctac              921
   Phe Tyr His
   275
   aaagcccgaa aggaagctga nttggctgct gcccccnctg accataccta gcatacccct     981
   ngggncncta acgggtctgg ggggnttttg ctgaaggggg acctnttccg natggcnann   1041
   ggaccccccn gtaccgccct naaccngcgg gngggggttc ccccacggac cctacntgcn   1101
   gcccnanccc ccncttncct tntcctcntt ccnccgtccg gttcccnnan ctnanngggc    1161
   ccttggtcca tnggcttcgc cccccccaaa cnttagggng gtccngggcc cccnaaaggt   1221
   tcccttnnng ncccctttaa ggactntcnc ggaccccccn cnnctttttt nagntncctc   1281
   cctgnaangn ntnaaatncn ttnaanccct gggcn                              1316
<210> 8
   <211> 277
   <212> PRT
   <213> Homo sapiens
<400> 8
   Met Glu Asn Thr Glu Asn Ser Val Asp Ser Lys Ser Ile Lys Asn Leu
   1               5                   10                  15
   Glu Pro Lys Ile Ile His Gly Ser Glu Ser Met Asp Ser Gly Ile Ser
           20                  25                  30
   Leu Asp Asn Ser Tyr Lys Met Asp Tyr Pro Glu Met Gly Leu Cys Ile
       35                  40                  45
   Ile Ile Asn Asn Lys Asn Phe His Lys Ser Thr Gly Met Thr Ser Arg
       50                  55                  60
   Ser Gly Thr Asp Val Asp Ala Ala Asn Leu Arg Glu Thr Phe Arg Asn
   65                  70                  75                  80
   Leu Lys Tyr Glu Val Arg Asn Lys Asn Asp Leu Thr Arg Glu Glu Ile
                   85                  90                  95
   Val Glu Leu Met Arg Asp Val Ser Lys Glu Asp His Ser Lys Arg Ser
               100                 105                 110
   Ser Phe Val Cys Val Leu Leu Ser His Gly Glu Glu Gly Ile Ile Phe
           115                 120                 125
   Gly Thr Asn Gly Pro Val Asp Leu Lys Lys Ile Thr Asn Phe Phe Arg
       130                 135                 140
   Gly Asp Arg Cys Arg Ser Leu Thr Gly Lys Pro Lys Leu Phe Ile Ile
   145                 150                 155                 160
   Gln Ala Cys Arg Gly Thr Glu Leu Asp Cys Gly Ile Glu Thr Asp Ser
                   165                 170                 175
   Gly Val Asp Asp Ala Met Ala Cys His Lys Ile Pro Val Glu Ala Asp
               180                 185                 190
   Phe Leu Tyr Ala Tyr Ser Thr Ala Pro Gly Tyr Tyr Ser Trp Arg Asn
           195                 200                 205
   Ser Lys Asp Gly Ser Trp Phe Ile Gln Ser Leu Cys Ala Met Leu Lys
       210                 215                 220
   Gln Tyr Ala Asp Lys Leu Glu Phe Met His Ile Leu Thr Arg Val Asn
   225                 230                 235                 240
   Arg Lys Val Ala Thr Glu Phe Glu Ser Phe Ser Phe Asp Ala Thr Phe
                   245                 250                 255
   His Ala Lys Lys Gln Ile Pro Cys Ile Val Ser Met Leu Thr Lys Glu
                   260                 265                 270
   Leu Tyr Phe Tyr His
           275
<210> 9
   <211> 277
   <212> PRT
   <213> Homo sapiens
<220>
   <221> MISC_FEATURE
   <222> (1)..(277)
   <223> Procaspase-3; with amino acid DDD wild-type safety catch sequence (ACCESSION Number NM_004346)
<400> 9
   Met Glu Asn Thr Glu Asn Ser Val Asp Ser Lys Ser Ile Lys Asn Leu
   1               5                   10                  15
   Glu Pro Lys Ile Ile His Gly Ser Glu Ser Met Asp Ser Gly Ile Ser
               20                  25                  30
   Leu Asp Asn Ser Tyr Lys Met Asp Tyr Pro Glu Met Gly Leu Cys Ile
           35                  40                  45
   Ile Ile Asn Asn Lys Asn Phe His Lys Ser Thr Gly Met Thr Ser Arg
       50                  55                  60
   Ser Gly Thr Asp Val Asp Ala Ala Asn Leu Arg Glu Thr Phe Arg Asn
   65                  70                  75                  80
   Leu Lys Tyr Glu Val Arg Asn Lys Asn Asp Leu Thr Arg Glu Glu Ile
                   85                  90                  95
   Val Glu Leu Met Arg Asp Val Ser Lys Glu Asp His Ser Lys Arg Ser
               100                 105                 110
   Ser Phe Val Cys Val Leu Leu Ser His Gly Glu Glu Gly Ile Ile Phe
           115                 120                 125
   Gly Thr Asn Gly Pro Val Asp Leu Lys Lys Ile Thr Asn Phe Phe Arg
       130                 135                 140
   Gly Asp Arg Cys Arg Ser Leu Thr Gly Lys Pro Lys Leu Phe Ile Ile
   145                 150                 155                 160
   Gln Ala Cys Arg Gly Thr Glu Leu Asp Cys Gly Ile Glu Thr Asp Ser
                   165                 170                 175
   Gly Val Asp Asp Asp Met Ala Cys His Lys Ile Pro Val Glu Ala Asp
           180                 185                 190
   Phe Leu Tyr Ala Tyr Ser Thr Ala Pro Gly Tyr Tyr Ser Trp Arg Asn
       195                 200                 205
   Ser Lys Asp Gly Ser Trp Phe Ile Gln Ser Leu Cys Ala Met Leu Lys
       210                 215                 220
   Gln Tyr Ala Asp Lys Leu Glu Phe Met His Ile Leu Thr Arg Val Asn
   225                 230                 235                 240
   Arg Lys Val Ala Thr Glu Phe Glu Ser Phe Ser Phe Asp Ala Thr Phe
               245                 250                 255
   His Ala Lys Lys Gln Ile Pro Cys Ile Val Ser Met Leu Thr Lys Glu
           260                 265                 270
   Leu Tyr Phe Tyr His
       275
<210> 10
   <211> 277
   <212> PRT
   <213> Homo sapiens
<220>
   <221> MISC_FEATURE
   <222> (1)..(277)
   <223> Procaspase-3; with amino acid DDD to ADD safety catch mutant sequence
<400> 10
   Met Glu Asn Thr Glu Asn Ser Val Asp Ser Lys Ser Ile Lys Asn Leu
   1               5                   10                  15
   Glu Pro Lys Ile Ile His Gly Ser Glu Ser Met Asp Ser Gly Ile Ser
               20                  25                  30
   Leu Asp Asn Ser Tyr Lys Met Asp Tyr Pro Glu Met Gly Leu Cys Ile
           35                  40                  45
   Ile Ile Asn Asn Lys Asn Phe His Lys Ser Thr Gly Met Thr Ser Arg
       50                  55                  60
   Ser Gly Thr Asp Val Asp Ala Ala Asn Leu Arg Glu Thr Phe Arg Asn
   65                  70                  75                  80
   Leu Lys Tyr Glu Val Arg Asn Lys Asn Asp Leu Thr Arg Glu Glu Ile
                   85                  90                  95
   Val Glu Leu Met Arg Asp Val Ser Lys Glu Asp His Ser Lys Arg Ser
               100                 105                 110
   Ser Phe Val Cys Val Leu Leu Ser His Gly Glu Glu Gly Ile Ile Phe
           115                 120                 125
   Gly Thr Asn Gly Pro Val Asp Leu Lys Lys Ile Thr Asn Phe Phe Arg
       130                 135                 140
   Gly Asp Arg Cys Arg Ser Leu Thr Gly Lys Pro Lys Leu Phe Ile Ile
   145                 150                 155                 160
   Gln Ala Cys Arg Gly Thr Glu Leu Asp Cys Gly Ile Glu Thr Asp Ser
                   165                 170                 175
   Gly Val Ala Asp Asp Met Ala Cys His Lys Ile Pro Val Glu Ala Asp
               180                 185                 190
   Phe Leu Tyr Ala Tyr Ser Thr Ala Pro Gly Tyr Tyr Ser Trp Arg Asn
           195                 200                 205
   Ser Lys Asp Gly Ser Trp Phe Ile Gln Ser Leu Cys Ala Met Leu Lys
       210                 215                 220
   Gln Tyr Ala Asp Lys Leu Glu Phe Met His Ile Leu Thr Arg Val Asn
   225                 230                 235                 240
   Arg Lys Val Ala Thr Glu Phe Glu Ser Phe Ser Phe Asp Ala Thr Phe
                   245                 250                 255
   His Ala Lys Lys Gln Ile Pro Cys Ile Val Ser Met Leu Thr Lys Glu
               260                 265                 270
   Leu Tyr Phe Tyr His
           275
<210> 11
   <211> 277
   <212> PRT
   <213> Homo sapiens
<220>
   <221> MISC_FEATURE
   <222> (1)..(277)
   <223> Procaspase-3; with amino acid DDD to DAD safety catch mutant sequence
<400> 11
   Met Glu Asn Thr Glu Asn Ser Val Asp Ser Lys Ser Ile Lys Asn Leu
   1               5                   10                  15
   Glu Pro Lys Ile Ile His Gly Ser Glu Ser Met Asp Ser Gly Ile Ser
               20                  25                  30
   Leu Asp Asn Ser Tyr Lys Met Asp Tyr Pro Glu Met Gly Leu Cys Ile
           35                  40                  45
   Ile Ile Asn Asn Lys Asn Phe His Lys Ser Thr Gly Met Thr Ser Arg
       50                  55                  60
   Ser Gly Thr Asp Val Asp Ala Ala Asn Leu Arg Glu Thr Phe Arg Asn
   65                  70                  75                  80
   Leu Lys Tyr Glu Val Arg Asn Lys Asn Asp Leu Thr Arg Glu Glu Ile
                   85                  90                  95
   Val Glu Leu Met Arg Asp Val Ser Lys Glu Asp His Ser Lys Arg Ser
               100                 105                 110
   Ser Phe Val Cys Val Leu Leu Ser His Gly Glu Glu Gly Ile Ile Phe
           115                 120                 125
   Gly Thr Asn Gly Pro Val Asp Leu Lys Lys Ile Thr Asn Phe Phe Arg
       130                 135                 140
   Gly Asp Arg Cys Arg Ser Leu Thr Gly Lys Pro Lys Leu Phe Ile Ile
   145                 150                 155                 160
   Gln Ala Cys Arg Gly Thr Glu Leu Asp Cys Gly Ile Glu Thr Asp Ser
                   165                 170                 175
   Gly Val Asp Ala Asp Met Ala Cys His Lys Ile Pro Val Glu Ala Asp
               180                 185                 190
   Phe Leu Tyr Ala Tyr Ser Thr Ala Pro Gly Tyr Tyr Ser Trp Arg Asn
           195                 200                 205
   Ser Lys Asp Gly Ser Trp Phe Ile Gln Ser Leu Cys Ala Met Leu Lys
       210                 215                 220
   Gln Tyr Ala Asp Lys Leu Glu Phe Met His Ile Leu Thr Arg Val Asn
   225                 230                 235                 240
   Arg Lys Val Ala Thr Glu Phe Glu Ser Phe Ser Phe Asp Ala Thr Phe
                   245                 250                 255
   His Xaa Lys Lys Gln Ile Pro Cys Ile Val Ser Met Leu Thr Lys Glu
               260                 265                 270
   Leu Tyr Phe Tyr His
           275
<210> 12
   <211> 277
   <212> PRT
   <213> Homo sapiens
<220>
   <221> MISC_FEATURE
   <222> (1)..(277)
   <223> Procaspase-3; with amino acid DDD to DAD safety catch mutant sequence
<400> 12
   Met Glu Asn Thr Glu Asn Ser Val Asp Ser Lys Ser Ile Lys Asn Leu
   1               5                   10                  15
   Glu Pro Lys Ile Ile His Gly Ser Glu Ser Met Asp Ser Gly Ile Ser
               20                  25                  30
   Leu Asp Asn Ser Tyr Lys Met Asp Tyr Pro Glu Met Gly Leu Cys Ile
           35                  40                  45
   Ile Ile Asn Asn Lys Asn Phe His Lys Ser Thr Gly Met Thr Ser Arg
       50                  55                  60
   Ser Gly Thr Asp Val Asp Ala Ala Asn Leu Arg Glu Thr Phe Arg Asn
   65                  70                  75                  80
   Leu Lys Tyr Glu Val Arg Asn Lys Asn Asp Leu Thr Arg Glu Glu Ile
                   85                  90                  95
   Val Glu Leu Met Arg Asp Val Ser Lys Glu Asp His Ser Lys Arg Ser
               100                 105                 110
   Ser Phe Val Cys Val Leu Leu Ser His Gly Glu Glu Gly Ile Ile Phe
           115                 120                 125
   Gly Thr Asn Gly Pro Val Asp Leu Lys Lys Ile Thr Asn Phe Phe Arg
       130                 135                 140
   Gly Asp Arg Cys Arg Ser Leu Thr Gly Lys Pro Lys Leu Phe Ile Ile
   145                 150                 155                 160
   Gln Ala Cys Arg Gly Thr Glu Leu Asp Cys Gly Ile Glu Thr Asp Ser
                   165                 170                 175
   Gly Val Asp Asp Ala Met Ala Cys His Lys Ile Pro Val Glu Ala Asp
               180                 185                 190
   Phe Leu Tyr Ala Tyr Ser Thr Ala Pro Gly Tyr Tyr Ser Trp Arg Asn
           195                 200                 205
   Ser Lys Asp Gly Ser Trp Phe Ile Gln Ser Leu Cys Ala Met Leu Lys
       210                 215                 220
   Gln Tyr Ala Asp Lys Leu Glu Phe Met His Ile Leu Thr Arg Val Asn
   225                 230                 235                 240
   Arg Lys Val Ala Thr Glu Phe Glu Ser Phe Ser Phe Asp Ala Thr Phe
                   245                 250                 255
   His Ala Lys Lys Gln Ile Pro Cys Ile Val Ser Met Leu Thr Lys Glu
               260                 265                 270
   Leu Tyr Phe Tyr His
           275
<210> 13
   <211> 45
   <212> DNA
   <213> Artificial
<220>
   <223> Synthetic oligo
<400> 13
   gacagacagt ggtgttgcgg atgacatggc gtgtcataaa atacc 45
<210> 14
   <211> 45
   <212> DNA
   <213> Artificial
<220>
   <223> Synthetic oligo
<400> 14
   gacagacagt ggtgttgatg ctgacatggc gtgtcataaa atacc 45
<210> 15
   <211> 45
   <212> DNA
   <213> Artificial
<220>
   <223> Synthetic oligo
<400> 15
   gacagacagt ggtgttgatg atgccatggc gtgtcataaa atacc 45
<210> 16
   <211> 912
   <212> DNA
   <213> Homo sapiens
<220>
   <221> CDS
   <222> (1)..(912)
   <223> Procaspase-7 with amino acid DTD wild-type safety catch sequence
   (Accession Number NM_001227)
<400> 16
   atg gca gat gat cag ggc tgt att gaa gag cag ggg gtt gag gat tca      48
   Met Ala Asp Asp Gln Gly Cys Ile Glu Glu Gln Gly Val Glu Asp Ser
   1               5                   10                  15
   gca aat gaa gat tca gtg gat gct aag cca gac cgg tcc tcg ttt gta      96
   Ala Asn Glu Asp Ser Val Asp Ala Lys Pro Asp Arg Ser Ser Phe Val
               20                  25                  30
   ccg tcc ctc ttc agt aag aag aag aaa aat gtc acc atg cga tcc atc     144
   Pro Ser Leu Phe Ser Lys Lys Lys Lys Asn Val Thr Met Arg Ser Ile
           35                  40                  45
   aag acc acc cgg gac cga gtg cct aca tat cag tac aac atg aat ttt     192
   Lys Thr Thr Arg Asp Arg Val Pro Thr Tyr Gln Tyr Asn Met Asn Phe
       50                  55                  60
   gaa aag ctg ggc aaa tgc atc ata ata aac aac aag aac ttt gat aaa     240
   Glu Lys Leu Gly Lys Cys Ile Ile Ile Asn Asn Lys Asn Phe Asp Lys
   65                  70                  75                  80
   gtg aca ggt atg ggc gtt cga aac gga aca gac aaa gat gcc gag gcg     288
   Val Thr Gly Met Gly Val Arg Asn Gly Thr Asp Lys Asp Ala Glu Ala
                   85                  90                  95
   ctc ttc aag tgc ttc cga agc ctg ggt ttt gac gtg att gtc tat aat     336
   Leu Phe Lys Cys Phe Arg Ser Leu Gly Phe Asp Val Ile Val Tyr Asn
               100                 105                 110
   gac tgc tct tgt gcc aag atg caa gat ctg ctt aaa aaa gct tct gaa     384
   Asp Cys Ser Cys Ala Lys Met Gln Asp Leu Leu Lys Lys Ala Ser Glu
           115                 120                 125
   gag gac cat aca aat gcc gcc tgc ttc gcc tgc atc ctc tta agc cat     432
   Glu Asp His Thr Asn Ala Ala Cys Phe Ala Cys Ile Leu Leu Ser His
       130                 135                 140
   gga gaa gaa aat gta att tat ggg aaa gat ggt gtc aca cca ata aag     480
   Gly Glu Glu Asn Val Ile Tyr Gly Lys Asp Gly Val Thr Pro Ile Lys
   145                 150                 155                 160
   gat ttg aca gcc cac ttt agg ggg gat aga tgc aaa acc ctt tta gag     528
   Asp Leu Thr Ala His Phe Arg Gly Asp Arg Cys Lys Thr Leu Leu Glu
                   165                 170                 175
   aaa ccc aaa ctc ttc ttc att cag gct tgc cga ggg acc gag ctt gat      576
   Lys Pro Lys Leu Phe Phe Ile Gln Ala Cys Arg Gly Thr Glu Leu Asp
               180                 185                 190
   gat ggc atc cag gcc gac tcg ggg ccc atc aat gac aca gat gct aat      624
   Asp Gly Ile Gln Ala Asp Ser Gly Pro Ile Asn Asp Thr Asp Ala Asn
           195                 200                 205
   cct cga tac aag atc cca gtg gaa gct gac ttc ctc ttc gcc tat tcc      672
   Pro Arg Tyr Lys Ile Pro Val Glu Ala Asp Phe Leu Phe Ala Tyr Ser
       210                 215                 220
   acg gtt cca ggc tat tac tcg tgg agg agc cca gga aga ggc tcc tgg      720
   Thr Val Pro Gly Tyr Tyr Ser Trp Arg Ser Pro Gly Arg Gly Ser Trp
   225                 230                 235                 240
   ttt gtg caa gcc ctc tgc tcc atc ctg gag gag cac gga aaa gac ctg      768
   Phe Val Gln Ala Leu Cys Ser Ile Leu Glu Glu His Gly Lys Asp Leu
                   245                 250                 255
   gaa atc atg cag atc ctc acc agg gtg aat gac aga gtt gcc agg cac      816
   Glu Ile Met Gln Ile Leu Thr Arg Val Asn Asp Arg Val Ala Arg His
               260                 265                 270
   ttt gag tct cag tct gat gac cca cac ttc cat gag aag aag cag atc      864
   Phe Glu Ser Gln Ser Asp Asp Pro His Phe His Glu Lys Lys Gln Ile
           275                 280                 285
   ccc tgt gtg gtc tcc atg ctc acc aag gaa ctc tac ttc agt caa tag      912
   Pro Cys Val Val Ser Met Leu Thr Lys Glu Leu Tyr Phe Ser Gln
       290                 295                 300
<210> 17
   <211> 303
   <212> PRT
   <213> Homo sapiens
<400> 17
   Met Ala Asp Asp Gln Gly Cys Ile Glu Glu Gln Gly Val Glu Asp Ser
   1               5                   10                  15
   Ala Asn Glu Asp Ser Val Asp Ala Lys Pro Asp Arg Ser Ser Phe Val
               20                  25                  30
   Pro Ser Leu Phe Ser Lys Lys Lys Lys Asn Val Thr Met Arg Ser Ile
           35                  40                  45
   Lys Thr Thr Arg Asp Arg Val Pro Thr Tyr Gln Tyr Asn Met Asn Phe
       50                  55                  60
   Glu Lys Leu Gly Lys Cys Ile Ile Ile Asn Asn Lys Asn Phe Asp Lys
   65                  70                  75                  80
   Val Thr Gly Met Gly Val Arg Asn Gly Thr Asp Lys Asp Ala Glu Ala
                   85                  90                  95
   Leu Phe Lys Cys Phe Arg Ser Leu Gly Phe Asp Val Ile Val Tyr Asn
               100                 105                 110
   Asp Cys Ser Cys Ala Lys Met Gln Asp Leu Leu Lys Lys Ala Ser Glu
           115                 120                 125
   Glu Asp His Thr Asn Ala Ala Cys Phe Ala Cys Ile Leu Leu Ser His
       130                 135                 140
   Gly Glu Glu Asn Val Ile Tyr Gly Lys Asp Gly Val Thr Pro Ile Lys
   145                 150                 155                 160
   Asp Leu Thr Ala His Phe Arg Gly Asp Arg Cys Lys Thr Leu Leu Glu
                   165                 170                 175
   Lys Pro Lys Leu Phe Phe Ile Gln Ala Cys Arg Gly Thr Glu Leu Asp
               180                 185                 190
   Asp Gly Ile Gln Ala Asp Ser Gly Pro Ile Asn Asp Thr Asp Ala Asn
           195                 200                 205
   Pro Arg Tyr Lys Ile Pro Val Glu Ala Asp Phe Leu Phe Ala Tyr Ser
       210                 215                 220
   Thr Val Pro Gly Tyr Tyr Ser Trp Arg Ser Pro Gly Arg Gly Ser Trp
   225                 230                 235                 240
   Phe Val Gln Ala Leu Cys Ser Ile Leu Glu Glu His Gly Lys Asp Leu
                   245                 250                 255
   Glu Ile Met Gln Ile Leu Thr Arg Val Asn Asp Arg Val Ala Arg His
               260                 265                 270
   Phe Glu Ser Gln Ser Asp Asp Pro His Phe His Glu Lys Lys Gln Ile
           275                 280                 285
   Pro Cys Val Val Ser Met Leu Thr Lys Glu Leu Tyr Phe Ser Gln
       290                 295                 300
<210> 18
   <211> 912
   <212> DNA
   <213> Homo sapiens
<220>
   <221> CDS
   <222> (1)..(912)
   <223> Procaspase-7 DDD wild-type safety catch sequence
<400> 18
   atg gca gat gat cag ggc tgt att gaa gag cag ggg gtt gag gat tca        48
   Met Ala Asp Asp Gln Gly Cys Ile Glu Glu Gln Gly Val Glu Asp Ser
   1               5                   10                  15
   gca aat gaa gat tca gtg gat gct aag cca gac cgg tcc tcg ttt gta        96
   Ala Asn Glu Asp Ser Val Asp Ala Lys Pro Asp Arg Ser Ser Phe Val
               20                  25                  30
   ccg tcc ctc ttc agt aag aag aag aaa aat gtc acc atg cga tcc atc       144
   Pro Ser Leu Phe Ser Lys Lys Lys Lys Asn Val Thr Met Arg Ser Ile
           35                  40                  45
   aag acc acc cgg gac cga gtg cct aca tat cag tac aac atg aat ttt       192
   Lys Thr Thr Arg Asp Arg Val Pro Thr Tyr Gln Tyr Asn Met Asn Phe
       50                  55                  60
   gaa aag ctg ggc aaa tgc atc ata ata aac aac aag aac ttt gat aaa       240
   Glu Lys Leu Gly Lys Cys Ile Ile Ile Asn Asn Lys Asn Phe Asp Lys
   65                  70                  75                  80
   gtg aca ggt atg ggc gtt cga aac gga aca gac aaa gat gcc gag gcg       288
   Val Thr Gly Met Gly Val Arg Asn Gly Thr Asp Lys Asp Ala Glu Ala
                   85                  90                  95
   ctc ttc aag tgc ttc cga agc ctg ggt ttt gac gtg att gtc tat aat       336
   Leu Phe Lys Cys Phe Arg Ser Leu Gly Phe Asp Val Ile Val Tyr Asn
               100                 105                 110
   gac tgc tct tgt gcc aag atg caa gat ctg ctt aaa aaa gct tct gaa       384
   Asp Cys Ser Cys Ala Lys Met Gln Asp Leu Leu Lys Lys Ala Ser Glu
           115                 120                 125
   gag gac cat aca aat gcc gcc tgc ttc gcc tgc atc ctc tta agc cat       432
   Glu Asp His Thr Asn Ala Ala Cys Phe Ala Cys Ile Leu Leu Ser His
       130                 135                 140
   gga gaa gaa aat gta att tat ggg aaa gat ggt gtc aca cca ata aag       480
   Gly Glu Glu Asn Val Ile Tyr Gly Lys Asp Gly Val Thr Pro Ile Lys
   145                 150                 155                 160
   gat ttg aca gcc cac ttt agg ggg gat aga tgc aaa acc ctt tta gag       528
   Asp Leu Thr Ala His Phe Arg Gly Asp Arg Cys Lys Thr Leu Leu Glu
                   165                 170                 175
   aaa ccc aaa ctc ttc ttc att cag gct tgc cga ggg acc gag ctt gat       576
   Lys Pro Lys Leu Phe Phe Ile Gln Ala Cys Arg Gly Thr Glu Leu Asp
               180                 185                 190
   gat ggc atc cag gcc gac tcg ggg ccc atc aat gac gca gat gct aat       624
   Asp Gly Ile Gln Ala Asp Ser Gly Pro Ile Asn Asp Ala Asp Ala Asn
           195                 200                 205
   cct cga tac aag atc cca gtg gaa gct gac ttc ctc ttc gcc tat tcc       672
   Pro Arg Tyr Lys Ile Pro Val Glu Ala Asp Phe Leu Phe Ala Tyr Ser
       210                 215                 220
   acg gtt cca ggc tat tac tcg tgg agg agc cca gga aga ggc tcc tgg       720
   Thr Val Pro Gly Tyr Tyr Ser Trp Arg Ser Pro Gly Arg Gly Ser Trp
   225                 230                 235                 240
   ttt gtg caa gcc ctc tgc tcc atc ctg gag gag cac gga aaa gac ctg      768
   Phe Val Gln Ala Leu Cys Ser Ile Leu Glu Glu His Gly Lys Asp Leu
                   245                 250                 255
   gaa atc atg cag atc ctc acc agg gtg aat gac aga gtt gcc agg cac      816
   Glu Ile Met Gln Ile Leu Thr Arg Val Asn Asp Arg Val Ala Arg His
               260                 265                 270
   ttt gag tct cag tct gat gac cca cac ttc cat gag aag aag cag atc      864
   Phe Glu Ser Gln Ser Asp Asp Pro His Phe His Glu Lys Lys Gln Ile
           275                 280                 285
   ccc tgt gtg gtc tcc atg ctc acc aag gaa ctc tac ttc agt caa tag      912
   Pro Cys Val Val Ser Met Leu Thr Lys Glu Leu Tyr Phe Ser Gln
       290                 295                 300
<210> 19
   <211> 303
   <212> PRT
   <213> Homo sapiens
<400> 19
   Met Ala Asp Asp Gln Gly Cys Ile Glu Glu Gln Gly Val Glu Asp Ser
   1               5                   10                  15
   Ala Asn Glu Asp Ser Val Asp Ala Lys Pro Asp Arg Ser Ser Phe Val
               20                  25                  30
   Pro Ser Leu Phe Ser Lys Lys Lys Lys Asn Val Thr Met Arg Ser Ile
           35                  40                  45
   Lys Thr Thr Arg Asp Arg Val Pro Thr Tyr Gln Tyr Asn Met Asn Phe
       50                  55                  60
   Glu Lys Leu Gly Lys Cys Ile Ile Ile Asn Asn Lys Asn Phe Asp Lys
   65                  70                  75                  80
   Val Thr Gly Met Gly Val Arg Asn Gly Thr Asp Lys Asp Ala Glu Ala
                   85                  90                  95
   Leu Phe Lys Cys Phe Arg Ser Leu Gly Phe Asp Val Ile Val Tyr Asn
               100                 105                 110
   Asp Cys Ser Cys Ala Lys Met Gln Asp Leu Leu Lys Lys Ala Ser Glu
           115                 120                 125
   Glu Asp His Thr Asn Ala Ala Cys Phe Ala Cys Ile Leu Leu Ser His
       130                 135                 140
   Gly Glu Glu Asn Val Ile Tyr Gly Lys Asp Gly Val Thr Pro Ile Lys
   145                 150                 155                 160
   Asp Leu Thr Ala His Phe Arg Gly Asp Arg Cys Lys Thr Leu Leu Glu
                   165                 170                 175
   Lys Pro Lys Leu Phe Phe Ile Gln Ala Cys Arg Gly Thr Glu Leu Asp
               180                 185                 190
   Asp Gly Ile Gln Ala Asp Ser Gly Pro Ile Asn Asp Ala Asp Ala Asn
           195                 200                 205
   Pro Arg Tyr Lys Ile Pro Val Glu Ala Asp Phe Leu Phe Ala Tyr Ser
       210                 215                 220
   Thr Val Pro Gly Tyr Tyr Ser Trp Arg Ser Pro Gly Arg Gly Ser Trp
   225                 230                 235                 240
   Phe Val Gln Ala Leu Cys Ser Ile Leu Glu Glu His Gly Lys Asp Leu
                   245                 250                 255
   Glu Ile Met Gln Ile Leu Thr Arg Val Asn Asp Arg Val Ala Arg His
               260                 265                 270
   Phe Glu Ser Gln Ser Asp Asp Pro His Phe His Glu Lys Lys Gln Ile
           275                 280                 285
   Pro Cys Val Val Ser Met Leu Thr Lys Glu Leu Tyr Phe Ser Gln
       290                 295                 300
<210> 20
   <211> 936
   <212> DNA
   <213> Homo sapiens
<220>
   <221> CDS
   <222> (1)..(936)
   <223> Procaspase-7 DTD wild-type safety catch, active site C to A mutant sequence
<400> 20
   atg gca gat gat cag ggc tgt att gaa gag cag ggg gtt gag gat tca      48
   Met Ala Asp Asp Gln Gly Cys Ile Glu Glu Gln Gly Val Glu Asp Ser
   1               5                   10                  15
   gca aat gaa gat tca gtg gat gct aag cca gac cgg tcc tcg ttt gta      96
   Ala Asn Glu Asp Ser Val Asp Ala Lys Pro Asp Arg Ser Ser Phe Val
               20                  25                  30
   ccg tcc ctc ttc agt aag aag aag aaa aat gtc acc atg cga tcc atc     144
   Pro Ser Leu Phe Ser Lys Lys Lys Lys Asn Val Thr Met Arg Ser Ile
           35                  40                  45
   aag acc acc cgg gac cga gtg cct aca tat cag tac aac atg aat ttt 192
   Lys Thr Thr Arg Asp Arg Val Pro Thr Tyr Gln Tyr Asn Met Asn Phe
   50 55 60
   gaa aag ctg ggc aaa tgc atc ata ata aac aac aag aac ttt gat aaa 240
   Glu Lys Leu Gly Lys Cys Ile Ile Ile Asn Asn Lys Asn Phe Asp Lys
   65 70 75 80
   gtg aca ggt atg ggc gtt cga aac gga aca gac aaa gat gcc gag gcg 288
   Val Thr Gly Met Gly Val Arg Asn Gly Thr Asp Lys Asp Ala Glu Ala
   85 90 95
   ctc ttc aag tgc ttc cga agc ctg ggt ttt gac gtg att gtc tat aat 336
   Leu Phe Lys Cys Phe Arg Ser Leu Gly Phe Asp Val Ile Val Tyr Asn
   100 105 110
   gac tgc tct tgt gcc aag atg caa gat ctg ctt aaa aaa gct tct gaa 384
   Asp Cys Ser Cys Ala Lys Met Gln Asp Leu Leu Lys Lys Ala Ser Glu
   115 120 125
   gag gac cat aca aat gcc gcc tgc ttc gcc tgc atc ctc tta agc cat 432
   Glu Asp His Thr Asn Ala Ala Cys Phe Ala Cys Ile Leu Leu Ser His
   130 135 140
   gga gaa gaa aat gta att tat ggg aaa gat ggt gtc aca cca ata aag 480
   Gly Glu Glu Asn Val Ile Tyr Gly Lys Asp Gly Val Thr Pro Ile Lys
   145 150 155 160
   gat ttg aca gcc cac ttt agg ggg gat aga tgc aaa acc ctt tta gag 528
   Asp Leu Thr Ala His Phe Arg Gly Asp Arg Cys Lys Thr Leu Leu Glu
   165 170 175
   aaa ccc aaa ctc ttc ttc att cag gct gcc cga ggg acc gag ctt gat 576
   Lys Pro Lys Leu Phe Phe Ile Gln Ala Ala Arg Gly Thr Glu Leu Asp
   180 185 190
   gat ggc atc cag gcc gac tcg ggg ccc atc aat gac aca gat gct aat 624
   Asp Gly Ile Gln Ala Asp Ser Gly Pro Ile Asn Asp Thr Asp Ala Asn
   195 200 205
   cct cga tac aag atc cca gtg gaa gct gac ttc ctc ttc gcc tat tcc 672
   Pro Arg Tyr Lys Ile Pro Val Glu Ala Asp Phe Leu Phe Ala Tyr Ser
   210 215 220
   acg gtt cca ggc tat tac tcg tgg agg agc cca gga aga ggc tcc tgg 720
   Thr Val Pro Gly Tyr Tyr Ser Trp Arg Ser Pro Gly Arg Gly Ser Trp
   225 230 235 240
   ttt gtg caa gcc ctc tgc tcc atc ctg gag gag cac gga aaa gac ctg 768
   Phe Val Gln Ala Leu Cys Ser Ile Leu Glu Glu His Gly Lys Asp Leu
   245 250 255
   gaa atc atg cag atc ctc acc agg gtg aat gac aga gtt gcc agg cac 816
   Glu Ile Met Gln Ile Leu Thr Arg Val Asn Asp Arg Val Ala Arg His
   260 265 270
   ttt gag tct cag tct gat gac cca cac ttc cat gag aag aag cag atc 864
   Phe Glu Ser Gln Ser Asp Asp Pro His Phe His Glu Lys Lys Gln Ile
   275 280 285
   ccc tgt gtg gtc tcc atg ctc acc aag gaa ctc tac ttc agt caa ctc 912
   Pro Cys Val Val Ser Met Leu Thr Lys Glu Leu Tyr Phe Ser Gln Leu
            290                 295                 300
   gag cac cac cac cac cac cac tga                                       936
   Glu His His His His His His
   305                 310
<210> 21
   <211> 311
   <212> PRT
   <213> Homo sapiens
<400> 21
   Met Ala Asp Asp Gln Gly Cys Ile Glu Glu Gln Gly Val Glu Asp Ser
   1               5                   10                  15
   Ala Asn Glu Asp Ser Val Asp Ala Lys Pro Asp Arg Ser Ser Phe Val
               20                  25                  30
   Pro Ser Leu Phe Ser Lys Lys Lys Lys Asn Val Thr Met Arg Ser Ile
           35                  40                  45
   Lys Thr Thr Arg Asp Arg Val Pro Thr Tyr Gln Tyr Asn Met Asn Phe
       50                  55                  60
   Glu Lys Leu Gly Lys Cys Ile Ile Ile Asn Asn Lys Asn Phe Asp Lys
   65                  70                  75                  80
   Val Thr Gly Met Gly Val Arg Asn Gly Thr Asp Lys Asp Ala Glu Ala
                   85                  90                  95
   Leu Phe Lys Cys Phe Arg Ser Leu Gly Phe Asp Val Ile Val Tyr Asn
               100                 105                 110
   Asp Cys Ser Cys Ala Lys Met Gln Asp Leu Leu Lys Lys Ala Ser Glu
           115                 120                 125
   Glu Asp His Thr Asn Ala Ala Cys Phe Ala Cys Ile Leu Leu Ser His
       130                 135                 140
   Gly Glu Glu Asn Val Ile Tyr Gly Lys Asp Gly Val Thr Pro Ile Lys
   145                 150                 155                 160
   Asp Leu Thr Ala His Phe Arg Gly Asp Arg Cys Lys Thr Leu Leu Glu
                   165                 170                 175
   Lys Pro Lys Leu Phe Phe Ile Gln Ala Ala Arg Gly Thr Glu Leu Asp
               180                 185                 190
   Asp Gly Ile Gln Ala Asp Ser Gly Pro Ile Asn Asp Thr Asp Ala Asn
                   195                 200                 205
   Pro Arg Tyr Lys Ile Pro Val Glu Ala Asp Phe Leu Phe Ala Tyr Ser
       210                 215                 220
   Thr Val Pro Gly Tyr Tyr Ser Trp Arg Ser Pro Gly Arg Gly Ser Trp
   225                 230                 235                 240
   Phe Val Gln Ala Leu Cys Ser Ile Leu Glu Glu His Gly Lys Asp Leu
                   245                 250                 255
   Glu Ile Met Gln Ile Leu Thr Arg Val Asn Asp Arg Val Ala Arg His
               260                 265                 270
   Phe Glu Ser Gln Ser Asp Asp Pro His Phe His Glu Lys Lys Gln Ile
           275                 280                 285
   Pro Cys Val Val Ser Met Leu Thr Lys Glu Leu Tyr Phe Ser Gln Leu
       290                 295                 300
   Glu His His His His His His
   305                 310
<210> 22
   <211> 936
   <212> DNA
   <213> Homo sapiens
<220>
   <221> CDS
   <222> (1)..(936)
   <223> Procaspase-7 DDD wild-type safety catch, active site C to A mutant sequence
<400> 22
   atg gca gat gat cag ggc tgt att gaa gag cag ggg gtt gag gat tca       48
   Met Ala Asp Asp Gln Gly Cys Ile Glu Glu Gln Gly Val Glu Asp Ser
   1               5                   10                  15
   gca aat gaa gat tca gtg gat gct aag cca gac cgg tcc tcg ttt gta       96
   Ala Asn Glu Asp Ser Val Asp Ala Lys Pro Asp Arg Ser Ser Phe Val
               20                  25                  30
   ccg tcc ctc ttc agt aag aag aag aaa aat gtc acc atg cga tcc atc      144
   Pro Ser Leu Phe Ser Lys Lys Lys Lys Asn Val Thr Met Arg Ser Ile
           35                  40                  45
   aag acc acc cgg gac cga gtg cct aca tat cag tac aac atg aat ttt      192
   Lys Thr Thr Arg Asp Arg Val Pro Thr Tyr Gln Tyr Asn Met Asn Phe
       50                  55                  60
   gaa aag ctg ggc aaa tgc atc ata ata aac aac aag aac ttt gat aaa      240
   Glu Lys Leu Gly Lys Cys Ile Ile Ile Asn Asn Lys Asn Phe Asp Lys
   65                  70                  75                  80
   gtg aca ggt atg ggc gtt cga aac gga aca gac aaa gat gcc gag gcg       288
   Val Thr Gly Met Gly Val Arg Asn Gly Thr Asp Lys Asp Ala Glu Ala
                   85                  90                  95
   ctc ttc aag tgc ttc cga agc ctg ggt ttt gac gtg att gtc tat aat       336
   Leu Phe Lys Cys Phe Arg Ser Leu Gly Phe Asp Val Ile Val Tyr Asn
               100                 105                 110
   gac tgc tct tgt gcc aag atg caa gat ctg ctt aaa aaa gct tct gaa       384
   Asp Cys Ser Cys Ala Lys Met Gln Asp Leu Leu Lys Lys Ala Ser Glu
           115                 120                 125
   gag gac cat aca aat gcc gcc tgc ttc gcc tgc atc ctc tta agc cat       432
   Glu Asp His Thr Asn Ala Ala Cys Phe Ala Cys Ile Leu Leu Ser His
       130                 135                 140
   gga gaa gaa aat gta att tat ggg aaa gat ggt gtc aca cca ata aag       480
   Gly Glu Glu Asn Val Ile Tyr Gly Lys Asp Gly Val Thr Pro Ile Lys
   145                 150                 155                 160
   gat ttg aca gcc cac ttt agg ggg gat aga tgc aaa acc ctt tta gag       528
   Asp Leu Thr Ala His Phe Arg Gly Asp Arg Cys Lys Thr Leu Leu Glu
                   165                 170                 175
   aaa ccc aaa ctc ttc ttc att cag gct gcc cga ggg acc gag ctt gat       576
   Lys Pro Lys Leu Phe Phe Ile Gln Ala Ala Arg Gly Thr Glu Leu Asp
               180                 185                 190
   gat ggc atc cag gcc gac tcg ggg ccc atc aat gac gca gat gct aat       624
   Asp Gly Ile Gln Ala Asp Ser Gly Pro Ile Asn Asp Ala Asp Ala Asn
           195                 200                 205
   cct cga tac aag atc cca gtg gaa gct gac ttc ctc ttc gcc tat tcc       672
   Pro Arg Tyr Lys Ile Pro Val Glu Ala Asp Phe Leu Phe Ala Tyr Ser
       210                 215                 220
   acg gtt cca ggc tat tac tcg tgg agg agc cca gga aga ggc tcc tgg       720
   Thr Val Pro Gly Tyr Tyr Ser Trp Arg Ser Pro Gly Arg Gly Ser Trp
   225                 230                 235                 240
   ttt gtg caa gcc ctc tgc tcc atc ctg gag gag cac gga aaa gac ctg       768
   Phe Val Gln Ala Leu Cys Ser Ile Leu Glu Glu His Gly Lys Asp Leu
                   245                 250                 255
   gaa atc atg cag atc ctc acc agg gtg aat gac aga gtt gcc agg cac       816
   Glu Ile Met Gln Ile Leu Thr Arg Val Asn Asp Arg Val Ala Arg His
               260                 265                 270
   ttt gag tct cag tct gat gac cca cac ttc cat gag aag aag cag atc       864
   Phe Glu Ser Gln Ser Asp Asp Pro His Phe His Glu Lys Lys Gln Ile
           275                 280                 285
   ccc tgt gtg gtc tcc atg ctc acc aag gaa ctc tac ttc agt caa ctc       912
   Pro Cys Val Val Ser Met Leu Thr Lys Glu Leu Tyr Phe Ser Gln Leu
       290                 295                 300
   gag cac cac cac cac cac cac tga                                       936
   Glu His His His His His His
   305                 310
<210> 23
   <211> 311
   <212> PRT
   <213> Homo sapiens
<400> 23
   Met Ala Asp Asp Gln Gly Cys Ile Glu Glu Gln Gly Val Glu Asp Ser
   1               5                   10                  15
   Ala Asn Glu Asp Ser Val Asp Ala Lys Pro Asp Arg Ser Ser Phe Val
               20                  25                  30
   Pro Ser Leu Phe Ser Lys Lys Lys Lys Asn Val Thr Met Arg Ser Ile
           35                  40                  45
   Lys Thr Thr Arg Asp Arg Val Pro Thr Tyr Gln Tyr Asn Met Asn Phe
       50                  55                  60
   Glu Lys Leu Gly Lys Cys Ile Ile Ile Asn Asn Lys Asn Phe Asp Lys
   65                  70                  75                  80
   Val Thr Gly Met Gly Val Arg Asn Gly Thr Asp Lys Asp Ala Glu Ala
                   85                  90                  95
   Leu Phe Lys Cys Phe Arg Ser Leu Gly Phe Asp Val Ile Val Tyr Asn
               100                 105                 110
   Asp Cys Ser Cys Ala Lys Met Gln Asp Leu Leu Lys Lys Ala Ser Glu
           115                 120                 125
   Glu Asp His Thr Asn Ala Ala Cys Phe Ala Cys Ile Leu Leu Ser His
       130                 135                 140
   Gly Glu Glu Asn Val Ile Tyr Gly Lys Asp Gly Val Thr Pro Ile Lys
   145                 150                 155                 160
   Asp Leu Thr Ala His Phe Arg Gly Asp Arg Cys Lys Thr Leu Leu Glu
                   165                 170                 175
   Lys Pro Lys Leu Phe Phe Ile Gln Ala Ala Arg Gly Thr Glu Leu Asp
               180                 185                 190
   Asp Gly Ile Gln Ala Asp Ser Gly Pro Ile Asn Asp Ala Asp Ala Asn
           195                 200                 205
   Pro Arg Tyr Lys Ile Pro Val Glu Ala Asp Phe Leu Phe Ala Tyr Ser
       210                 215                 220
   Thr Val Pro Gly Tyr Tyr Ser Trp Arg Ser Pro Gly Arg Gly Ser Trp
   225 230 235 240
   Phe Val Gln Ala Leu Cys Ser Ile Leu Glu Glu His Gly Lys Asp Leu
   245 250 255
   Glu Ile Met Gln Ile Leu Thr Arg Val Asn Asp Arg Val Ala Arg His
   260 265 270
   Phe Glu Ser Gln Ser Asp Asp Pro His Phe His Glu Lys Lys Gln Ile
   275 280 285
   Pro Cys Val Val Ser Met Leu Thr Lys Glu Leu Tyr Phe Ser Gln Leu
   290 295 300
   Glu His His His His His His
   305 310
<210> 24
   <211> 303
   <212> PRT
   <213> Homo sapiens
<220>
   <221> MISC_FEATURE
   <222> (1)..(303)
   <223> Procaspase-7 with amino acid DTD wild-type safety catch sequence
   (Accession Number NM_001227)
<400> 24
   Met Ala Asp Asp Gln Gly Cys Ile Glu Glu Gln Gly Val Glu Asp Ser
   1 5 10 15
   Ala Asn Glu Asp Ser Val Asp Ala Lys Pro Asp Arg Ser Ser Phe Val
   20 25 30
   Pro Ser Leu Phe Ser Lys Lys Lys Lys Asn Val Thr Met Arg Ser Ile
   35 40 45
   Lys Thr Thr Arg Asp Arg Val Pro Thr Tyr Gln Tyr Asn Met Asn Phe
   50 55 60
   Glu Lys Leu Gly Lys Cys Ile Ile Ile Asn Asn Lys Asn Phe Asp Lys
   65 70 75 80
   Val Thr Gly Met Gly Val Arg Asn Gly Thr Asp Lys Asp Ala Glu Ala
   85 90 95
   Leu Phe Lys Cys Phe Arg Ser Leu Gly Phe Asp Val Ile Val Tyr Asn
   100 105 110
   Asp Cys Ser Cys Ala Lys Met Gln Asp Leu Leu Lys Lys Ala Ser Glu
           115                 120                 125
   Glu Asp His Thr Asn Ala Ala Cys Phe Ala Cys Ile Leu Leu Ser His
       130                 135                 140
   Gly Glu Glu Asn Val Ile Tyr Gly Lys Asp Gly Val Thr Pro Ile Lys
   145                 150                 155                 160
   Asp Leu Thr Ala His Phe Arg Gly Asp Arg Cys Lys Thr Leu Leu Glu
                   165                 170                 175
   Lys Pro Lys Leu Phe Phe Ile Gln Ala Cys Arg Gly Thr Glu Leu Asp
               180                 185                 190
   Asp Gly Ile Gln Ala Asp Ser Gly Pro Ile Asn Asp Thr Asp Ala Asn
           195                 200                 205
   Pro Arg Tyr Lys Ile Pro Val Glu Ala Asp Phe Leu Phe Ala Tyr Ser
       210                 215                 220
   Thr Val Pro Gly Tyr Tyr Ser Trp Arg Ser Pro Gly Arg Gly Ser Trp
   225                 230                 235                 240
   Phe Val Gln Ala Leu Cys Ser Ile Leu Glu Glu His Gly Lys Asp Leu
                   245                 250                 255
   Glu Ile Met Gln Ile Leu Thr Arg Val Asn Asp Arg Val Ala Arg His
               260                 265                 270
   Phe Glu Ser Gln Ser Asp Asp Pro His Phe His Glu Lys Lys Gln Ile
           275                 280                 285
   Pro Cys Val Val Ser Met Leu Thr Lys Glu Leu Tyr Phe Ser Gln
       290                 295                 300
<210> 25
   <211> 303
   <212> PRT
   <213> Homo sapiens
<220>
   <221> MISC_FEATURE
   <222> (1)..(303)
   <223> Procaspase-7 DDD wild-type safety catch sequence
<400> 25
   Met Ala Asp Asp Gln Gly Cys Ile Glu Glu Gln Gly Val Glu Asp Ser
   1               5                   10                  15
   Ala Asn Glu Asp Ser Val Asp Ala Lys Pro Asp Arg Ser Ser Phe Val
               20                  25                  30
   Pro Ser Leu Phe Ser Lys Lys Lys Lys Asn Val Thr Met Arg Ser Ile
           35                  40                  45
   Lys Thr Thr Arg Asp Arg Val Pro Thr Tyr Gln Tyr Asn Met Asn Phe
       50                  55                  60
   Glu Lys Leu Gly Lys Cys Ile Ile Ile Asn Asn Lys Asn Phe Asp Lys
   65                  70                  75                  80
   Val Thr Gly Met Gly Val Arg Asn Gly Thr Asp Lys Asp Ala Glu Ala
                   85                  90                  95
   Leu Phe Lys Cys Phe Arg Ser Leu Gly Phe Asp Val Ile Val Tyr Asn
               100                 105                 110
   Asp Cys Ser Cys Ala Lys Met Gln Asp Leu Leu Lys Lys Ala Ser Glu
           115                 120                 125
   Glu Asp His Thr Asn Ala Ala Cys Phe Ala Cys Ile Leu Leu Ser His
       130                 135                 140
   Gly Glu Glu Asn Val Ile Tyr Gly Lys Asp Gly Val Thr Pro Ile Lys
   145                 150                 155                 160
   Asp Leu Thr Ala His Phe Arg Gly Asp Arg Cys Lys Thr Leu Leu Glu
                   165                 170                 175
   Lys Pro Lys Leu Phe Phe Ile Gln Ala Cys Arg Gly Thr Glu Leu Asp
               180                 185                 190
   Asp Gly Ile Gln Ala Asp Ser Gly Pro Ile Asn Asp Asp Asp Ala Asn
           195                 200                 205
   Pro Arg Tyr Lys Ile Pro Val Glu Ala Asp Phe Leu Phe Ala Tyr Ser
       210                 215                 220
   Thr Val Pro Gly Tyr Tyr Ser Trp Arg Ser Pro Gly Arg Gly Ser Trp
   225                 230                 235                 240
   Phe Val Gln Ala Leu Cys Ser Ile Leu Glu Glu His Gly Lys Asp Leu
                   245                 250                 255
   Glu Ile Met Gln Ile Leu Thr Arg Val Asn Asp Arg Val Ala Arg His
               260                 265                 270
   Phe Glu Ser Gln Ser Asp Asp Pro His Phe His Glu Lys Lys Gln Ile
           275                 280                 285
   Pro Cys Val Val Ser Met Leu Thr Lys Glu Leu Tyr Phe Ser Gln
       290                 295                 300
<210> 26
   <211> 303
   <212> PRT
   <213> Homo sapiens
<220>
   <221> MISC_FEATURE
   <222> (1)..(303)
   <223> Procaspase-7 DTD wild-type safety catch, active site C to A mutant sequence
<400> 26
   Met Ala Asp Asp Gln Gly Cys Ile Glu Glu Gln Gly Val Glu Asp Ser
   1               5                   10                  15
   Ala Asn Glu Asp Ser Val Asp Ala Lys Pro Asp Arg Ser Ser Phe Val
               20                  25                  30
   Pro Ser Leu Phe Ser Lys Lys Lys Lys Asn Val Thr Met Arg Ser Ile
           35                  40                  45
   Lys Thr Thr Arg Asp Arg Val Pro Thr Tyr Gln Tyr Asn Met Asn Phe
       50                  55                  60
   Glu Lys Leu Gly Lys Cys Ile Ile Ile Asn Asn Lys Asn Phe Asp Lys
   65                  70                  75                  80
   Val Thr Gly Met Gly Val Arg Asn Gly Thr Asp Lys Asp Ala Glu Ala
                   85                  90                  95
   Leu Phe Lys Cys Phe Arg Ser Leu Gly Phe Asp Val Ile Val Tyr Asn
               100                 105                 110
   Asp Cys Ser Cys Ala Lys Met Gln Asp Leu Leu Lys Lys Ala Ser Glu
           115                 120                 125
   Glu Asp His Thr Asn Ala Ala Cys Phe Ala Cys Ile Leu Leu Ser His
       130                 135                 140
   Gly Glu Glu Asn Val Ile Tyr Gly Lys Asp Gly Val Thr Pro Ile Lys
   145                 150                 155                 160
   Asp Leu Thr Ala His Phe Arg Gly Asp Arg Cys Lys Thr Leu Leu Glu
                   165                 170                 175
   Lys Pro Lys Leu Phe Phe Ile Gln Ala Ala Arg Gly Thr Glu Leu Asp
               180                 185                 190
   Asp Gly Ile Gln Ala Asp Ser Gly Pro Ile Asn Asp Thr Asp Ala Asn
           195                 200                 205
   Pro Arg Tyr Lys Ile Pro Val Glu Ala Asp Phe Leu Phe Ala Tyr Ser
       210                 215                 220
   Thr Val Pro Gly Tyr Tyr Ser Trp Arg Ser Pro Gly Arg Gly Ser Trp
   225                 230                 235                 240
   Phe Val Gln Ala Leu Cys Ser Ile Leu Glu Glu His Gly Lys Asp Leu
                   245                 250                 255
   Glu Ile Met Gln Ile Leu Thr Arg Val Asn Asp Arg Val Ala Arg His
               260                 265                 270
   Phe Glu Ser Gln Ser Asp Asp Pro His Phe His Glu Lys Lys Gln Ile
           275                 280                 285
   Pro Cys Val Val Ser Met Leu Thr Lys Glu Leu Tyr Phe Ser Gln
       290                 295                 300
<210> 27
   <211> 303
   <212> PRT
   <213> Homo sapiens
<220>
   <221> MISC_FEATURE
   <222> (1)..(303)
   <223> Procaspase-7 DDD wild-type safety catch, active site C to A mutant sequence
<400> 27
   Met Ala Asp Asp Gln Gly Cys Ile Glu Glu Gln Gly Val Glu Asp Ser
   1               5                   10                  15
   Ala Asn Glu Asp Ser Val Asp Ala Lys Pro Asp Arg Ser Ser Phe Val
               20                  25                  30
   Pro Ser Leu Phe Ser Lys Lys Lys Lys Asn Val Thr Met Arg Ser Ile
           35                  40                  45
   Lys Thr Thr Arg Asp Arg Val Pro Thr Tyr Gln Tyr Asn Met Asn Phe
       50                  55                  60
   Glu Lys Leu Gly Lys Cys Ile Ile Ile Asn Asn Lys Asn Phe Asp Lys
   65                  70                  75                  80
   Val Thr Gly Met Gly Val Arg Asn Gly Thr Asp Lys Asp Ala Glu Ala
                   85                  90                  95
   Leu Phe Lys Cys Phe Arg Ser Leu Gly Phe Asp Val Ile Val Tyr Asn
               100                 105                 110
   Asp Cys Ser Cys Ala Lys Met Gln Asp Leu Leu Lys Lys Ala Ser Glu
           115                 120                 125
   Glu Asp His Thr Asn Ala Ala Cys Phe Ala Cys Ile Leu Leu Ser His
       130                 135                 140
   Gly Glu Glu Asn Val Ile Tyr Gly Lys Asp Gly Val Thr Pro Ile Lys
   145                 150                 155                 160
   Asp Leu Thr Ala His Phe Arg Gly Asp Arg Cys Lys Thr Leu Leu Glu
                   165                 170                 175
   Lys Pro Lys Leu Phe Phe Ile Gln Ala Ala Arg Gly Thr Glu Leu Asp
               180                 185                 190
   Asp Gly Ile Gln Ala Asp Ser Gly Pro Ile Asn Asp Asp Asp Ala Asn
           195                 200                 205
   Pro Arg Tyr Lys Ile Pro Val Glu Ala Asp Phe Leu Phe Ala Tyr Ser
       210                 215                 220
   Thr Val Pro Gly Tyr Tyr Ser Trp Arg Ser Pro Gly Arg Gly Ser Trp
   225                 230                 235                 240
   Phe Val Gln Ala Leu Cys Ser Ile Leu Glu Glu His Gly Lys Asp Leu
                   245                 250                 255
   Glu Ile Met Gln Ile Leu Thr Arg Val Asn Asp Arg Val Ala Arg His
               260                 265                 270
   Phe Glu Ser Gln Ser Asp Asp Pro His Phe His Glu Lys Lys Gln Ile
           275                 280                 285
   Pro Cys Val Val Ser Met Leu Thr Lys Glu Leu Tyr Phe Ser Gln
       290                 295                 300

## Description

### CROSS-REFERENCES TO RELATED APPLICATIONS

This application claims the benefit under 35 USC 119(e) of U.S. Provisional Application Serial 60/684,807 filed May 26, 2005; and U.S. Provisional Application Serial 60743878 filed March 28, 2006.

### STATEMENT ON FEDERALLY SPONSORED RESEARCH OR DEVELOPMENT

This invention was made with government support under NSF Grant/Contract CHE-0134779 awarded by the National Science Foundation. The government has certain rights in the invention.

### BACKGROUND OF THE INVENTION

Apoptosis, or programmed cell death, plays a central role in the development and homeostasis of all multicellular organisms (Shi Y, 2002, Molecular Cell 9:459-470). A frequent hallmark of cancer is resistance to natural apoptotic signals. Depending on the cancer type, this resistance is typically due to up- or down-regulation of key proteins in the apoptotic cascade or to mutations in genes encoding these proteins. Such changes occur in both the intrinsic apoptotic pathway, which funnels through the mitochondria and caspase-9, and the extrinsic apoptotic pathway, which involves the action of death receptors and caspase-8. For example, alterations in proper levels of proteins such as p53, Bim, Bax, Apaf-1, FLIP and many others have been observed in cancers. The alterations can lead to a defective apoptotic cascade, one in which the upstream pro-apoptotic signal is not adequately transmitted to activate the executioner caspases, caspase-3 and caspase-7.

As most apoptotic pathways ultimately involve the activation of procaspase-3, upstream genetic abnormalities are effectively "breaks" in the apoptotic circuitry, and as a result such cells proliferate atypically. Given the central role of apoptosis in cancer, efforts have been made to develop therapeutics that target specific proteins in the apoptotic cascade. For instance, peptidic or small molecule binders to cascade members such as p53 and proteins in the Bcl family or to the inhibitor of apoptosis (IAP) family of proteins have pro-apoptotic activity, as do compounds that promote the oligomerization of Apaf-1. However, because such compounds target early (or intermediate to high) positions on the apoptotic cascade, cancers with mutations in proteins downstream of those members can still be resistant to the possible beneficial effects of those compounds.

For therapeutic purposes it would be advantageous to identify a small molecule that directly activates a proapoptotic protein far downstream in the apoptotic cascade. The approach to our invention involves such a relatively low position in the cascade, thus enabling the killing of even those cells that have mutations in their upstream apoptotic machinery. Moreover, the therapeutic strategies disclosed herein can have a higher likelihood of success if that proapoptotic protein were upregulated in cancer cells. In the present invention, our efforts to identify small molecules began with targeting the significant downstream effector protein of apoptosis, procaspase-3.

The conversion or activation of procaspase-3 to caspase-3 results in the generation of the active "executioner" caspase form that subsequently catalyzes the hydrolysis of a multitude of protein substrates. Active caspase-3 is a homodimer of heterodimers and is produced by proteolysis of procaspase-3. In vivo, this proteolytic activation typically occurs through the action of caspase-8 or caspase-9. To ensure that the proenzyme or zymogen is not prematurely activated, procaspase-3 has a 12 amino acid "safety catch" that blocks access to the IETD site (amino acid sequence, ile-glu-thr-asp) of proteolysis. See Roy, S. et al.; Maintenance of caspase-3 proenzyme dormancy by an intrinsic "safety catch" regulatory tripeptide, Proc. Natl. Acad. Sci. 98, 6132-6137 (2001).

This safety catch enables procaspase-3 to resist autocatalytic activation and proteolysis by caspase-9. Mutagenic studies indicate that three consecutive aspartic acid residues appear to be the critical components of the safety catch. The position of the safety catch is sensitive to pH; thus, upon cellular acidification (as occurs during apoptosis) the safety catch is thought to allow access to the site of proteolysis, and active caspase-3 can be produced either by the action of caspase-9 or through an autoactivation mechanism.

In particular cancers, the expression of procaspase-3 is upregulated. A study of primary isolates from 20 colon cancer patients revealed that on average, procaspase-3 was upregulated six-fold in such isolates relative to adjacent non-cancerous tissue (Roy et al., 2001). In addition, procaspase-3 is upregulated in certain neuroblastomas, lymphomas, and liver cancers (Nakagawara, A. et al., 1997, Cancer Res. 57:4578-4584; Izban, K. F. et al., Am. J. Pathol. 154:1439-1447; Persad, R. et al., Modern Patholo. 17:861-867). Furthermore, a systematic evaluation was performed of procaspase-3 levels in the 60 cell-line panel used for cancer screening by the National Cancer Institute (NCI) Developmental Therapeutics Program. The evaluation revealed that certain lung, melanoma, renal, and breast cancers show greatly enhanced levels of procaspase-3 expression (Svingen, P.A. et al., Clin. Cancer Res. 10:6807-6820).

Due to the role of active caspase-3 in achieving apoptosis, the relatively high expression levels of procaspase-3 in certain cancerous cell types, and the intriguing safety catch-mediated suppression of its autoactivation, we reasoned that small molecules that directly modify procaspase-3 could be identified and that such molecules could have great applicability in targeted cancer therapy.

Herein we disclose small molecule modifiers of procaspases, including in particular activators capable of converting procaspase-3 to its effector form. Furthermore, we demonstrate that certain small drug molecules can directly and immediately activate procaspase-3 to achieve a proapoptotic effect in cancer cells in vivo. We believe these are the first small molecules known to directly activate procaspase-3. The direct activation of executioner caspases represents a novel and valuable anti-cancer strategy.

### SUMMARY OF THE INVENTION

In general the terms and phrases used herein have their art-recognized meaning, which can be found by reference to standard texts, journal references and contexts known to those skilled in the art.

The following abbreviations are applicable. IAP, inhibitor of apoptosis; PAC-1, procaspase activating compound 1; PARP, Poly(ADP-ribose) polymerase.

The invention broadly provides compounds, compounds for use in the treatment of cancer, pharmaceutical compositions for use in the treatment of cancer, methods of screening for compounds, and methods of screening for cell and patient suitability for treatment in connection with modifiers of procaspases. In an embodiment, the modifiers are activators. In an embodiment, the invention provides such compounds and methods in connection with activators of procaspase-3 and procaspase-7. In embodiments, the inventions are applicable in the context of a variety of cancer diseases and cancer cell types such as breast, lymphoma, adrenal, renal, melanoma, leukemia, neuroblastoma, lung, brain, and others known in the art.

As a further introduction, we have discovered compounds capable of activating an enzyme that is often overexpressed in its inactive form in cancer cells. The compound induces programmed cell death (apoptosis) in cancer cells, including those that have upregulated procaspase-3. Cancer is a large and growing problem, and is now the number one killer of in the United States. Many cancers resist standard chemotherapy. Compounds of the invention can take advantage of a biological target that may be upregulated in cancer cells and thus can prove effective even in cells with defects in their apoptotic machinery. These compounds can also be successful in targeted cancer therapy, where there can be advantages of selectivity in the killing of cancer cells with comparably reduced toxicity to non-cancerous cells having lower levels of procaspase-3.

Without wishing to be bound by a particular theory, it is believed that compounds of the invention may act via the mechanism of modulation of apoptosis or programmed cell death to be effective in the treatment of cancer cells. In a preferred embodiment, the modulation of apoptosis is by induction of apoptosis. The modulation of apoptosis may be by inhibition of apoptosis.

Disclosed herein is a method of selectively inducing apoptosis in a cancer cell, comprising: (a) administering to said cancer cell a compound capable of modifying a procaspase-3 molecule of said cancer cell; and (b) modifying said procaspase-3 molecule so as to induce apoptosis. In an embodiment, said cancer cell is in a patient in need of treatment.

In an embodiment, the invention provides compounds of formula ZZ; 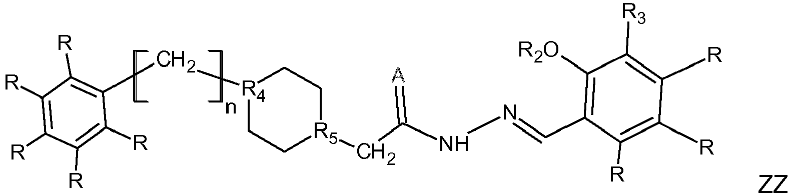 wherein n = 1 or 2; R, independently of other R, is hydrogen, halogen, allyl, or short alkyl; R2 = hydrogen, short alkyl, ester; R3 = hydrogen, halogen, alkyl, haloalkyl, allyl, alkenyl, or haloalkenyl; R4 and R5 are N; or R4=N and R5=C; or R4 and R5 = C; and A = oxygen or sulfur, for use in the treatment of cancer, by administering to a patient in need thereof an effective amount of said compound. In an embodiment, said compound is selected from the group consisting of formula ZZ, PAC-1, and Structure 5. In an embodiment, said compound is PAC-1.

In an embodiment, the compound is selected from those having formula ZZ wherein R4 and R5 are both N, A is oxygen, and other variable groups are as defined above. In an embodiment, the compound is selected from those having formula ZZ wherein R4 and R5 and both N, A is oxygen, R2 is hydrogen, and other variable groups are as defined above. In an embodiment, the compound is selected from those having formula ZZ wherein R4 and R5 and both N, A is oxygen, R2 is hydrogen, R3 is allyl, and other variable groups are as defined above.

In an embodiment, the method further comprises the step of assessing a procaspase-3 or caspase-3 parameter in a cancer cell; wherein said parameter is one or more of a semi-quantitative or quantitative amount, a functional amount, and an activity level of said procaspase-3 or caspase-3.

In an embodiment, the invention provides a method of direct in vitro screening for a compound capable of modifying a procaspase-3 molecule, comprising: (a) providing a test compound; (b) providing a purified procaspase-3; (c) exposing the test compound to the purified procaspase-3; (d) measuring a procaspase-3 activity following exposure to the test compound; (e) identifying a modifying compound by comparing a test activity upon the exposure to the test compound with an unmodified activity in the absence of exposure to the test compound; thereby screening for a compound capable of modifying a procaspase-3 molecule. In an embodiment, the method further comprises comparing said modified activity or said unmodified activity with a reference activity; wherein said reference activity is due to exposure of procaspase-3 to a compound selected from the group consisting of structural formula ZZ or subsets of compounds of such formula, PAC-1, and Structure 5.

Disclosed herein is a method of screening for a compound capable of activating procaspase-3 comprising: a) providing procaspase-3; providing a test compound, preferably a small molecule; b) reacting the procaspase-3 with the test compound, thereby putatively generating caspase-3; and c) measuring caspase-3 activity. In a particular embodiment, the measuring caspase-3 activity employs a substrate, Ac-DEVD-pNA. In a particular embodiment, the measuring uses a wavelength readout parameter of about 410 nm. In a particular embodiment, the screening is carried out in parallel using multiple test compounds.

Disclosed herein is a method of screening which uses the detection of a subunit of procaspase-3 as an indicator that the full length (inactive) procaspase-3 is processed to caspase-3. In a particular embodiment, the subunit has a molecular weight of about 19kD as measured by a protein gel migration technique, for example in a Western blot.

In an embodiment, the invention provides a method of in cellular screening for a compound capable of modifying a procaspase-3 molecule, comprising: (a) providing a test compound; (b) providing a cell, wherein the cell putatively expresses procaspase-3; (c) exposing the cell to the test compound; (d) measuring a cell parameter following exposure to the test compound; wherein said parameter comprises one or more of cell viability, apoptotic indicator, and other parameters; (e) identifying a modifying compound by comparing a tested cell parameter upon the exposure to the test compound with an unmodified cell parameter in the absence of exposure to the test compound; thereby screening for a compound capable of modifying a procaspase-3 molecule. In an embodiment, the method further comprises comparing said modified activity or said unmodified activity with a reference activity; wherein said reference activity is due to exposure to a compound selected from the group consisting of formula ZZ or subsets of compounds of such formula, PAC-1, and Structure 5.

Disclosed herein is a method of identifying or diagnosing a potential susceptibility to treatment for a cancer cell with a procaspase activator compound, comprising (a) assessing a procaspase parameter in said cancer cell; and (b) determining if said parameter allows an increased susceptibility to activation of a procaspase. Said procaspase parameter may be a procaspase-3 level and said procaspase may be procaspase-3. Said procaspase parameter may be a procaspase-7 level and said procaspase may be procaspase-7. A level can be a semi-quantitative or quantitative amount, or functional amount (e.g. an activity-based amount, e.g. a standardized unit or international unit).

In an embodiment, the invention provides a compound for use in treating a cancer cell, the use comprising (a) identifying a potential susceptibility to treatment of a cancer cell with a procaspase activator compound; and (b) exposing said cancer cell to an effective amount of the procaspase activator compound. In an embodiment, the procaspase activator compound is selected from the group consisting of formula ZZ or subsets of compounds of such formula, PAC-1, and Structure 5. In an embodiment, said procaspase activator compound is capable of activating procaspase-3, procaspase-7, or both procaspase-3 and procaspase-7.

Disclosed herein is a method of synthesizing PAC-1, comprising the steps of Scheme 1. Disclosed herein is a method of synthesizing Compound 5, comprising the steps of Scheme 1 with appropriate modification. Also disclosed is a method of synthesizing compounds of the formula ZZ as disclosed herein and as would be understood in the art.

Disclosed herein are compounds of the formula ZZ: 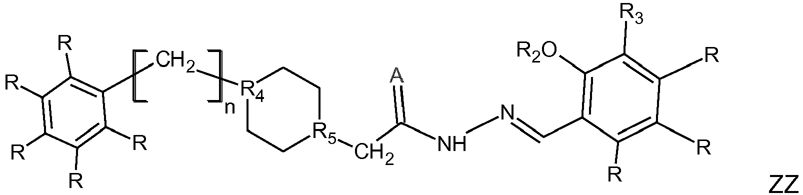 wherein n = 1 or 2; R, independently of other R, is hydrogen, halogen, allyl, or short alkyl; R2 = hydrogen, short alkyl, ester, or other moiety that is removable under physiological conditions; R3 = hydrogen, halogen, alkyl, haloalkyl, allyl, alkenyl, or haloalkenyl; R4 and R5 are N; or R4=N and R5=C; or R4 and R5 = C; and A = oxygen or sulfur.

Disclosed herein are compounds of the formula ZZ excluding PAC-1, wherein the structure of PAC-1 is:

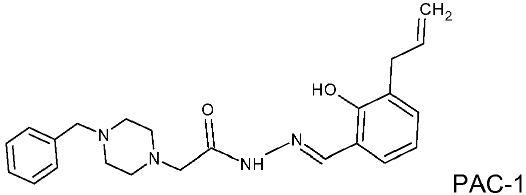

In an embodiment, the invention provides a compound of Structure 5 for use in the treatment of cancer, wherein the structure is:

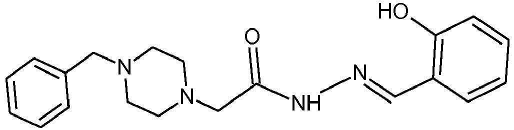

In an embodiment, a composition for use according to the invention is a chemotherapeutic agent.

In some embodiments, the invention provides compounds and methods involving effective concentrations preferably from about 10 nM to about 100 µM of the disclosed structural formulas. The effective concentrations may be from about 200 nM to about 5 µM. The effective concentration may be considered to be a value such as a 50% activity concentration in a direct procaspase activation assay, in a cell apoptosis induction assay, or in an animal clinical therapeutic assessment. Such value may be less than about 200 µM. The value may be less than about 10 µM.

Compounds of the disclosure include those of the disclosed formulas and salts and esters of those compounds, including preferably pharmaceutically-acceptable salts and esters.

Disclosed herein are prodrug forms of compositions. Prodrugs of the disclosed compounds are useful in the methods of this disclosure. Any compound that will be converted in vivo to provide a biologically, pharmaceutically or therapeutically active form of a compound of the invention is a prodrug. Various examples and forms of prodrugs are well known in the art. A biomolecule such as a precursor protein or precursor nucleic acid can be a prodrug. Examples of prodrugs are found, inter alia, in Design of Prodrugs, edited by H. Bundgaard, (Elsevier, 1985), Methods in Enzymology, Vol. 42, at pp. 309-396, edited by K. Widder, et. al. (Academic Press, 1985); A Textbook of Drug Design and Development, edited by Krosgaard-Larsen and H. Bundgaard, Chapter 5, "Design and Application of Prodrugs," by H. Bundgaard, at pp. 113-191, 1991); H. Bundgaard, Advanced Drug Delivery Reviews, Vol. 8, p. 1-38 (1992); H. Bundgaard, et al., Journal of Pharmaceutical Sciences, Vol. 77, p. 285 (1988); and Nogrady (1985) Medicinal Chemistry A Biochemical Approach, Oxford University Press, New York, pages 388-392).

When a group of substituents is disclosed herein, it is understood that all individual members of that group and all subgroups, including any isomers and enantiomers of the group members, are disclosed separately. When a Markush group or other grouping is used herein, all individual members of the group and all combinations and subcombinations possible of the group are intended to be individually included in the disclosure. When a compound is described herein such that a particular isomer or enantiomer of the compound is not specified, for example, in a formula or in a chemical name, that description is intended to include each isomers and enantiomer of the compound described individual or in any combination. Additionally, unless otherwise specified, all isotopic variants of compounds disclosed herein are intended to be encompassed by the disclosure. For example, it will be understood that any one or more hydrogens in a molecule disclosed can be replaced with deuterium or tritium. Isotopic variants of a molecule are generally useful as standards in assays for the molecule and in chemical and biological research related to the molecule or its use. Specific names of compounds are intended to be exemplary, as it is known that one of ordinary skill in the art can name the same compounds differently.

Molecules disclosed herein may contain one or more ionizable groups [groups from which a proton can be removed (e.g., -OH, -COOH, etc.) or added (e.g., amines) or which can be quaternized (e.g., amines)]. All possible ionic forms of such molecules and salts thereof are intended to be included individually in the disclosure herein. With regard to salts of the compounds herein, one of ordinary skill in the art can select from among a wide variety of available counterions those that are appropriate for preparation of salts for a given application. For example, in general any anions can be employed in the formation of salts of compounds herein; e.g. halide, sulfate, carboxylate, acetate, phosphate, nitrate, trifluoroacetate, glycolate, pyruvate, oxalate, malate, succinate, fumarate, tartarate, citrate, benzoate, methanesulfonate, ethanesulfonate, p-toluenesulfonate, salicylate and others.

Compounds of the present disclosure, and salts or esters thereof, may exist in their tautomeric form, in which hydrogen atoms are transposed to other parts of the molecules and the chemical bonds between the atoms of the molecules are consequently rearranged. It should be understood that all tautomeric forms, insofar as they may exist, are disclosed herein. Additionally, the compounds may have trans and cis isomers and may contain one or more chiral centers, therefore existing in enantiomeric and diastereomeric forms. Disclosed herein are all such isomers, individual enantiomers, as well as mixtures of cis and trans isomers, mixtures of diastereomers; non-racemic and racemic mixtures of enantiomers (optical isomers); and the foregoing mixtures enriched for one or more forms; except as stated otherwise herein. When no specific mention is made of the configuration (cis, trans or R or S) of a compound (or of an asymmetric carbon), then any one of the isomers or a mixture of more than one isomer is intended. The processes for preparation can use racemates, enantiomers, or diastereomers as starting materials. When enantiomeric or diastereomeric products are prepared, they can be separated by conventional methods, for example, by chromatographic or fractional crystallization. The inventive compounds may be in the free or hydrate form.

Every formulation or combination of components described or exemplified herein can be used, unless otherwise stated.

Whenever a range is described in the present application, for example, a temperature range, a time range, or a composition or concentration range, all intermediate ranges and subranges, as well as all individual values included in the ranges given are intended to be included in the disclosure.

Some references provided herein provide details concerning sources of starting materials, additional starting materials, additional reagents, additional methods of synthesis, additional methods of analysis, and additional uses of the invention. One of ordinary skill in the art will appreciate that starting materials, reagents, solid substrates, synthetic methods, purification methods, and analytical methods other than those specifically exemplified can be employed in the practice of the invention based on knowledge in the art and without resort to undue experimentation.

In an embodiment, the invention provides a therapeutic composition for use in treating cancer, comprising a compound of formula ZZ and a pharmaceutically acceptable carrier. Disclosed herein are therapeutic compositions comprising one or more compounds and for each compound a pharmaceutically acceptable salt or ester thereof; wherein the compounds are present in the composition in an amount or in a combined amount effective for obtaining the desired therapeutic benefit. The therapeutic compositions optionally further comprise one or more pharmaceutically acceptable components, for example carriers and excipients as known in the art.

In an embodiment, the invention provides a compound having the formula ZZ2: 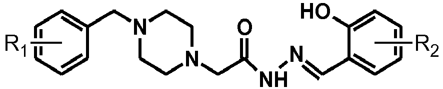 wherein R1 and R2 each independently is hydrogen, halogen, alkyl, allyl, haloalkyl, alkenyl, or haloalkenyl. In an embodiment, R1 and R2 each independently is hydrogen, halogen, allyl, or short alkyl, for use in the treatment of cancer.

In an embodiment, the invention provides a compound having the formula: 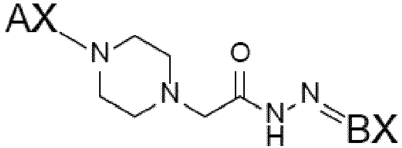 wherein AX and BX are groups as defined in claim 17.

In an embodiment, the invention provides a compound selected from the group consisting of: L01R06, L02R03, L02R06, L08R06, L09R03, L09R06, and L09R08.

Disclosed herein is a method of screening a candidate cancer patient for possible treatment with a procaspase activator by identifying an elevated level of a procaspase in the candidate, comprising obtaining a cell or tissue test sample from the candidate, assessing the procaspase level in the test sample, and determining whether the procaspase level is elevated in the test sample relative to a reference level, thereby screening a candidate cancer patient for possible treatment with a procaspase activator. The procaspase may be selected from the group consisting of procaspase-2, -3, -6-, -7, -8, and -9. In a particular method, the procaspase is procaspase-3.

In some methods, an elevated level of the test sample is at least about 2-fold greater than the reference level. In some methods, an elevated level of the test sample is at least about 4-fold greater than the reference level. In some methods, the reference level is from a second test sample from the same patient. In some methods, the reference level is from a normal cell or tissue sample. The reference level can be from a cell line, such as a cancer cell line or a normal cell line. In some methods, the reference level is an absolute threshold amount. See Svingen, P.A. et al., Clin. Cancer Res. 10:6807-6820 which describes various amounts of levels of procaspases including numbers of molecules per cell.

In an embodiment, the invention provides a compound for use in the treatment of cancer by inducing death in a cancer cell, comprising administering to said cancer cell a compound capable of activating a procaspase molecule of said cancer cell. In an embodiment the procaspase is one or more of procasepas-3 and procaspase-7. In a preferred embodiment the procaspase is procaspase 3. In an embodiment, the compound has structural formula ZZ. In an embodiment, the compound has structural formula ZZ2.

It is recognized that regardless of the ultimate correctness of any mechanistic explanation or hypothesis believed or disclosed herein, an embodiment of the invention can nonetheless be operative and useful.

### BRIEF DESCRIPTION OF THE FIGURES

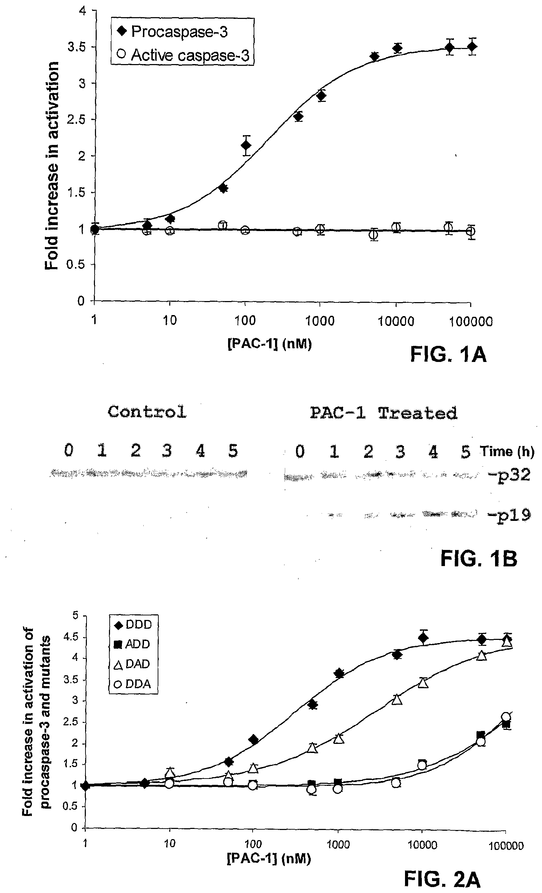

Figure 1. A) In vitro activation of procaspase-3 and active caspase-3 by PAC-1. PAC-1 activates procaspase-3 with an EC₅₀ = 0.22 µM. Error bars represent standard deviations from the mean. B) Cleavage of procaspase-3 to active caspase-3 as induced by PAC-1. Procaspase-3 was recombinantly expressed in E. coli with an N-terminal His-6 tag and purified. Immunoblotting was performed with an anti-His-6 antibody. In the absence of PAC-1, no maturation of procaspase-3 is observed. In the presence of 100 µM PAC-1, cleavage to generate the p19 fragment is observed within 1 hour, and >50% cleavage is observed after 4 hours.
Figure 2. A) Activation of mutants in the "safety catch" region of procaspase-3 by PAC-1. PAC-1 has an EC₅₀ for activation of 0.22 µM on wild type procaspase-3 (DDD), and corresponding EC₅₀ values of 2.77 µM (DAD), 113 µM (DDA), and 131 µM (ADD) for certain mutants. B) PAC-1 activates procaspase-7 with an EC₅₀ of 4.5 µM. C) Dependence of PAC-1 activation of procaspase-3 on pH. At low pH the safety catch is "off", and procaspase-3 is essentially maximally activated. Error bars represent standard deviations from the mean.
Figure 3. PAC-1 induces apoptosis in HL-60 cells. A) Phosphatidylserine exposure (as measured by Annexin-V staining) after a 20 hour treatment with 100 µM PAC-1. B) Chromatin condensation as visualized by Hoescht staining after a 20 hour treatment with 100 µM PAC-1.

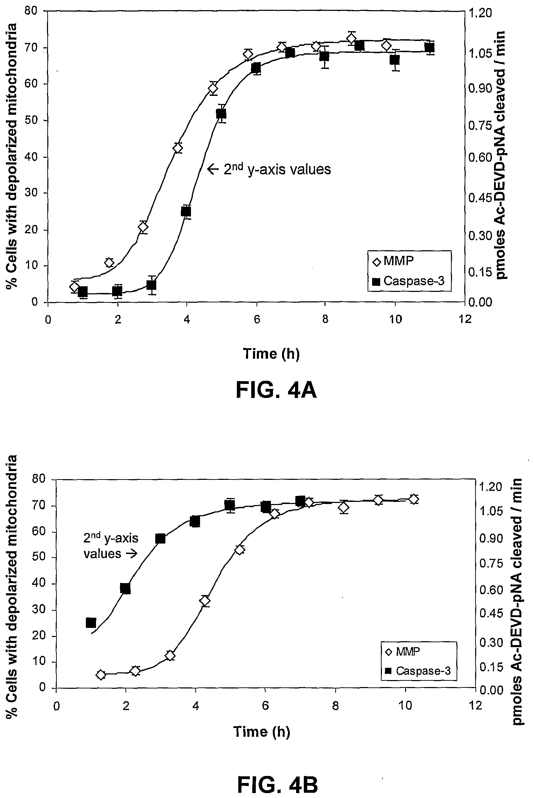

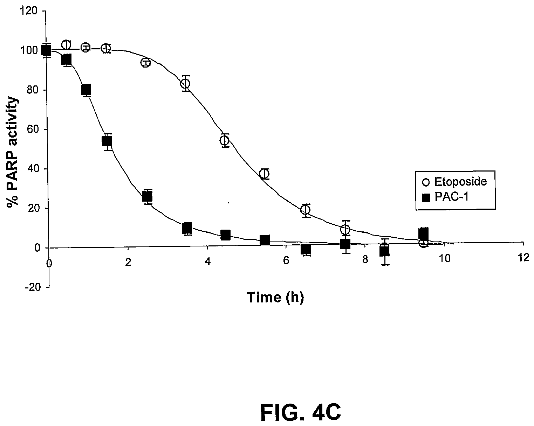

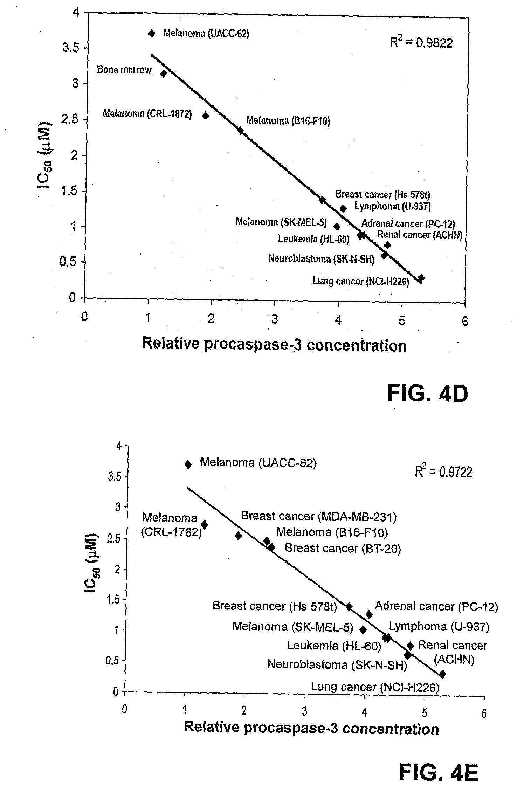

Figure 4. A) Mitochondrial membrane depolarization (MMP) and caspase-3 like activity in HL-60 cells treated with 10 µM etoposide. B) Mitochondrial membrane depolarization (MMP) and caspase-3 like activity in HL-60 cells treated with 100 µM PAC-1. C) PAC-1 treatment (100 µM) induces a rapid decrease in cellular PARP activity in HL-60 cells, consistent with an immediate activation of cellular caspase-3/- 7. In contrast, etoposide (10 µM) treated cells show a decrease in PARP activity at much later time points. D) PAC-1 induces cell death in a procaspase-3 dependent manner. For a number of diverse cancer cell lines, the procaspase-3 levels were determined (by flow cytometry) and the IC₅₀ of PAC-1 was measured (R2=0.9822). PAC-1 is quite potent (IC₅₀=0.35 µM) in the NCI-H226 lung cancer cell line known to have high levels of procaspase-3, but markedly less potent in normal white blood cells derived from the bone marrow of a healthy human donor.
Fig. 5A illustrates relative procaspase-3 levels in normal and cancerous cells from several patients.
Fig. 5B illustrates IC₅₀ levels for PAC-1 in a variety of cell types having a range of relative procaspase-3 levels.
Fig. 5C illustrates the effect of treating animals with PAC-1 on outcomes of tumor growth.
Fig. 5D illustrates the effect of oral treatment of animals with PAC-1 on outcomes of tumor growth.
Fig. 5E illustrates results of progression of cancer in a lung cancer model for control, PAC-1, and gefitinib (Iressa™; AstraZeneca) treatment groups. Tumor cells were injected into mice by i.v. administration; Iressa and PAC-1 were given orally at 100 mg/kg.
Fig. 6A illustrates relative procaspase-3 levels in normal and cancerous cells of three patients.
Fig. 6B illustrates the sensitivity of normal and cancerous cells from Patient 3 to treatment with PAC-1.
Fig. 7 illustrates results of administering PAC-1 intraperitoneally in the context of a mouse model of lung cancer.
Fig. 8A and 8B illustrate structures for compounds of PAC-1 derivatives and a combinatorial library.
Fig. 9 illustrates a nucleotide sequence for Homo sapiens caspase 3, apoptosis-related cysteine peptidase (CASP3), transcript variant alpha, mRNA (Accession No. NM_004346; 2689 bp mRNA linear; obtained from http://www.ncbi.nlm.nih.gov/entrez).
Fig. 10 illustrates a nucleotide sequence for Homo sapiens caspase 7, apoptosis-related cysteine peptidase (CASP7), transcript variant alpha, mRNA (Accession No. NM_001227; 2605 bp; mRNA linear; obtained from http://www.ncbi.nlm.nih.gov/entrez).

### DETAILED DESCRIPTION OF THE INVENTION

The following definitions are provided to clarify their specific use in the context of the invention.

When used herein, the term "chemotherapeutic agent" refers to any substance capable of reducing or preventing the growth, proliferation, or spread of a cancer cell, a population of cancer cells, tumor, or other malignant tissue. The term is intended also to encompass any antitumor or anticancer agent.

When used herein, the term "effective amount" is intended to encompass contexts such as a pharmaceutically effective amount or therapeutically effective amount. For example, in embodiments the amount is capable of achieving a beneficial state, beneficial outcome, functional activity in a screening assay, or improvement of a clinical condition.

When used herein, the term "cancer cell" is intended to encompass definitions as broadly understood in the art. In an embodiment, the term refers to an abnormally regulated cell that can contribute to a clinical condition of cancer in a human or animal. In an embodiment, the term can refer to a cultured cell line or a cell within or derived from a human or animal body. A cancer cell can be of a wide variety of differentiated cell, tissue, or organ types as is understood in the art.

The term "alkyl" refers to a monoradical branched or unbranched saturated hydrocarbon chain preferably having from 1 to 22 carbon atoms and to cycloalkyl groups having one or more rings having 3 to 22 carbon atoms. Short alkyl groups are those having 1 to 6 carbon atoms including methyl, ethyl, propyl, butyl, pentyl and hexyl groups, including all isomers thereof. Long alkyl groups are those having 8-22 carbon atoms and preferably those having 12-22 carbon atoms as well as those having 12-20 and those having 16-18 carbon atoms.

The term "cycloalkyl" refers to cyclic alkyl groups of from 3 to 22 carbon atoms having a single cyclic ring or multiple condensed rings. Cycloalkyl groups include, by way of example, single ring structures such as cyclopropyl, cyclobutyl, cyclopentyl, cyclohexyl, cyclooctyl, and the like, or multiple ring structures such as adamantanyl, and the like.

The term "alkenyl" refers to a monoradical of a branched or unbranched unsaturated hydrocarbon group preferably having from 2 to 22 carbon atoms and to cycloalkenyl groups having one or more rings having 3 to 22 carbon atoms wherein at least one ring contains a double bond. Alkenyl groups may contain one or more double bonds (C=C) which may be conjugated. Preferred alkenyl groups are those having 1 or 2 double bonds. Short alkenyl groups are those having 2 to 6 carbon atoms including ethylene (vinyl) propylene, butylene, pentylene and hexylene groups, including all isomers thereof. Long alkenyl groups are those having 8-22 carbon atoms and preferably those having 12-22 carbon atoms as well as those having 12-20 carbon atoms and those having 16-18 carbon atoms. The term "cycloalkenyl" refers to cyclic alkenyl groups of from 3 to 22 carbon atoms having a single cyclic ring or multiple condensed rings in which at least one ring contains a double bond (C=C). Cycloalkenyl groups include, by way of example, single ring structures such as cyclopropenyl, cyclobutenyl, cyclopentenyl, cyclooctenyl, cylcooctadienyl and cyclooctatrienyl.

The term "alkynyl" refers to a monoradical of an unsaturated hydrocarbon preferably having from 2 to 22 carbon atoms and having one or more triple bonds (C□C). Alkynyl groups include ethynyl, propargyl, and the like. Short alkynyl groups are those having 2 to 6 carbon atoms, including all isomers thereof. Long alkynyl groups are those having 8-22 carbon atoms and preferably those having 12-22 carbon atoms as well as those having 12-20 carbon atoms and those having 16-18 carbon atoms.

The term "aryl" refers to a group containing an unsaturated aromatic carbocyclic group of from 6 to 22 carbon atoms having a single ring (e.g., phenyl), one or mroe rings (e.g., biphenyl)or multiple condensed (fused) rings, wherein at least one ring is aromatic (e.g., naphthyl, dihydrophenanthrenyl, fluorenyl, or anthryl). Aryls include phenyl, naphthyl and the like. Aryl groups may contain portions that are alkyl, alkenyl or akynyl in addition to the the unsaturated aromatic ring(s). The term "alkaryl" refers to the aryl groups containing alkyl portions, i.e., -alkylene-aryl and -substituted alkylene-aryly. Such alkaryl groups are exemplified by benzyl, phenethyl and the like.

Alkyl, alkenyl, alkynyl and aryl groups are optionally substituted as described herein (the term(s) can include substituted variations) and may contain 1-8 non-hydrogen substituents dependent upon the number of carbon atoms in the group and the degree of unsaturation of the group.

The term "alkylene" refers to a diradical of a branched or unbranched saturated hydrocarbon chain, preferably having from 1 to 10 carbon atoms, more preferably having 1-6 carbon atoms, and more preferably having 2-4 carbon atoms; the term can include substituted variations. This term is exemplified by groups such as methylene (-CH2-), ethylene (-CH2CH2-), more generally -(CH2)n-, where n is 1-10 or more preferably 1-6 or n is 2, 3 or 4. Alkylene groups may be branched. Alkylene groups may be optionally substituted. Alkylene groups may have up to two non-hydrogen substituents per carbon atoms. Perferred substituted alkylene groups have 1, 2, 3 or 4 non-hydrogen substituents.

The term "arylene" refers to the diradical derived from aryl (including substituted aryl) as defined above and is exemplified by 1,2-phenylene, 1,3-phenylene, 1,4-phenylene, 1,2-naphthylene and the like.

The term "amino" refers to the group -NH2 or to the group -NRR where each R is independently selected from the group consisting of hydrogen, alkyl, substituted alkyl, cycloalkyl, substituted cycloalkyl, alkenyl, substituted alkenyl, cycloalkenyl, substituted cycloalkenyl, alkynyl, substituted alkynyl, aryl, heteroaryl and heterocyclic provided that both R's are not hydrogen.

Alkyl groups are optionally substituted as discussed herein and may, dependent upon the size of the alkyl group, have preferably from 1-10 substituent groups. Substituted alkyl groups include those that carry 1 to 8 substituents, 1 to 5 substituents, 1 to 3 substituents, and 1 or 2 substituents.

Haloalkyl" refers to alkyl as defined herein substituted by one or more halo groups as defined herein, which may be the same or different. Representative haloalkyl groups include, by way of example, trifluoromethyl, 3-fluorododecyl, 12,12,12-trifluorododecyl, 2-bromooctyl, 3-bromo-6-chloroheptyl, and the like.

The term "heteroaryl" refers to an aromatic group of from 2 to 22carbon atoms having 1 to 4 heteroatoms selected from oxygen, nitrogen and sulfur within at least one ring (if there is more than one ring). Heteroaryl groups may be optionally substituted.

The term "heterocycle" or "heterocyclic" refers to a monoradical saturated or unsaturated group having a single ring or multiple condensed rings, from 2-22 carbon atoms and from 1 to 6 hetero atoms, preferably 1 to 4 heteroatoms, selected from nitrogen, sulfur, phosphorus, and/or oxygen within at least one ring. Heterocyclic groups may be substituted.

The term "ester" refers to chemical entities as understood in the art and in particular can include groups of the form (RCO-).

As to any of the above groups which contain one or more substituents, it is understood, that such groups do not contain any substitution or substitution patterns which are sterically impractical and/or synthetically non-feasible. The compounds of this disclosure include all novel stereochemical isomers arising from the substitution of disclosed compounds.

The invention may be further understood by the following non-limiting examples.

### EXAMPLE 1. Procaspase activating compounds.

Mutation or aberrant expression of proteins in the apoptotic cascade is a frequent hallmark of cancer. These changes can prevent proapoptotic signals from being transmitted to the executioner caspases, thus preventing apoptotic cell death and allowing cellular proliferation. Caspase-3 and caspase-7 are key executioner caspases, existing as inactive zymogens that are activated by upstream signals. Importantly, expression levels of procaspase-3 are significantly higher in certain cancerous cells relative to non-cancerous controls. Here we report the identification of small molecules that directly activate procaspase-3 to active caspase-3. A particular compound, PAC-1, effects activation in vitro with an EC₅₀ on the order of 220 nanomolar and induces apoptosis in a multitude of cancerous cell lines.

In contrast to many known anti-cancer drugs, cells treated with PAC-1 show an immediate activation of procaspase-3, and the toxicity of PAC-1 is shown to be directly proportional to the amount of procaspase-3 contained in a cell. Thus PAC-1 directly activates procaspase-3 to caspase-3 in vivo, allowing this compound to induce apoptosis even in cells that have defective apoptotic machinery. PAC-1 is the first small molecule known to directly activate procaspase-3; the direct activation of executioner caspases is a novel anti-cancer strategy that may prove beneficial in a variety of cancers, including the many cancers in which procaspase-3 is upregulated.

A collection of about 20,000 structurally diverse small molecules was screened for the ability to activate procaspase-3 in vitro. Procaspase-3 was expressed and purified in E. coli (Roy et al., 2001). Procaspase-3 (at a concentration of 50 ng/mL) was added to the wells of a 384-well plate, and the compounds were added to a final concentration of approximately 40 µM. Each plate was then incubated for two hours at 37°C, after which the caspase-3 peptidic substrate Ac-Asp-Glu-Val-Asp-p-nitroanilide (Ac-DEVD-pNa) was added to a concentration of 200 µM. The formation of the p-nitroaniline chromophore was followed at 405 nm over the course of two hours.

Of the compounds evaluated, four induced a significant increase over background in the hydrolysis of the peptidic caspase-3 substrate. Of those four, one showed a strong dose-dependent effect on in vitro procaspase-3 activation. As shown in Figure 1A, this first procaspase activating compound (PAC-1) gives half-maximal activation of procaspase-3 at a concentration of 0.22 µM. This compound is not simply increasing the activity of caspase-3 itself, as it has no effect on the catalytic activity of the fully processed caspase-3 enzyme (Figure 1A).

Procaspase-3 has an N-terminal pro domain (residues 1-28), followed by a large subunit (17 kDa) and a small subunit (12 kDa) that are separated by an intersubunit linker (Pop et al., 2003). In vivo, two procaspase-3 monomers assemble to form a catalytically inactive homodimer that can be activated by cleavage at D175 in the intersubunit linker. The precise role of the pro domain is unclear, and it has been shown that cleavage in the intersubunit region alone is sufficient for full catalytic activity (Stennicke, H. R. et al., 1998). Although procaspase-3 is catalytically competent, it is highly resistant to autoactivation due to the presence of the 12 amino acid safety catch; however, when the safety catch is mutated significant autoactivation of procaspase-3 is observed (Roy et al., 2001). Compounds that interact with this important regulatory region or at other positions can allow the autoactivation of procaspase-3.

To directly assess the ability of PAC-1 to catalyze the autoactivation of procaspase-3, the procaspase-3 protein was incubated with 100 µM of PAC-1 for time points ranging from one to five hours. As shown by the Western blot in Figure 1B, PAC-1 induces the cleavage of procaspase-3 in a time- dependent fashion, with >50% processing observed after 4 hours. In contrast, procaspase-3 incubated in buffer shows virtually no autoactivation over that same time span. In an attempt to pinpoint the region of procaspase-3 with which PAC-1 is interacting, alanine substitutions were made in the key aspartic acid triad in the safety catch region, residues Asp179, Asp180 and Asp181. Mutations at these positions all dramatically decreased the ability of PAC-1 to activate procaspase-3, with certain mutations more detrimental to activation of procaspase-3 by PAC-1 (Figure 2A).

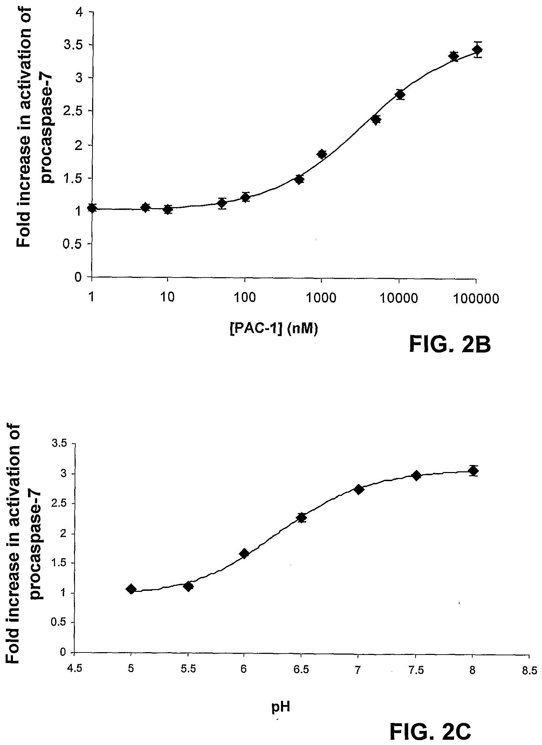

Like caspase-3, caspase-7 also exists as an inactive zymogen that is activated by proteolysis. Caspase-3 and caspase-7 are both executioner caspases and have considerable sequence and structural homology (Denault, J.-B. et al., 2003). Procaspase-7 may also have a similar safety catch region, although it has only two aspartic acids in the key triad (Asp-Thr-Asp), instead of three. As indicated by the data in Figure 2B, PAC-1 can also activate procaspase-7, although in a less efficient manner than its activation of procaspase-3 (EC₅₀ of 4.5 µM versus 0.22 µM for procaspase-3 activation). The potency of procaspase-7 activation by PAC-1 is similar to its effect on the Asp-Ala-Asp mutant of procaspase-3 (EC₅₀ = 2.77 µM). The effect of PAC-1 is abolished at low pH values where procaspase-3 undergoes rapid autoactivation (Figure 2C).

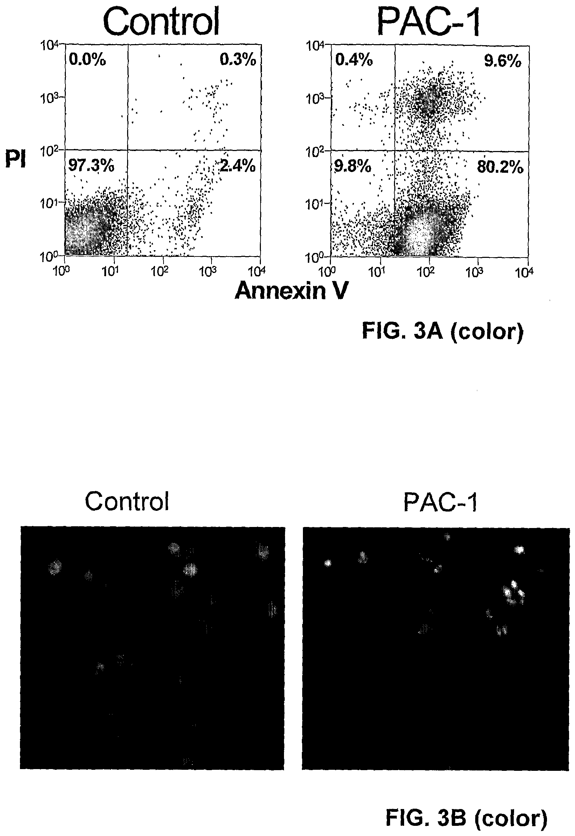

The ability for a small molecule that activates procaspase-3 to induce apoptosis in human cell lines was tested, and PAC-1 was found to induce apoptosis in a variety of cancer cell lines. In HL-60 cells addition of PAC-1 leads to considerable phosphatidylserine exposure on the cell membrane accompanied by significant chromatin condensation (Figures 3A and B). In addition, the compound induces cleavage of PARP-1 (as assessed by an in vivo PARP activity assay; Putt KS et al., 2005) and causes mitochondrial membrane depolarization (see below). Significant cellular blebbing was also observed by microscopy. Furthermore, the toxicity of PAC-1 could be abolished in the presence of the caspase inhibitor z-VAD-fmk (data not shown; see Slee et al., 1996).

If PAC-1 is indeed inducing apoptosis via direct activation of procaspase-3, the time course of apoptotic events should be altered relative to that observed with standard proapoptotic agents. Etoposide is well known to induce apoptosis through the intrinsic pathway; thus, mitochondrial membrane depolarization is followed by procaspase-3 activation in etoposide-treated cells. Indeed, in HL-60 cells treated with 10 µM etoposide, mitochondrial membrane depolarization is observed, followed by detection of caspase-3-like activity (Figure 4A). In contrast, treatment of cells with PAC-1 gives a markedly different result. With PAC-1, the first observed biochemical hallmark of apoptosis is caspase-3-like enzymatic activity. This activity is noted within minutes of compound addition, and 50% activation takes place in just over 2 hours and well before any significant mitochondrial membrane depolarization (Figure 4B). In addition, PARP activity is rapidly reduced in cells treated with PAC-1, whereas this reduction is observed at later time points in etoposide treated cells (Figure 4C). Control experiments show that PAC-1 does not directly inhibit enzymatic activity of PARP-1. In the typical sequence of apoptotic events, the mitochondrial membrane depolarizes, caspases are activated, and caspase substrates (such as PARP-1) are cleaved. The observation that cells treated with PAC-1 show a rapid activation of caspase-3/-7 (before mitochondrial membrane depolarization) and a rapid cleavage of a caspase substrate is indicative of this compound exerting its cellular toxicity through the direct activation of procaspase-3.

To further define the potency of PAC-1, the ability of this compound to induce cell death in cancer cell lines with varying levels of procaspase-3 was assessed. A determination was made of the amount of procaspase-3 present in multiple cancer cell lines (leukemia, lymphoma, melanoma, neuroblastoma, breast cancer, lung cancer and renal cancer) and in the white blood cells isolated from the bone marrow of a healthy donor. The IC₅₀ values for cell death induction were obtained for PAC-1 in these cell lines. The combined data shows a strong correlation between cellular concentration of procaspase-3 and sensitivity to PAC-1 **(****Figure 4D****).** Notably, the white blood cells derived from the bone marrow of a healthy human donor are among those with the lowest amount of procaspase-3, and PAC-1 is comparatively less toxic to these cells. PAC-1 is most potent versus the lung cancer cell line NCI-H226, with an IC₅₀ of 0.35 µM. In accordance with data in the literature (Svingen et al., 2004), we found this cell line to have a concentration of procaspase-3 that is greater than five times that of the non-cancerous control.

In contrast to these experiments with PAC-1, etoposide showed no such correlation between potency in cell culture and cellular levels of procaspase-3. For instance, etoposide was ineffective (IC₅₀ >50 µM) in inducing death in three of the melanoma cell lines (UACC-62, CRL-1872, and B16-F10), the breast cancer cell line (Hs 578t), and the lung cancer cell line (NCI-H226); these cell lines have procaspase-3 levels of 1.0, 2.4, 1.9, 3.7, and 5.3, respectively. Etoposide was effective (IC₅₀ <1 µM) versus HL-60, U-937, SK-N-SH and PC-12, which have procaspase-3 levels of 4.3, 4.0, 4.7, and 4.4, respectively. Thus, overall there is no correlation between procaspase-3 levels and IC₅₀ for etoposide.

Cancerous cells typically have a reduced sensitivity to proapoptotic signals due to the mutation or aberrant expression of an assortment of proteins in the apoptotic cascade. As such, many types of cancer are notoriously resistant to not only the endogenous signals for apoptotic cell death, but also to chemotherapeutic agents that act through similar mechanisms. The paradoxical upregulation of procaspase-3 expression levels in certain cancers provides an opportunity to use this existing intracellular pool of protein to directly induce apoptosis, thus bypassing the often non-functional or compromised upstream portion of the cascade. Although procaspase-3 is notorious for its relative inability to undergo autoactivation, it is dependent upon a 12 amino acid safety catch to keep itself in the inactive state. PAC-1 induces the autoactivation of procaspase-3 in vitro, and this activation is greatly diminished by mutation of the key tri-aspartate region of the safety catch. This data is consistent with the notion that PAC-1 is directly interfering with the ability of the safety catch to maintain procaspase-3 dormancy.

In cell culture, PAC-1 treatment induces rapid caspase-3-like activity. It is likely that the caspase-3 mediated cleavage of anti-apoptotic proteins (Bcl-2, Bcl-XL, etc.) then induces depolarization of the mitochondrial membrane and amplifies apoptosis. Further, the potency of PAC-1 toward a variety of cancer cell lines is directly proportional to the concentration of procaspase-3 in the cell. It is worth noting that several of the cell lines that PAC-1 is effective against have faulty apoptotic pathways that make them resistant to apoptosis; for instance, Apaf-1 expression is dramatically decreased in SK-MEL-5 cells, and Bcl-2 is overexpressed in the NCI-H226 lung cancer cell line.

Data presented herein fully support the notion that procaspase-3 activating compounds can be exceedingly effective against common cancers. The effectiveness can be enhanced for situations in which procaspase-3 levels are aberrantly high.

Assessment of procaspase-3 levels in cancer biopsies can be simple and rapid; as such, the potential effectiveness of a compound such as PAC-1 can be assessed a priori with a high degree of accuracy. Procaspase-3 activators and methods herein thus provide personalized medicine strategies that can be preferential to therapies that rely on general cytotoxins in the realm of anti-cancer treatments.

### MATERIALS AND METHODS

Materials: Ni-NTA resin and anti-Penta His Alexa Fluor 647 antibody was purchased from Qiagen (Valencia, CA). Bradford dye was purchased from Bio-Rad (Hercules, CA). Pin transfer devices were purchased from V & P Scientific (San Diego, CA). The reagent z-vad-fmk was purchased from Calbiochem (San Diego, CA). Rosetta E. coli was purchased from Novagen (Madison, WI). Anti-caspase-3 antibody was purchased from Sigma (St. Louis, MO). Annexin V Alexa Fluor 488 conjugate, JC-9, and propidium iodide were purchased from Molecular Probes (Eugene, OR). IPTG and MTS/PMS CellTiter 96 Cell Proliferation Assay reagent were purchased from Promega (Madison, WI). Fetal Bovine Serum was purchased from Biomeda (Foster City, CA). 96 and 384-well microtiter plates, microscope slides, microscope coverslips, horse serum and all other reagents were purchased from Fisher (Chicago, IL).

Methods: Cell Culture Conditions. U-937, HL-60, CRL-1872, ACHN, NCI-H226, SK-MEL-5 and UACC-62 cells were grown in RPMI 1640 media supplemented with 10% FBS. SK-N-SH, B16-F10 and Hs 578t cells were grown in Eagle's minimal essential medium with Earle's BSS, 1.5 g/L sodium bicarbonate and supplemented with 10% FBS. PC-12 cells were grown in RPMI 1640 media supplemented with 5% FBS and 10% horse serum. Human bone marrow was grown in IDMEM supplemented with 40% FBS. All cell lines were incubated at 37°C in a 5% CO₂, 95% air atmosphere. U-937 and HL-60 cells were split every two to three days as needed. Human bone marrow was thawed from frozen stock and immediately diluted and used for experiments. All other cells were split when they reached approximately 80% confluency.

Protein Expression and Purification. 1 mL of an overnight culture of Rosetta E. coli containing the procaspase-3 or procaspase-7 expression plasmid was seeded into 1 L of LB media containing proper antibiotic. Cells were induced with 1 mM IPTG for 30 minutes. Cells were then spun down and re-suspended in NTA binding buffer (150 mM NaCl, 50 mM Tris, 10 mM Imidazole, pH 7.9). The cells were lysed by passing twice through a French press. The cell lysate was then spun at 14,000 x g for 30 min. The supernatant was decanted and 1 mL of nickel-NTA resin was added. The cell lysate was incubated for 1 hour at 4°C. The resin was loaded on a column, washed with 10 mL NTA binding buffer followed by 10 mL NTA wash buffer (150 mM NaCl, 50 mM Tris, 20 mM Imidazole, pH 7.9). The proteins were eluted in 1 mL fractions with 10 mL of NTA elution buffer (150 mM NaCl, 50 mM Tris, 250 mM Imidazole, pH 7.9). Fractions containing protein were pooled and the amount of protein was determined using the Bradford assay.

Library Screen. Isolated procaspase-3 was diluted to 50 ng/mL in caspase assay buffer (50 mM HEPES, 100 mM NaCl, 10 mM DTT, 0.1 mM EDTA, 0.1% CHAPS and 10% glycerol, pH 7.4). 45 µL of the procaspase-3 solution was added to each well of a Nunc 384-well flat bottom microtiter plate. Approximately 20,000 compounds were screened. About 6,000 of the compounds were collected from various sources within the department of chemistry at the University of Illinois; their structures are available at: http://www.scs.uiuc.edu/∼phgroup/comcollections.html. The other approximately 14,000 compounds were purchased from Chembridge Corporation (San Diego, California). PAC-1 was a member of the compounds purchased from Chembridge Corporation.

The compounds, made up as 10 mM stock solutions in DMSO, were transferred into the wells using a 384-pin transfer apparatus that transfers 0.2 µL of compound. This yielded a final compound concentration of about 40 µM. Controls were performed in which only DMSO (containing no compound) was pin-transferred. The plates were then incubated for 2 hours at 37°C. 5 µL of a 2 mM solution of Ac-DEVD-pNA (N-acetyl-ASP-Glu-Val-Asp-p-nitroanilide) in caspase assay buffer was added to each well. The plate was then read every 2 minutes at 405 nm for 2 hours in a Spectra Max Plus 384 plate reader (Molecular Devices, Sunnyvale CA). The slope of the linear portion for each well was used to determine the activity of caspase-3.

Activation curves. The dose dependence of procaspase-3 activators was determined by adding various concentrations of compound to 90 µL of a 50 ng/mL procaspase-3, active caspase-3, procaspase-7 or active caspase-7 in caspase assay buffer in a 96-well plate. The plate was then incubated for 12 hours at 37°C. 10 µL of a 2 mM solution of Ac-DEVD-pNA in caspase assay buffer was then added to each well. The plate was read every 2 minutes at 405 nm for 2 hours in a Spectra Max Plus 384 well plate reader. The slope of the linear portion for each well was determined and the fold increase in activation from non-treated control wells was calculated.

PAC-1 activation gel. Procaspase-3 was expressed and isolated exactly as above. Procaspase-3 was diluted to about 50 µg/mL in caspase assay buffer. The procaspase-3 was then incubated in the presence or absence of 100 µM PAC-1 for varying times at 37°C. After this incubation, an equal volume of load buffer (150 mM NaCl, 50 mM Tris, 2% SDS, 20% glycerol, pH 8.0) was added to each procaspase-3 sample. All samples were then stored at -80°C until the time-course was completed. All samples were then incubated at 95°C for 5 minutes and run on a 12% SDS-PAGE gel. Proteins were then transferred to nitrocellulose paper overnight. Blots were washed in TTBS (150 mM NaCl, 50 mM Tris, 0.1% Tween-20, pH 7.4) and blocked with a 10% milk solution for 2 hours. Blots were then incubated in a 1:5000 dilution of anti-Penta His Alexa Fluor 647 antibody for 2 hours. The blot was then washed with TTBS and scanned on a Typhoon fluorescence scanner (Amersham Biosciences, SunnyVale CA).

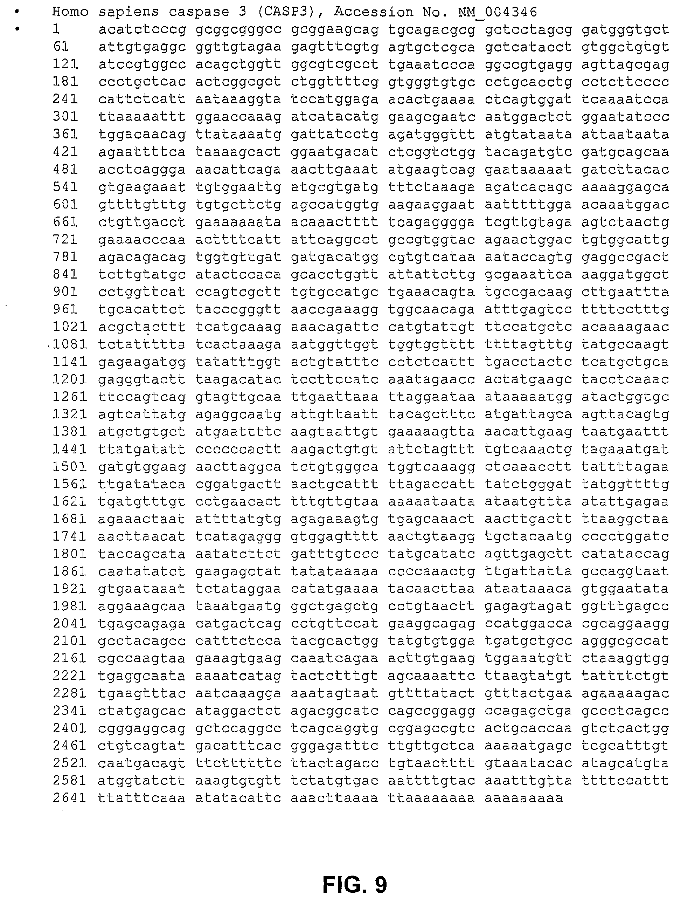

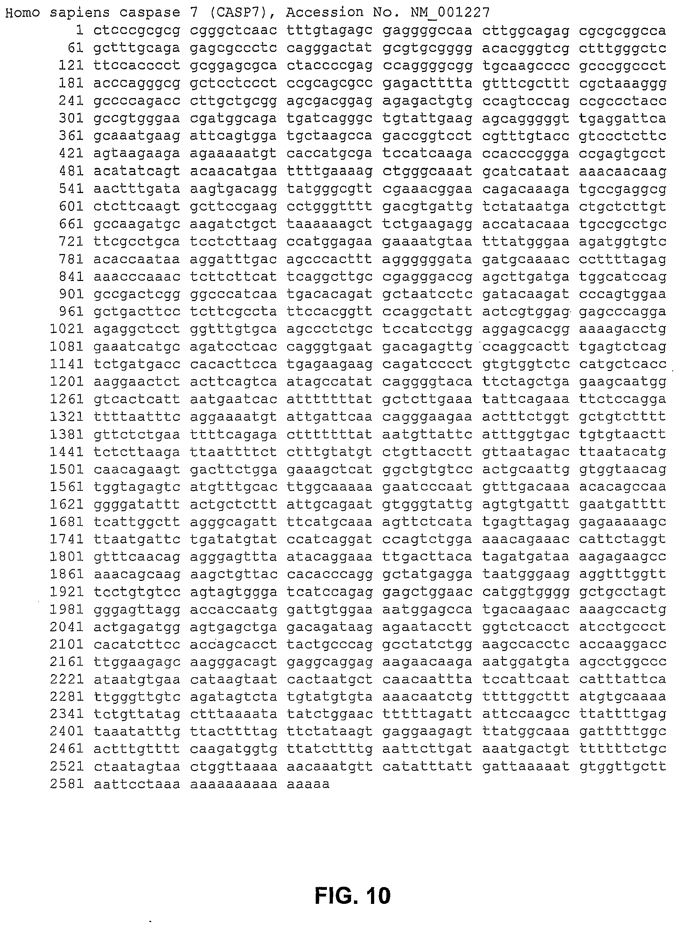

Safety catch mutations. The DDD procaspase-3 safety catch (SEQ ID NO:1; SEQ ID NO:2; SEQ ID NO:9) was mutated to ADD (SEQ ID NO:3, SEQ ID NO:4, SEQ ID NO:10), DAD (SEQ ID NO:5, SEQ ID NO:6, SEQ ID NO:11) and DDA (SEQ ID NO:7, SEQ ID NO:8, SEQ ID NO:12) using the quickchange strategy with the following primers, **gacagacagtggtgttgCGgatgacatggcgtgtcataaaatacc** (SEQ ID NO:13), **gacagacagtggtgttgatgCtgacatggcgtgtcataaaatacc** (SEQ ID NO:14) and **gacagacagtggtgttgatgatgCcatggcgtgtcataaaatacc** (SEQ ID NO:15) respectively. See also Figure 9 and Figure 10. Mutated bases are underlined and capitalized. All mutant plasmids were sequenced to ensure proper sequence throughout the gene. All mutant plasmids were expressed exactly as wild-type procaspase-3 as described above. The ability of PAC-1 to activate each procaspase-3 mutant was determined by adding various concentrations of PAC-1 to 90 µL of a 50 ng/mL wild-type procaspase-3 and mutant procaspase-3 in caspase assay buffer in a 96-well plate. The plate was then incubated for 12 hours at 37°C. 10 µL of a 2 mM solution of Ac-DEVD-pNA in caspase assay buffer was then added to each well. The plate was read every 2 minutes at 405 nm for 2 hours in a Spectra Max Plus 384 well plate reader. The slope of the linear portion for each well was determined and the fold increase in activity for each mutant was calculated.

Effect of pH on PAC-1 activation of procaspase-3. The effect of pH on procaspase-3 activation by PAC-1 was determined by diluting procaspase-3 in pH caspase assay buffer (25 mM MES, 25 mM Tris, 25 mM HEPES, 25 mM PIPES, 100 mM NaCl, 10 mM DTT, 0.1 mM EDTA, 0.1% CHAPS and 10% glycerol) to a concentration of 50 ng/mL. The buffer was then changed to various pH values and 90 µL was added to each well of a 96-well plate. PAC-1 was added to a concentration of 100 µM or DMSO was added as a control for each pH value. The plate was then incubated for 12 hours at 37°C. 10 µL of a 2 mM solution of Ac-DEVD-pNA in caspase assay buffer was then added to each well. The plate was read every 2 minutes at 405 nm for 2 hours in a Spectra Max Plus 384 plate reader (Molecular Devices, Sunnyvale CA). The slope of the linear portion for each well was determined and the fold increase in activation for each pH value was calculated.

Annexin V staining. 500 µL of media containing 200 µM PAC-1 or only DMSO as a control was added to the wells of a 24-well plate. 500 µL HL-60 cells at a concentration of 2 x 10⁶ cells/mL were then added to the 24-well plate. The plate was incubated for 20 hours at 37°C. Cells were harvested by centrifugation and washed twice in PBS. The cells were then washed in AnnexinV binding buffer (10 mM HEPES, 140 mM NaCl, 2.5 mM CaCI2, pH 7.4) and resuspended in 100 µL of Annexin V binding buffer. 5 µL of annexin V, Alexa Fluor 488 conjugate was added and the tubes were incubated at room temperature for 15 minutes protected from light. 400 µL of Annexin V binding buffer was then added, followed by the addition of 1 µL of a 1 mg/mL solution of propidium iodide. The fluorescent intensity of each cell was determined by flow cytometry at 525 nm (green channel) and 675 nm (red channel). At least 50,000 cells were analyzed in each experiment.

Condensed chromatin staining. 500 µL of media containing 200 µM PAC-1 or only DMSO as a control was added to the wells of a 24-well plate. 500 µL HL-60 cells at a concentration of 2 x 10⁶ cells/mL were then added to the 24-well plate. The cells were incubated for 20 hours and harvested by centrifugation. The cells were then washed in PBS buffer followed by the addition of ice-cold 100% ethanol. The cells were fixed overnight at 4°C. Fixed cells were incubated with 2 µg/mL Hoechst-33258 for 30 minutes at room temperature. A drop of cells was then added to a microscope slide and covered with a No. 1 thickness coverslip. Condensed chromatin was observed at 400x magnification on a Zeiss Axiovert 100 microscope.

Cell death inhibition by z-vad-fmk. 100 µL HL-60 cells at a concentration of 5 x 10⁵ cells/mL were added to the wells of a 96-well plate. The cells were then incubated for 1 hour in the presence or absence of 100 µM z-vad-fmk, a cell-permeable pan caspase inhibitor. PAC-1 was then added at various concentrations, and the cells were incubated for an additional 24 hours. Cell death was quantitated by the addition of 20 µL of the MTS/PMS CellTiter 96 Cell Proliferation Assay reagent to each well. The plates were incubated at 37°C for approximately 45 minutes until the colored product formed. The absorbance was then measured at 490 nm in a Spectra Max Plus 384 plate reader (Molecular Devices, Sunnyvale CA).

In vivo determination of mitochondrial membrane potential. 1 mL of HL-60 cells at a concentration of 1 x 10⁶ cells/mL were added to the wells of a 24-well plate. PAC-1 was then added to a concentration of 100 µM or only DMSO was added as a control. The cells were incubated for various times, and the cells then were harvested by centrifugation. The cells were washed in PBS and resuspended in 1 mL of PBS. 10 µg of the JC-9 dye was added and the cells were incubated at room temperature for 10 minutes protected from light. The cells were then washed two times with PBS and brought up in 500 µL PBS. The fluorescent intensity of each cell was determined by flow cytometry at 525 nm (green channel) and 675 nm (red channel). 50,000 cells were analyzed in each experiment. The shift in the red channel was then used to determine the amount of mitochondrial membrane depolarization.

In vivo determination of caspase-3 like activity. The amount of caspase-3 like protease activity was determined by the amount of Ac-DEVD-pNA (N-acetyl-ASP-Glu-Val-Asp-p-nitroanilide) cleaved per minute by cell lysates. To accomplish this, 50 µL of media containing varying concentrations of PAC-1 was added to the wells of a 96-well plate. 50 µL of HL-60 cells at a concentration of 5 x 10⁶ cells/mL were added to the plate and incubated for various times. After the incubation period, the plate was spun at 1000 x g for 5 minutes to pellet the cells. The cells were then washed with 100 µL of PBS and resuspended in 150 µL of ice cold Caspase Assay Buffer. Each well was then sonicated to lyse the cells. 90 µL of cell lysate was transferred from each well into a new plate. Ac-DEVD-pNA was added into each well to give a final concentration of 200 µM. The plate was then read every 2 minutes at 405 nm for 2 hours in a Spectra Max Plus 384 plate reader (Molecular Devices, Sunnyvale CA). The slope of the linear portion for each well was determined and the amount of Ac-DEVD-pNA cleaved per minute was calculated.

In vivo determination of PARP cleavage. The amount of PARP cleavage was determined by using an in vivo PARP activity assay. To accomplish this, 50 µL of media containing 200 µM NAD⁺ was added to the control wells of a 96-well plate. 50 µL of media containing 200 µM PAC-1 and 200 µM NAD⁺ was then added to the experimental wells. 25 µL of HL-60 cells at a concentration of 5 x 10⁶ cells/mL were then added to each well. The cells were incubated for various times and then spun at 1000 x g for 5 minutes. The cell media was removed and replaced with 50 µL Lysing PARP Buffer (50 mM Tris, 10 mM MgCI2, pH 8.0, 1% Triton X-100) containing 25 mM H₂O₂. The plate was then incubated for 60 minutes at 37°C. To determine the amount of NAD⁺ still present, 20 µL of 2 M KOH and 20 µL of a 20% (v/v) acetophenone (in ethanol) solution was added to each well of the 96-well plate. The plate was then incubated for 10 minutes at 4°C. 90 µL of an 88% (v/v) formic acid solution was added to each well of the 96-well plate. The plate was then incubated for 5 min. in an oven set to 110°C. The plate was allowed to cool and then read on a Criterion Analyst AD (Molecular Devices, Sunnyvale, CA) with an excitation of 360 nm, an emission of 445 nm and a 400 nm cutoff dichroic mirror. The fluorophore was excited using a 1000 W continuous lamp for 1.6 x 10⁵ µs with 5 reads performed per well. The number of moles of NAD⁺ cleaved per minute was then calculated and the remaining PARP activity as compared to control wells was determined.

Relative concentration of procaspase-3 in various cell lines. U-937, HL-60 and human bone marrow cells were harvested by centrifugation while all other cell lines were first trypsinized to release the cells and then harvested by centrifugation. All cells were washed in PBS and resusupended in 1 mL of ice-cold 100% ethanol. Cells were fixed overnight at 4°C. The cells were spun at 1000 x g for 5 minutes, washed with PBS and 100 µL of a 1:100 dilution of anti-caspase-3 antibody in PBS was then added. The cells were incubated for 2 hours at room temperature followed by five PBS washes. The cells were then resuspended in 1 mL of a 1:10,000 dilution of anti-mouse Ab Cy3 labeled antibody for 2 hours at room temperature protected from light. The cells were washed five times with PBS and resuspended in 500 µL of PBS. The fluorescent intensity of each cell was determined by flow cytometry at 675 nm (red channel). At least 20,000 cells were analyzed in each experiment. The median of the population was used to determine the relative concentration of procaspase-3 in each cell line.

Determination of IC₅₀ values in various cell lines. 50 µL of media containing various concentrations of PAC-1 or etoposide was added to each well of a 96-well plate except control wells, which contained only DMSO. U-937, HL-60 and human bone marrow cells were harvested by centrifugation, while all other cell lines were first trypsinized before centrifugation. Cells were then resuspended in media and diluted to either 1 x 10⁶ cells/mL for U-937, HL-60 and human bone marrow cells or 50,000 cells/mL for all other cell lines. 50 µL of the cell solutions were then added to each well and the plates were incubated for either 24 or 72 hours for etoposide and PAC-1 respectively. Cell death was quantitated by the addition of 20 µL of the MTS/PMS CellTiter 96 Cell Proliferation Assay reagent to each well. The plates were then incubated at 37°C for approximately one hour until the colored product formed. The absorbance was measured at 490 nm in a Spectra Max Plus 384 plate reader (Molecular Devices, Sunnyvale CA).

Data Analysis: The data from all flow cytometry experiments was analyzed using Summit Software (Cytomation, Fort Collins CO). All graphs were analyzed using Table Curve 2D.

Professor Ronald Hoffman (University of Illinois-Chicago Cancer Center) provided human bone marrow. Professor Guy Salvesen (Burnham Institute) provided the procaspase-3 and procaspase-7 expression vectors.

REFERENCE TO SEQUENCE LISTING - Appendix A. The separately accompanying sequence listing information, designated Appendix A, is to be considered and incorporated as part of the specification herewith.

**Table 1. Overview of Sequence Listing information.**

| **SEQ ID NO:** | **Brief Description** | **Type** |
|---|---|---|
| 1 | Procaspase-3; with amino acid DDD wild-type safety catch sequence (ACCESSION Number NM_004346) | DNA/RNA |
| 2 | automatic translation | PRT |
| 3 | procaspase-3 mutant ADD | DNA/RNA |
| 4 | automatic translation | PRT |
| 5 | procaspase-3 mutant DAD | DNA/RNA |
| 6 | automatic translation | PRT |
| 7 | procaspase-3 mutant DDA | DNA/RNA |
| 8 | automatic translation | PRT |
| 9 | procaspase-3 wild-type DDD | PRT |
| 10 | procaspase-3 mutant ADD | PRT |
| 11 | procaspase-3 mutant DAD | PRT |
| 12 | procaspase-3 mutant DDA | PRT |
| 13 | PCR primer1 | DNA |
| 14 | PCR primer2 | DNA |
| 15 | PCR primer3 | DNA |
| 16 | Procaspase-7 with amino acid DTD wild-type safety catch sequence (Accession Number NM_001227) | DNA/RNA |
| 17 | automatic translation | PRT |
| 18 | Procaspase-7 DDD wild-type safety catch sequence | DNA/RNA |
| 19 | automatic translation | PRT |
| 20 | Procaspase-7 DTD wild-type safety catch, active site C to A mutant sequence | DNA/RNA |
| 21 | automatic translation | PRT |
| 22 | Procaspase-7 DDD wild-type safety catch, active site C to A mutant sequence | DNA/RNA |
| 23 | automatic translation | PRT |
| 24 | Procaspase-7 with amino acid DTD | PRT |
| 25 | Procaspase-7 DDD wild-type safety catch sequence | PRT |
| 26 | Procaspase-7 DTD wild-type safety catch, active site C to A mutant sequence | PRT |
| 27 | Procaspase-7 DDD wild-type safety catch, active site C to A mutant sequence | PRT |

### EXAMPLE 2. Synthesis of procaspase activating compounds.

PAC-1 and other compounds are prepared according to the following schemes, e.g., Scheme 1 and/or Scheme 2. Further variations are prepared according to methods known in the art.

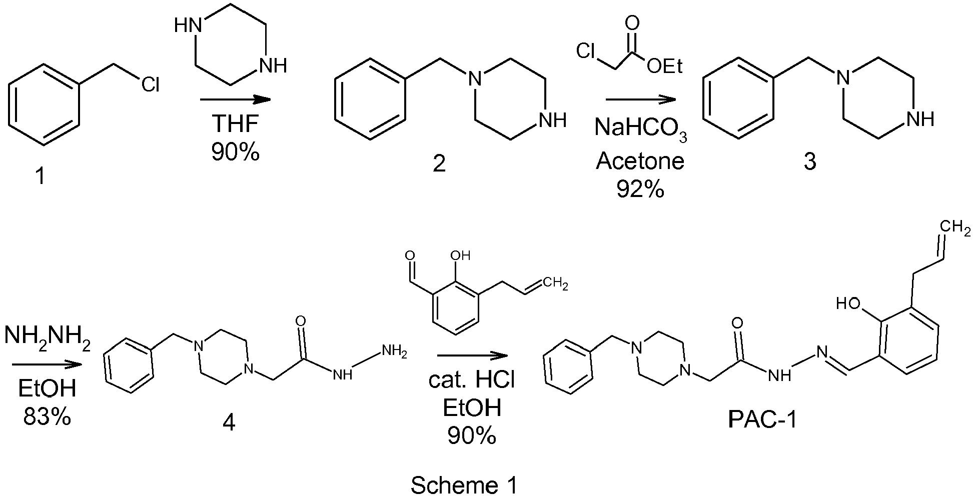

In a particular example, PAC-1 is prepared according to Scheme 2:

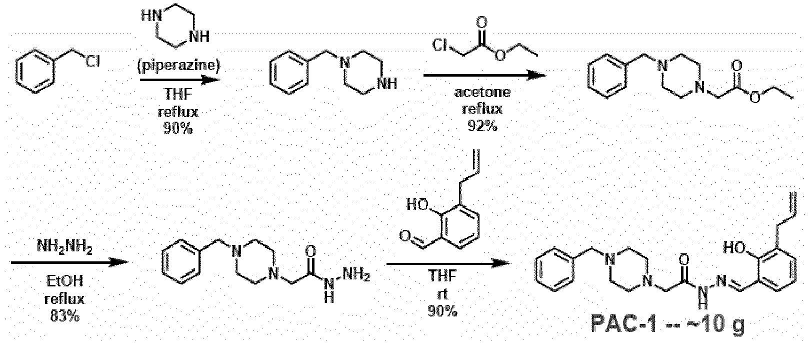

### EXAMPLE 3. Analogs of PAC-1.

Analog compounds of PAC-1 were prepared and assessed for the capability to directly activate purified procaspase-3 in vitro.

**Table 2. Activity of PAC-1 and analog compounds.**

| Compound / Structure designation | Structure | Activity |
|---|---|---|
| PAC-1 | 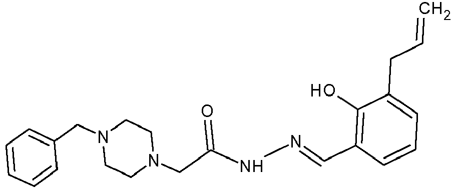 | Active |
| 5 | 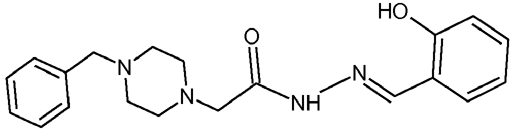 | Active |
| 6 | 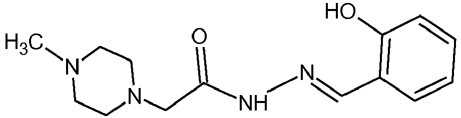 | Inactive |
| 7 | 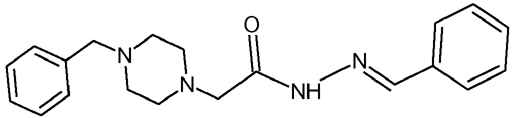 | Inactive |
| 2 | 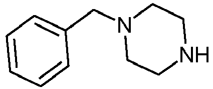 | Inactive |
| 4 | 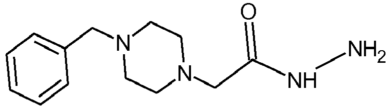 | Inactive |

### EXAMPLE 4. Pharmaceutical embodiments.

The following describes information relevant to pharmaceutical and pharmacological embodiments and is further supplemented by information in the art available to one of ordinary skill. The exact formulation, route of administration and dosage can be chosen by an individual physician in view of a patient's condition (see e.g. Fingl et. al., in The Pharmacological Basis of Therapeutics, 1975, Ch. 1 p. 1).

It should be noted that the attending physician would know how to and when to terminate, interrupt, or adjust administration due to toxicity, or to organ dysfunctions, etc. Conversely, the attending physician would also know to adjust treatment to higher levels if the clinical response were not adequate (in light of or precluding toxicity aspects). The magnitude of an administered dose in the management of the disorder of interest can vary with the severity of the condition to be treated and to the route of administration. The severity of the condition may, for example, be evaluated, in part, by standard prognostic evaluation methods. Further, the dose and perhaps dose frequency, can also vary according to circumstances, e.g. the age, body weight, and response of the individual patient. A program comparable to that discussed above also may be used in veterinary medicine.

Depending on the specific conditions being treated and the targeting method selected, such agents may be formulated and administered systemically or locally. Techniques for formulation and administration may be found in Alfonso and Gennaro (1995) and elsewhere in the art. Suitable routes may include, for example, oral, rectal, transdermal, vaginal, transmucosal, or intestinal administration; parenteral delivery, including intramuscular, subcutaneous, or intramedullary injections, as well as intraocular, intrathecal, intravenous, or intraperitoneal administration.

For injection or other routes, agents of the invention may be formulated in aqueous solutions, preferably in physiologically compatible buffers such as Hanks' solution, Ringer's solution, water for injection, physiological saline buffer, or other solution. For transmucosal administration, penetrants appropriate to the barrier to be permeated can be used in the formulation. Such penetrants are generally known in the art.

Use of pharmaceutically acceptable carriers to formulate the compounds herein disclosed into dosages suitable for systemic or other administration is also disclosed. With proper choice of carrier and suitable manufacturing practice, the compositions of the present disclosure, in particular those formulated as solutions, may be administered parenterally, such as by intravenous injection, or other routes. Appropriate compounds can be formulated readily using pharmaceutically acceptable carriers well known in the art into dosages suitable for oral administration. Such carriers enable the compounds to be formulated as tablets, pills, capsules, liquids, gels, syrups, slurries, elixirs, solutions, suspensions and the like, e.g. for oral ingestion by a patient to be treated. For other routes, formulations can be prepared for creams, ointments, lotions, and the like.

Agents intended to be administered intracellularly may be administered using techniques well known to those of ordinary skill in the art. For example, such agents may be encapsulated into liposomes, other membrane translocation facilitating moieties, or other targeting moieties; then administered as described above. Liposomes can include spherical lipid bilayers with aqueous interiors. All molecules present in an aqueous solution at the time of liposome formation can be incorporated into the aqueous interior. The liposomal contents are both protected from the external microenvironment and, because liposomes fuse with cell membranes, are efficiently delivered into the cell cytoplasm. Additionally, due to hydrophobicity attributes, small organic molecules may be directly administered intracellularly.

Pharmaceutical compositions suitable for use in the present invention include compositions wherein the active ingredients are contained in an effective amount to achieve the intended purpose. Determination of the effective amounts is well within the capability of those skilled in the art, especially in light of the disclosure provided herein and other information in the art.

In addition to the active ingredients, these pharmaceutical compositions may contain suitable pharmaceutically acceptable carriers comprising excipients and auxiliaries which facilitate processing of the active compounds into preparations which can be used pharmaceutically. The preparations formulated for oral administration may be in the form of tablets, dragees, capsules, or solutions, including those formulated for delayed release or only to be released when the pharmaceutical reaches the small or large intestine.

The pharmaceutical compositions of the present invention may be manufactured in a manner that is itself known, e.g., by means of conventional mixing, dissolving, suspending, granulating, dragee-making, levitating, emulsifying, encapsulating, entrapping, lyophilizing, and other processes.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Pharmaceutical preparations for oral use can be obtained by combining the active compounds with solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate.

Dragee cores are optionally provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added.

### EXAMPLE 5. Direct Induction of Apoptosis in Cancer Cells with a Small Molecule Activator of Procaspase-3.

ABSTRACT: Mutation or aberrant expression of proteins in the apoptotic cascade is a hallmark of cancer. These changes prevent proapoptotic signals from being transmitted to the executioner caspases, thus preventing apoptotic cell death and allowing cellular proliferation. Caspase-3 and caspase-7 are the key executioner caspases, existing as inactive zymogens that are activated by upstream signals. Importantly, levels of procaspase-3 are significantly higher in certain cancerous cells relative to non-cancerous controls. Here we report the identification of a small molecule (PAC-1) that directly activates procaspase-3 to active caspase-3 *in vitro* with an EC₅₀ of 220 nanomolar, and induces apoptosis in a variety of cancer cell lines. In contrast to many known anti-cancer drugs, cells treated with PAC-1 show an immediate activation of procaspase-3, and the efficacy of PAC-1 is shown to be proportional to the amount of procaspase-3 contained in a cell. Derivatives of PAC-1 that do not activate procaspase-3 *in vitro* also have no proapoptotic activity. Cancerous cells isolated from primary colon tumors are considerably more sensitive to apoptotic induction by PAC-1 than the cells from adjacent non-cancerous tissue from the same patient; these cancerous cells contain on average about 7-fold more procaspase-3 than the cells from the adjacent non-cancerous primary tissue. In addition, the sensitivity to PAC-1 of the primary cells from the colon cancer tumors strongly correlates with the level of the procaspase-3 target. Finally, PAC-1 as a single entity was shown as active to retard the growth of tumors in three different mouse models, including two models where PAC-1 was administered orally. Thus PAC-1 directly activates procaspase-3 to caspase-3 *in vivo*, thereby allowing this compound to induce apoptosis even in cells that have defective apoptotic machinery. PAC-1 is the first small molecule known to directly activate procaspase-3; the direct activation of executioner caspases is an anti-cancer strategy that may prove beneficial in the many cancers in which procaspase-3 levels are elevated.

INTRODUCTION. A hallmark of cancer is its resistance to natural apoptotic signals. Depending on the cancer type, this resistance is typically due to either up- or down-regulation of key proteins in the apoptotic cascade, or to mutations in genes encoding these proteins. Such changes occur in both the intrinsic apoptotic pathway, which funnels through the mitochondria and caspase-9, and the extrinsic apoptotic pathway, which involves the action of death receptors and caspase-8. For example, alterations in proper levels of p53, Bim, Bax, Apaf-1, FLIP and many others have been observed in cancers and lead to a defective apoptotic cascade, one in which the upstream pro-apoptotic signal is not properly transmitted to activate the executioner caspases, caspase-3 and caspase-7. As most apoptotic pathways ultimately involve the activation of procaspase-3, these genetic abnormalities are effectively "breaks" in the apoptotic circuitry, and as a result such cells proliferate uncontrolled.

Given the central role of apoptosis in cancer, efforts have been made to develop therapeutics that target specific proteins in the apoptotic cascade. For instance, peptidic or small molecule binders to p53, proteins in the Bcl family, or to the lAPs have pro-apoptotic activity, as do compounds that promote the oligomerization of Apaf-1. However, because many of these compounds target early or intermediate positions on the apoptotic cascade, cancers with mutations in downstream proteins will likely be resistant to their effects. For therapeutic purposes it would be ideal to identify a small molecule that directly activates a proapoptotic protein far downstream in the apoptotic cascade. In addition, such a therapeutic strategy would have a higher likelihood of success if levels of that proapoptotic protein were elevated in cancer cells.

The conversion of procaspase-3 to caspase-3 results in the generation of the active "executioner" caspase that subsequently catalyzes the hydrolysis of a multitude of protein substrates. Active caspase-3 is a homodimer of heterodimers and is produced by proteolysis of procaspase-3. *In vivo,* this proteolytic activation typically occurs through the action of caspase-8 or caspase-9. To ensure that this zymogen is not prematurely activated, procaspase-3 has a tri-aspartic acid "safety catch" that blocks access to the IETD site of proteolysis. This safety catch enables procaspase-3 to resist autocatalytic activation and proteolysis by caspase-9. The position of the safety catch is sensitive to pH; thus, upon cellular acidification (as occurs during apoptosis) the safety catch is thought to allow access to the site of proteolysis, and active caspase-3 can be produced either by the action of caspase-9 or through an autoactivation mechanism.

Cells from certain types of cancerous tissue have elevated levels of procaspase-3. A study of primary isolates from 20 colon cancer patients revealed that on average procaspase-3 was elevated six-fold in such isolates relative to adjacent non-cancerous tissue. In addition, procaspase-3 levels are elevated in certain neuroblastomas, lymphomas, and liver cancers. In fact, a systematic evaluation of procaspase-3 levels in the 60 cell-line panel used by the NCI revealed that particular lung, melanoma, renal, and breast cancers show greatly enhanced levels of procaspase-3. Given the central importance of active caspase-3 to successful apoptosis, the high levels of procaspase-3 in certain cancerous cell types, and the intriguing safety catch-mediated suppression of its autoactivation, we reasoned that small molecules that directly activate procaspase-3 could be identified and that such molecules could have great potential in targeted cancer therapy. In this manuscript we report the *in vitro* identification of a small molecule activator of procaspase-3, **PAC-1. PAC-1** is powerfully proapoptotic in cancer cell lines in a manner proportional to procaspase-3 levels, its proapoptotic effect is due to its direct and immediate activation of procaspase-3, and it is effective against primary colon cancer isolates and in three different mouse models of cancer.

Approximately 20,500 structurally diverse small molecules were screened for the ability to activate procaspase-3 *in vitro.* Procaspase-3 was expressed and purified in *E. coli* according to standard procedures. Procaspase-3 was added to the wells of a 384-well plate, and the compounds were added to a final concentration of about 40 µM (the final concentration of procaspase-3 was 50 ng/mL). Each plate was then incubated for two hours at 37°C, after which the caspase-3 peptidic substrate Ac-Asp-Glu-Val-Asp-*p*-nitroanilide (Ac-DEVD-*p*Na) was added to a concentration of 200 µM. The formation of the *p-*nitroaniline chromophore was followed at 405 nm over the course of two hours. Of the ∼20,500 compounds evaluated, four induced a significant increase over background in the hydrolysis of the peptidic caspase-3 substrate. Of those four, one showed a strong dose dependent effect on *in vitro* procaspase-3 activation. As shown in **Figure 1A****,** this first procaspase-activating compound **(PAC-1)** gives half-maximal activation of procaspase-3 at a concentration of 0.22 µM. This compound is not simply increasing the activity of caspase-3 itself, as it has no effect on the catalytic activity of the fully processed caspase-3 enzyme **(****Figure 1A****).**

Procaspase-3 consists of a N-terminal pro domain (residues 1-28), followed by a large subunit (17 kDa) and a small subunit (12 kDa) that are separated by an intersubunit linker.²² *In vivo*, two procaspase-3 monomers assemble to form a homodimer that can be activated by cleavage at D175 in the intersubunit linker. The precise role of the pro domain is unclear, and it has been shown that cleavage in the intersubunit region alone is sufficient for full catalytic activity. Although procaspase-3 has enough catalytic activity to drive its own proteolytic maturation, it is highly resistant to this autoactivation due to the presence of the three amino acid safety catch. However, when the safety catch is mutated significant autoactivation of procaspase-3 is observed. To directly assess the ability of **PAC-1** to catalyze the maturation of procaspase-3 to the active caspase-3, the procaspase-3 protein was incubated with 100 µM of **PAC-1** for time points ranging from one to five hours. As shown by the Western blot in **Figure 1B****, PAC-1** induces the cleavage of procaspase-3 in a time-dependant fashion, with >50% processing observed after 4 hours. In contrast, procaspase-3 incubated in buffer shows virtually no autoactivation over that same time span. **PAC-1** was also effective in this assay at a concentration of 5 µM.

Alanine substitutions were then made in the key aspartic acid triad in the safety catch region of procaspase-3, residues Asp179, Asp180 and Asp181. Mutations at these positions all dramatically decreased the ability of **PAC-1** to activate procaspase-3, with certain mutations more detrimental to activation of procaspase-3 by **PAC-1 (****Figure 2A****).** Like caspase-3, caspase-7 also exists as an inactive zymogen that is activated by proteolysis. Caspase-3 and caspase-7 are both executioner caspases and have considerable structural homology. Procaspase-7 is also predicted to have a similar safety catch region, although it has only two aspartic acids in the key triad (Asp-Thr-Asp), instead of three. As indicated by the data in **Figure 2B****, PAC-1** can also activate procaspase-7, although in a less efficient manner than its activation of procaspase-3 (EC₅₀ of 4.5 µM versus 0.22 µM for procaspase-3 activation). The potency of procaspase-7 activation by **PAC-1** is similar to its effect on the Asp-Ala-Asp mutant of procaspase-3 (EC₅₀ = 2.77 µM). As expected, the effect of **PAC-1** is abolished at low pH values where procaspase-3 undergoes rapid autoactivation **(****Figure 2C****).**

**PAC-1** was found to induce apoptosis in a variety of cancer cell lines. In HL-60 cells addition of **PAC-1** leads to considerable phosphatidylserine exposure on the cell membrane accompanied by significant chromatin condensation **(****Figures 3A, 3B****).** In addition, the compound induces cleavage of the caspase substrate PARP-1 (as assessed by an *in vivo* PARP activity assay) and causes mitochondrial membrane depolarization (see below). Significant cellular blebbing of **PAC-1** treated cells was also observed by microscopy. Furthermore, the toxicity of **PAC-1** could be abolished in the presence of the caspase inhibitor z-VAD-fmk.

If **PAC-1** is indeed inducing apoptosis via direct activation of procaspase-3, then the time course of apoptotic events should be altered relative to that observed with standard proapoptotic agents. Etoposide is well known to induce apoptosis through the intrinsic pathway; thus, mitochondrial membrane depolarization is followed by procaspase-3 activation in etoposide-treated cells. Indeed, in HL-60 cells treated with 10 µM etoposide, mitochondrial membrane depolarization is observed, followed by detection of caspase-3-like activity **(****Figure 4A****).** In contrast, treatment of cells with **PAC-1** gives a markedly different result. With this compound, the first observed biochemical hallmark of apoptosis is caspase-3-like enzymatic activity, with activity noted within minutes of **PAC-1** addition and 50% activation taking place in just over 2 hours and well before any significant mitochondrial membrane depolarization **(****Figure 4B****).** In addition, PARP-1 activity is rapidly reduced in cells treated with **PAC-1,** whereas this reduction is observed at later time points in etoposide treated cells **(****Figure 4C****);** control experiments show that **PAC-1** does not directly inhibit enzymatic activity of PARP-1. In the typical sequence of apoptotic events the mitochondrial membrane depolarizes, caspases are activated, and caspase substrates (such as PARP-1) are cleaved. The observation that cells treated with **PAC-1** show a rapid activation of caspase-3/-7 (before mitochondrial membrane depolarization) and a rapid cleavage of a caspase substrate (PARP-1) is indicative of **PAC-1** exerting its cellular toxicity through the direct activation of procaspase-3.

To further define the potency of **PAC-1,** the ability of this compound to induce cell death in cancer cell lines with varying levels of procaspase-3 was assessed. A determination was first made of the levels of procaspase-3 present in multiple cancer cell lines (leukemia, lymphoma, melanoma, neuroblastoma, breast cancer, lung cancer, adrenal cancer and renal cancer). The IC₅₀ values for cell death induction were obtained for **PAC-1** versus these cell lines. The combined data shows a strong correlation between cellular concentration of procaspase-3 and sensitivity to **PAC-1 (****Figure 4D, Figure 4E****). PAC-1** is most potent versus the lung cancer cell line NCI-H226, with an IC₅₀ of 0.35 µM. We found this cell line to have a concentration of procaspase-3 that is greater than five times that of baseline levels. Importantly, there is one cancer cell line (MCF-7, breast cancer cells) that is known to have no expression of procaspase-3. **PAC-1** has virtually no effect on MCF-7 cells, inducing death with an IC₅₀>75 µM.

In contrast, etoposide showed no such correlation between potency in cell culture and cellular levels of procaspase-3. For instance, etoposide was ineffective (IC₅₀>50 µM) in inducing death in three of the melanoma cell lines (UACC-62, CRL-1872, and B16-F10), the breast cancer cell line (Hs 578t), and the lung cancer cell line (NCI-H226); these cell lines have procaspase-3 levels of 1.0, 2.4, 1.9, 3.7, and 5.3, respectively. Etoposide was effective (IC₅₀<1 µM) versus HL-60, U-937, SK-N-SH and PC-12, which have procaspase-3 levels of 4.3, 4.0, 4.7, and 4.4, respectively. Thus, overall there is no correlation between procaspase-3 levels and IC₅₀ for etoposide.

Several derivatives of **PAC-1** were synthesized and evaluated for both their procaspase-3 activating properties and their effects on cancer cells in cell culture (Table 3). The **PAC-1** derivative that lacks the allyl group **(de-allyl PAC-1)** is able to induce procaspase-3 activation and cell death at levels similar to **PAC-1.** However, all other derivatives showed no activity in either assay. Thus, while it appears the allyl group is dispensable for biological activity, the phenolic hydroxyl and aromatic rings are all critical for **PAC-1** activity. This data is also consistent with the proposed mechanism of action of **PAC-1;** compounds that do not activate procaspase-3 *in vitro* have no proapoptotic effect on cancer cells in culture.

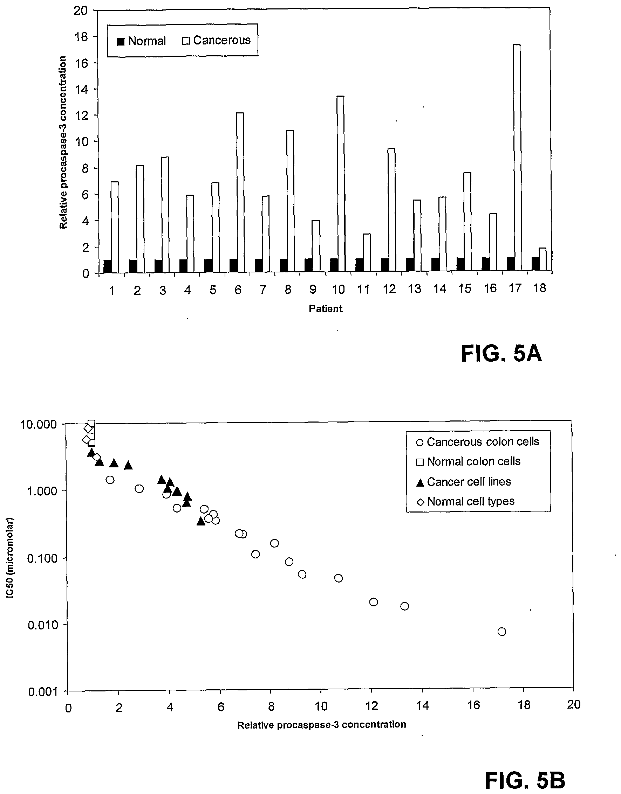

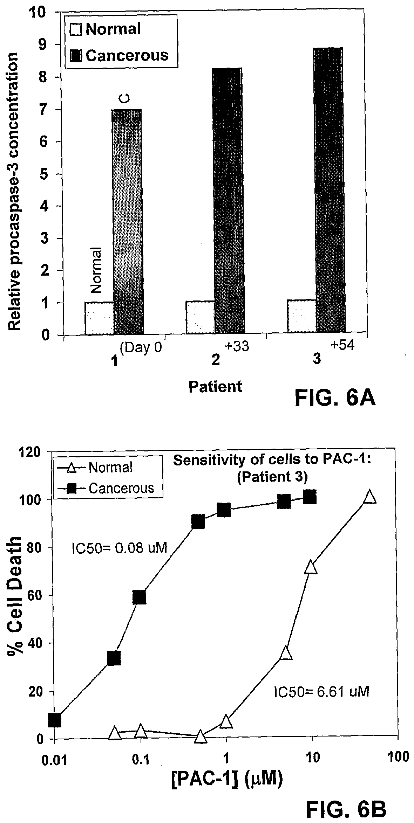

To test this direct, small molecule-mediated procaspase-3 activation strategy in clinical isolates of cancer, we obtained freshly resected colon tumors (together with adjacent non-cancerous tissue) from 18 patients from Carle Foundation Hospital (Urbana, IL). The cancerous and non-cancerous tissue was separated, and the cells derived from these were evaluated for their levels of procaspase-3 and their sensitivity to **PAC-1.** As shown in **Figure 5A****,** in all cases the cancerous cells had elevated levels (1.7- to 17.2-fold, with an average of 7.6-fold elevation) of procaspase-3 relative to the cells from the adjacent non-cancerous tissue from the same patient. Further, these cancerous cells were quite susceptible to death induction by **PAC-1. PAC-1** induced cell death in the primary cancerous cells with IC₅₀ values from 0.007-1.41 µM, while **PAC-1** induced cell death in the adjacent non-cancerous tissue with IC₅₀ values from 5.02-9.98 µM **(****Figure 5B** and Table 4). The cancerous tissue that had elevated levels of procaspase-3 was extremely sensitive to **PAC-1.** For example, **PAC-1** induced death in the cancer cells from patient 17 with an IC₅₀ of 7 nM, and these cells were over 700-fold more sensitive to **PAC-1** than cells from the adjacent normal tissue. See also **Figure 6A** showing relative procaspase-3 concentrations in normal and cancerous samples from Patients 1, 2, and 3 over a period of time of about 54 days; **Figure 6B** illustrates that cells in cancerous tissue can be greater than about 80-fold more sensitive to PAC-1 in comparison with normal tissue.

In addition to cells from the non-cancerous tissue of the 18 patients, **PAC-1** was also evaluated against four other non-cancerous cell types: white blood cells isolated from the bone marrow of a healthy donor, Hs888Lu (lung fibroblast cells), MCF-10A (breast fibroblast cells), and Hs578Bst (breast epithelial cells). Notably, the non-cancerous cell types are among those with the lowest amount of procaspase-3, and **PAC-1** is comparatively less able to induce death in these cells, with IC₅₀ values of 3.2 - 8.5 µM (**Figure 5B****,** green diamonds). As is apparent from **Figure 5B****, PAC-1** induces death in a wide variety of cell types (non-cancerous cell lines, non-cancerous primary cells, cancerous cell lines, primary cancerous cells) in a manner directly related to the level of procaspase-3. The elevation of procaspase-3 in cancerous cells allows **PAC-1** to selectively induce death in these cell types.

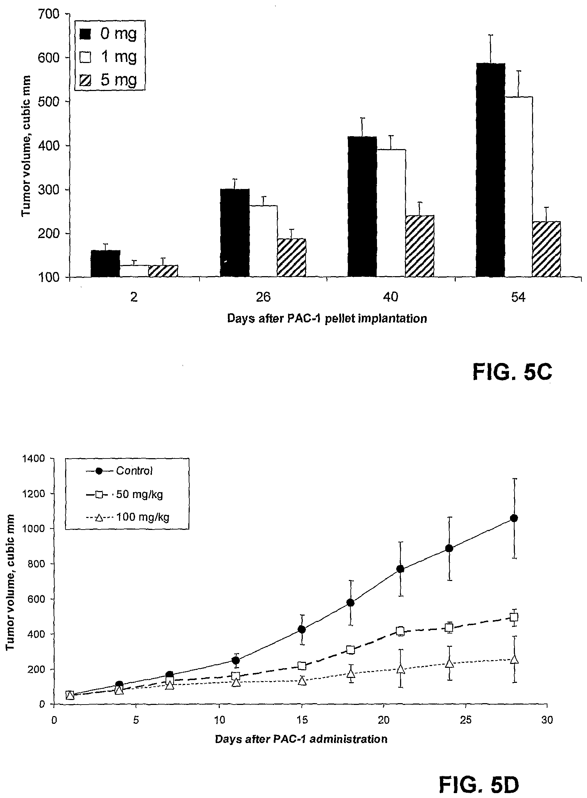

**PAC-1** was evaluated in a mouse xenograft model using a slow release mode of drug delivery. In this model, subcutaneous tumors were formed in ovariectomized female athymic BALB/c (nude) mice using the ACHN (renal cancer) cell line. Once the tumors were measured to be greater than about 30 mm², drug was administered via the implantation of a pellet of **PAC-1** and cholesterol, providing for slow and steady levels of compound release. Three groups of mice were used, with pellets containing 0 mg, 1 mg, and 5 mg of **PAC-1,** six mice per group, with four tumors per mouse. Tumor sizes were monitored for about 8 weeks. As shown in **Figure 5C****,** tumor growth is significantly retarded in the mice that were implanted with the pellet containing 5 mg of **PAC-1.** Food intake evaluation in the last week of the experiment showed no difference in food consumption between the three groups of mice. After the mice were sacrificed, plasma samples were taken from each mouse, and the **PAC-1** content of each was analyzed. For mice that received a 5 mg pellet of **PAC-1,** this analysis revealed **PAC-1** to be present at a concentration of 5 nM in the plasma after the 54 day experiment.

**PAC-1** was evaluated in a second mouse xenograft model, this one using oral administration as the drug delivery mode. In this model, subcutaneous xenograft tumors were formed in male athymic BALB/c-*nu*/*nu* mice (5 weeks old, SLC, Hamamaysu, Japan) using the NCI-H226 (lung cancer) cell line, eight mice per group, three tumors per mouse. After formation of the tumors in the mice, the mice were treated with **PAC-1** via oral gavage once a day for 21 days at a concentration of 0, 50, or 100 mg/kg and sacrificed 1 week later. As clearly indicated by the graph in **Figure 5D****,** oral administration of **PAC-1** significantly retards tumor growth in a dose-dependent manner.

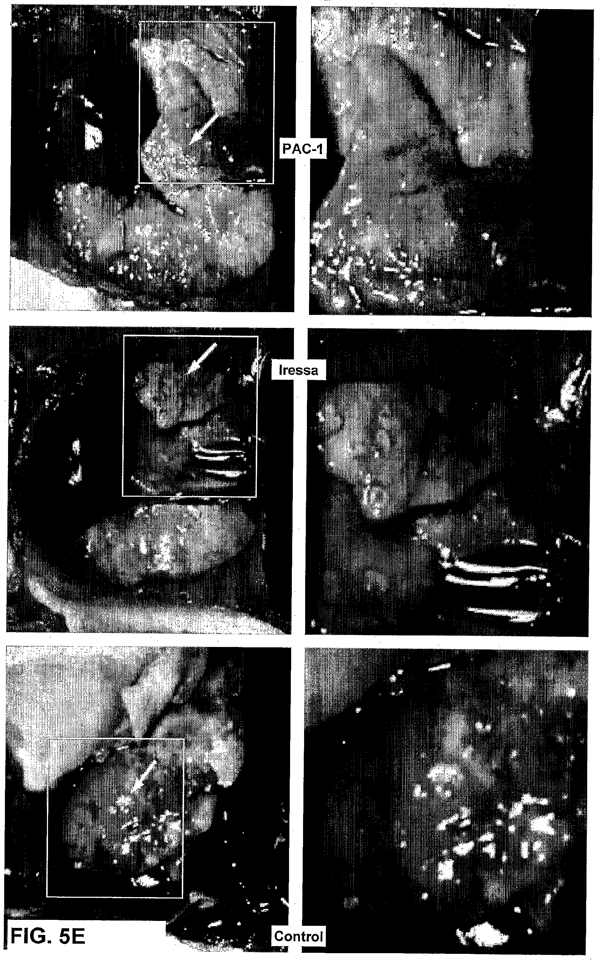

Finally, **PAC-1** was evaluated in a mouse model where the NCI-H226 cells were injected into male athymic BALB/c-*nu*/*nu* mice via tail vein injection. The total experiment lasted 28 days; the mice were treated once a day with PAC-1 (100 mg/kg) via oral gavage on days 1-4 and 7-11. On other days the mice did not receive PAC-1. A second group of mice received only vehicle. After 28 days the mice were sacrificed, and their lungs were examined. As shown in **Figure 5E****,** there is a clear difference between the lung of the control mouse (with obvious gray tumor mass) and the lung of the PAC-1 treated mouse. Results are also shown in a panel from an animal treated with gefitinib (Iressa™; AstraZeneca).

Cancerous cells typically have a reduced sensitivity to proapoptotic signals due to the mutation or aberrant expression of an assortment of proteins in the apoptotic cascade. As such, many types of cancer are notoriously resistant to not only the endogenous signals for apoptotic cell death, but also to chemotherapeutic agents that act through similar mechanisms. The paradoxical elevation of procaspase-3 levels in certain cancers provides an opportunity to use this existing intracellular pool of protein to directly induce apoptosis, thus bypassing the often non-functional upstream portion of the cascade. **PAC-1** induces the autoactivation of procaspase-3 *in vitro.* In cell culture, **PAC-1** treatment induces rapid caspase-3-like activity. It is likely that the caspase-3 mediated cleavage of anti-apoptotic proteins (Bcl-2, Bcl-XL, etc.) then induces depolarization of the mitochondrial membrane and amplifies apoptosis. Further, the potency of **PAC-1** toward a variety of cancerous and non-cancerous cell types is proportional to the concentration of procaspase-3 in the cell. As the primary cancerous cells isolated from resected colon tumors have elevated levels of procaspase-3, these cells are considerably more sensitive to **PAC-1** than cells from adjacent non-cancerous tissue. It is worth noting that several of the cell lines against which **PAC-1** is effective have faulty apoptotic pathways that make them resistant to apoptosis; for instance, Apaf-1 expression is dramatically decreased in SK-MEL-5 cells, and Bcl-2 is overexpressed in the NCI-H226 lung cancer cell line. Finally, **PAC-1** is effective in three different mouse models of cancer, including two where **PAC-1** is administered orally.

Data presented herein support the notion that procaspase-3 activating compounds can be exceedingly effective against a variety of common cancers in which procaspase-3 levels are aberrantly high. Assessment of procaspase-3 levels in cancer biopsies is simple and rapid; as such, the potential effectiveness of a compound such as **PAC-1** can be assessed a *priori* with a high degree of accuracy. Such personalized medicine strategies can be preferential to therapies that rely on general cytotoxins and can be valuable in anti-cancer therapy.

Professor Guy Salvesen (Burnham Institute) provided the procaspase-3 and procaspase-7 expression vectors.

### FIGURE LEGENDS

**Figures 1** **and** **2****.** The structure of **PAC-1** is shown elsewhere in the specification. **Fig. 1A****)** *In vitro* activation of procaspase-3 and active caspase-3 by **PAC-1. PAC-1** activates procaspase-3 with an EC₅₀ = 0.22 µM. **Fig. 1B****)** Cleavage of procaspase-3 to active caspase-3 as induced by **PAC-1.** Procaspase-3 was recombinantly expressed in *E*. *coli* with an N-terminal His-6 tag and purified. Immunoblotting was performed with an anti-His-6 antibody. In the absence of **PAC-1** no maturation of procaspase-3 is observed. In the presence of 100 µM **PAC-1,** cleavage to generate the p19 fragment is observed within 1 h, and >50% cleavage is observed after 4 h. **PAC-1** is also effective at 5 µM in this assay. **Fig. 2A****)** Activation of mutants in the "safety catch" region of procaspase-3 by **PAC-1. PAC-1** has an EC₅₀ for activation of 0.22 µM on wild type procaspase-3 (DDD), and corresponding EC₅₀ values of 2.77 µM (DAD), 113 µM (DDA), and 131 µM (ADD) for the mutants. **Fig. 2B****) PAC-1** activates procaspase-7 with an EC₅₀ of 4.5 µM. **Fig. 2C****)** Dependence of **PAC-1** activation of procaspase-3 on pH. At low pH the safety catch is off and procaspase-3 is essentially maximally activated. Error bars represent standard deviations from the mean.

**Figures 3** **and** **4****. PAC-1** induces apoptosis in HL-60 cells. **Fig. 3A****)** Phosphatidylserine exposure (as measured by Annexin-V staining) after a 20 h treatment with 100 µM **PAC-1. PAC-1** is also effective at 5 µM in this assay (see Supporting Figure 2). **Fig. 3B****)** Chromatin condensation as visualized by Hoescht staining after a 20 h treatment with 100 µM **PAC-1.** **Fig. 4A****)** Mitochondrial membrane depolarization (MMP) and caspase-3 like activity in HL-60 cells treated with 10 µM etoposide. **Fig. 4B****)** Mitochondrial membrane depolarization (MMP) and caspase-3 like activity in HL-60 cells treated with 100 µM **PAC-1.** **Fig. 4C****) PAC-1** treatment (100 µM) induces a rapid decrease in cellular PARP activity in HL-60 cells, consistent with an immediate activation of cellular caspase-3/-7. In contrast, etoposide (10 µM) treated cells show a decrease in PARP activity at much later time points. **Fig. 4D** and **Fig. 4E****) PAC-1** induces cell death in a procaspase-3 dependant manner. For a number of diverse cancerous cell lines, the procaspase-3 levels were determined (by flow cytometry with an antibody to procaspase-3) and the IC₅₀ of **PAC-1** was measured (through a 72 h treatment with a range of **PAC-1** concentrations and quantitation using the MTS assay). **PAC-1** is quite potent (IC₅₀=0.35 µM) in the NCI-H226 lung cancer cell line known to have high levels of procaspase-3. Error bars represent standard deviations from the mean.

**Table 3. PAC-1** and **de-allyl PAC-1** activate procaspase-3 *in vitro* and induce death in cancer cells in cell culture, but other structural analogues have no procaspase-3 activating effect *in vitro* and give no induction of death in cell culture.

**Figure 5****.** **Fig. 5A****)** Procaspase-3 levels are elevated in cells derived from freshly resected colon cancer tissue. Freshly resected primary colon tumors (together with adjacent non-cancerous tissue) were obtained from 18 different patients, the cancerous and non-cancerous tissue were separated, and the procaspase-3 levels were measured for each using an antibody to procaspase-3 and flow cytometry. On average, cells from the cancerous tissue have a 7.6-fold elevation in procaspase-3 as compared to the cells derived from the adjacent non-cancerous tissue from the same patient. **Fig. 5B****) PAC-1** induces cell death in a manner proportional to the cellular level of procaspase-3. The red circles represent the primary cancerous cells from the 18 colon tumors. The black triangles represent the same cancer cell lines depicted in **Figure 4D****.** The green diamonds are four non-cancerous cell types: Hs888Lu (lung fibroblast cells), MCF-10A (breast fibroblast cells), Hs578Bst (breast epithelial cells), and white blood cells isolated from the bone marrow of a healthy donor. The blue squares are the primary non-cancerous cells isolated from the tumor margins of the 18 patients. **Table 4)** Cells derived from primary colon cancer tissue are considerably more sensitive to death induction by **PAC-1** than are cells derived from adjacent non-cancerous tissue from the same patient. **Fig. 5C****) PAC-1** reduces the growth of tumors in a xenograft model of cancer. Tumors were formed with the ACHN (renal cancer) cell line by subcutaneous injection, with six mice in each group, and four tumors per mouse. Once the tumors grew to about 30 mm², **PAC-1** was implanted as a cholesterol pellet. Error bars represent standard error from the mean. **Fig. 5D****)** Oral administration of **PAC-1** significantly retards tumor growth in a mouse xenograft model. Tumors were formed using the NCI-H226 (lung cancer) cell line by subcutaneous injection, eight mice in each group, and three tumors per mouse. **PAC-1** or vehicle was administered once a day by oral gavage on days 1-21. Error bars represent standard error from the mean. **Fig. 5E****)** Oral administration of **PAC-1** significantly retards tumor growth in an i.v. injection model. Mice were injected i.v. with the NCI-H226 (lung cancer) cell line. The mice were treated with **PAC-1** (100 mg/kg) via oral gavage following the protocol as described in the text. Images show the lungs of the mice that did not receive **PAC-1** and have a large amount of gray tumor mass on the lung. In contrast, the mice that did receive **PAC-1** have almost no visible gray matter.

**Table 3. Selected compounds indicating activity levels.**

| **Compound** | **EC₅₀ (µM) for procaspase-3 activation** | **IC₅₀ (µM) for death induction in HL-60 cells** |
|---|---|---|
| 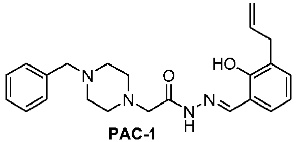 | 0.22 | 0.92 |
| 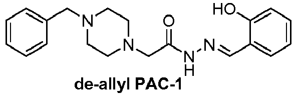 | 0.43 | 1.74 |
| 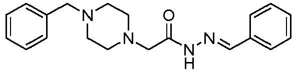 | >50 | >100 |
| 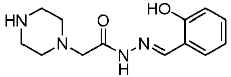 | >50 | >100 |
| 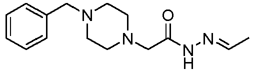 | >50 | >100 |
| 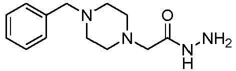 | >50 | >100 |
| 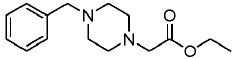 | >50 | >100 |
| 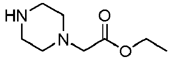 | >50 | >100 |
| 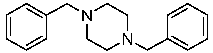 | >50 | >100 |
| 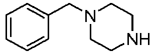 | >50 | >100 |

**Table 4. Concentration levels of PAC-1 activity in patients.**

| | PAC-1, IC₅₀ µM | |
|---|---|---|
| **Patient** | **Normal** | **Cancerous** |
| **1** | 6.78 | 0.212 |
| **2** | 9.79 | 0.154 |
| **3** | 6.61 | 0.080 |
| **4** | 9.50 | 0.340 |
| **5** | 6.88 | 0.216 |
| **6** | 6.28 | 0.020 |
| **7** | 7.34 | 0.422 |
| **8** | 5.67 | 0.045 |
| **9** | 6.54 | 0.844 |
| **10** | 9.98 | 0.017 |
| **11** | 5.94 | 1.030 |
| **12** | 5.63 | 0.052 |
| **13** | 5.50 | 0.499 |
| **14** | 7.58 | 0.366 |
| **15** | 5.96 | 0.106 |
| **16** | 5.02 | 0.527 |
| **17** | 5.17 | 0.007 |
| **18** | 6.39 | 1.410 |

### EXAMPLE 6. Testing of PAC-1 in mouse model of lung cancer.

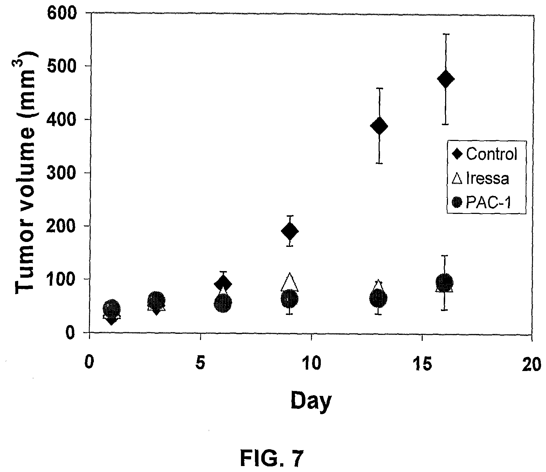

A xenograft model was employed using NCI-H226 (lung cancer) cells. PAC-1 was given intraperitoneally (i.p.) at 10 mg/kg. A comparison of efficacy was performed with gefitinib (Iressa™; AstraZeneca, Wilmington, Delaware) at 40 mg/kg using 5 mice per group. Results are shown in **Figure 7****,** indicating that PAC-1 was associated with reducing growth in tumor volume.

### EXAMPLE 7. Combinatorial derivatives, synthesis, and therapeutic use.

A number of compounds are prepared as derivatives of the PAC-1 structure. A hydrazide group is reacted with an aldehyde group to yield a combinatorial library of derivative compounds.

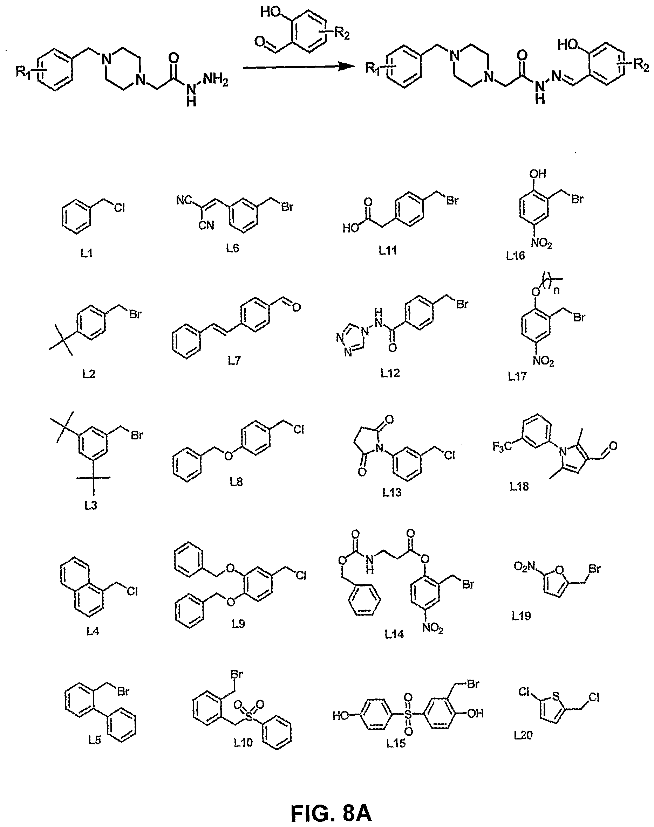

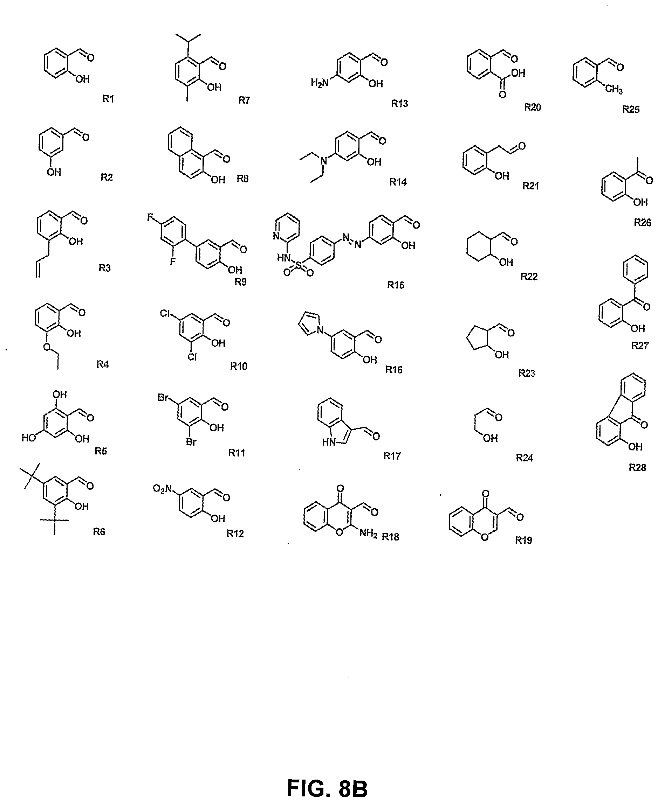

Any one of hydrazide precursor groups (AX) designated L1-L20 are used to generate hydrazides which are reacted with any one of aldehyde groups (BX) designated 1-28, thus yielding 560 PAC-1 derivative compounds. A derivative compound is synthesized using methods as described herein and according to knowledge available in the art. See the scheme and component structures below in addition to **Figure 8A** and **8B**.

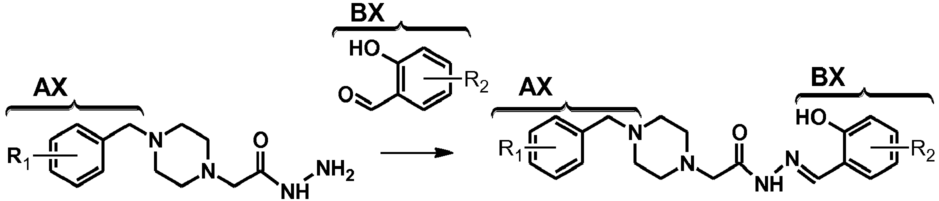

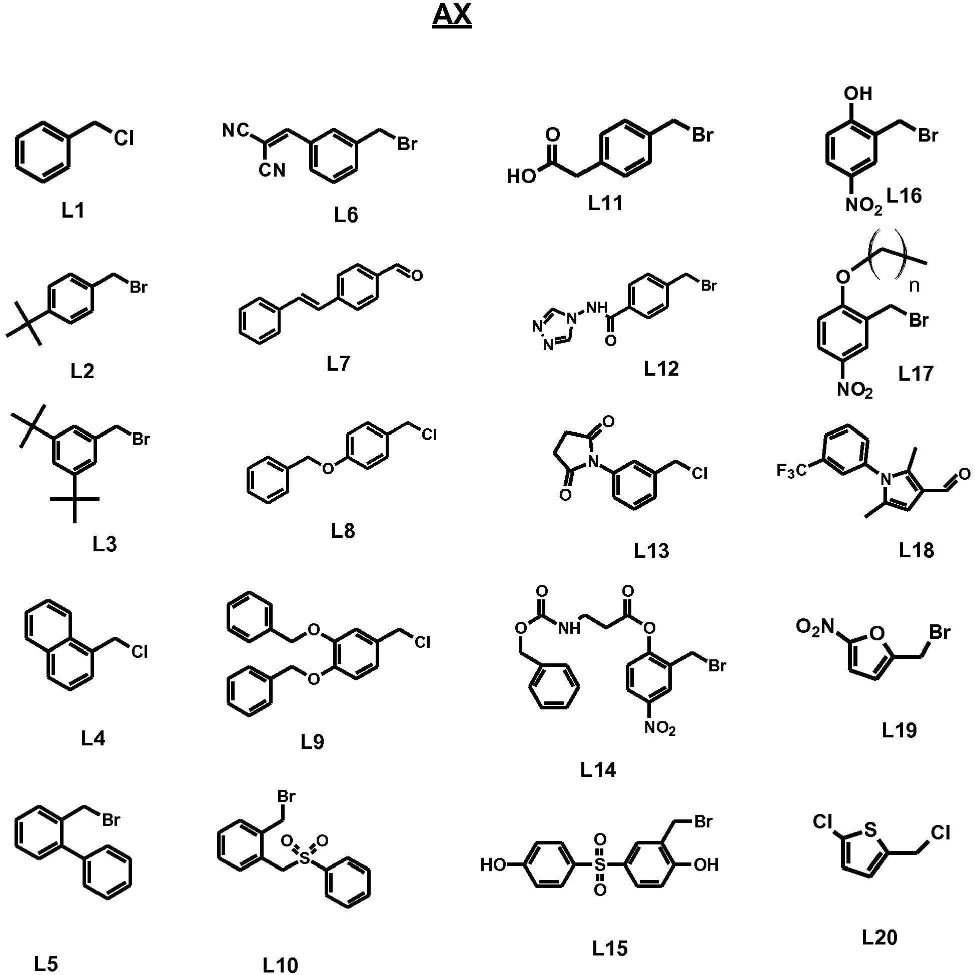

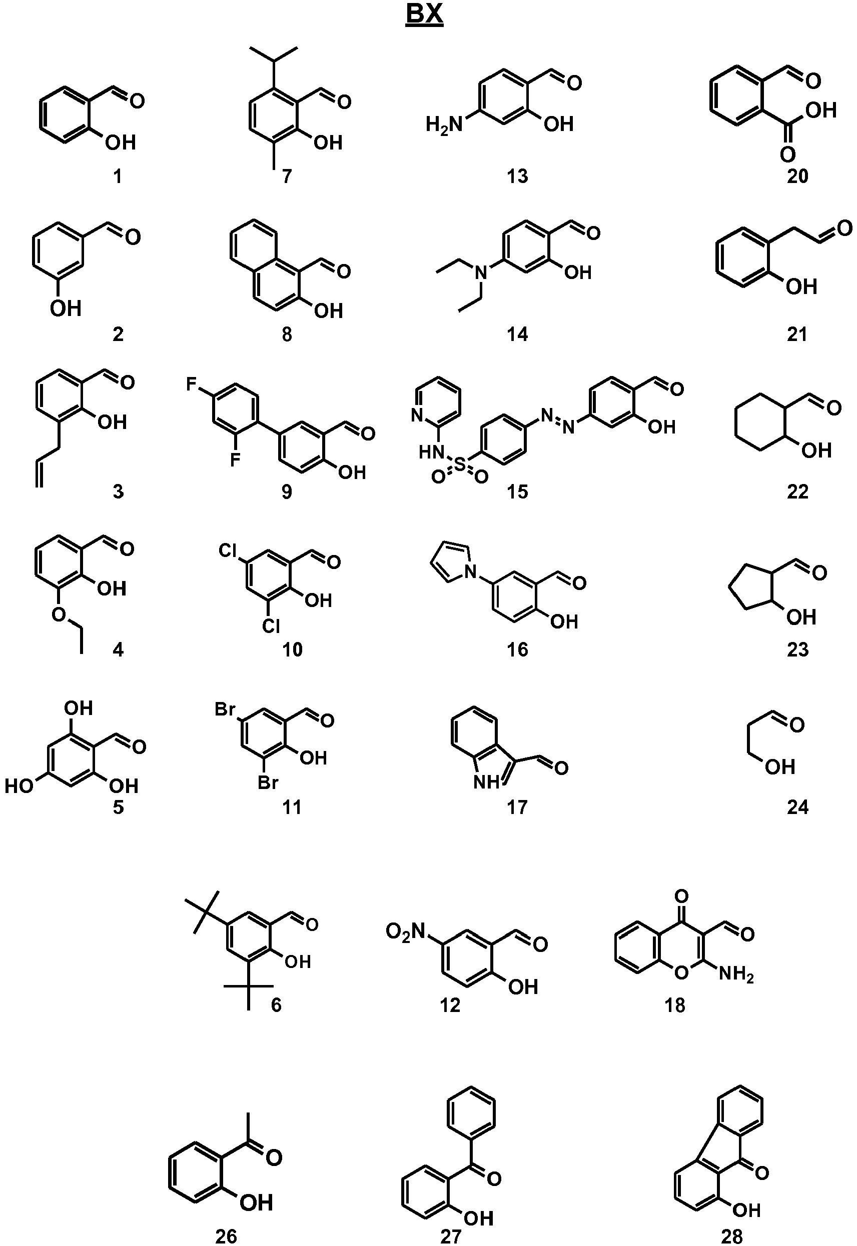

In an embodiment, such derivative compounds are further modified, e.g. to alter a property such as activity, solubility, toxicity, stability, and/or other properties in connection with pharmaceutical applications.

The derivative compounds are used as anti-cancer agents. Compounds are validated as capable of having antineoplastic activity, apoptosis regulation, and/or procaspase-3 activation. For example, primary isolates of freshly removed colon cancer are used to assess procaspase-3 levels and sensitivity of cells to test compound levels, where a test compound is PAC-1 or a derivative compound. Compounds are classified regarding a propensity to induce cell death in cancerous cells versus normal cells.

In further assessing a derivative compound, in vitro and in vivo testing is performed. Stability in connection with exposure to liver microsomes is evaluated.

### EXAMPLE 8. Activity of certain derivatives relative to PAC-1.

PAC-1 and certain derivatives were tested in the HL-60 cell line and IC50 values were determined. The results are indicated in Table 5 (where L-- and Rdesignations refer to structures shown in the AX and BX series above, respectively). Several of the PAC-1 derivatives exhibited an activity level that was generally about one order of magnitude greater than that of the PAC-1 compound.

**Table 5.**

| **NAME** | **STRUCTURE** | **IC₅₀ vs. HL-60** | **Fold better than PAC-1** |
|---|---|---|---|
| PAC-1 L01R03 | 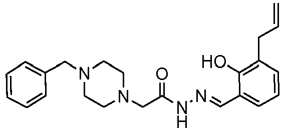 | 54.6 uM | 1.0 |
| L01R06 | 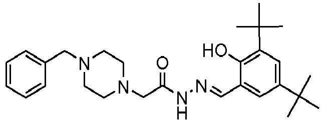 | 5.63 uM | 9.7-fold |
| L02R03 | 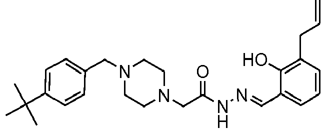 | 4.34 uM | 12.6-fold |
| L02R06 | 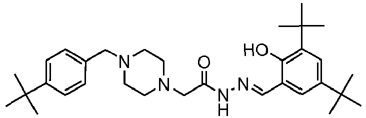 | 6.53 uM | 8.4-fold |
| L08R06 | 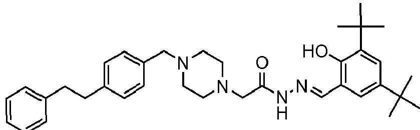 | 5.31 uM | 10.3-fold |
| L09R03 | 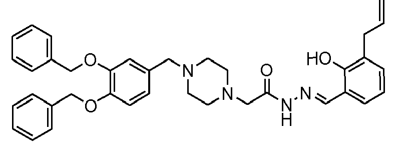 | 4.82 uM | 11.3-fold |
| L09R06 | 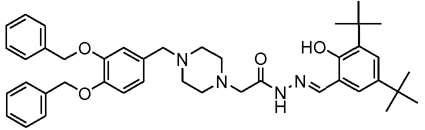 | 4.17 uM | 13.1-fold |
| L09R08 | 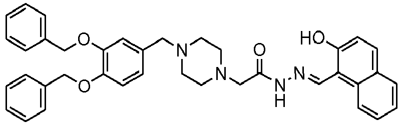 | 2.42 uM | 22.6-fold |

Where the terms "comprise", "comprises", "comprised", or "comprising" are used herein, they are to be interpreted as specifying the presence of the stated features, integers, steps, or components referred to, but not to preclude the presence or addition of one or more other feature, integer, step, component, or group thereof. Separate embodiments of the invention are also intended to be encompassed wherein the terms "comprising" or "comprise(s)" or "comprised" are optionally replaced with the terms, analogous in grammar, e.g.; "consisting/consist(s)" or "consisting essentially of/consist(s) essentially of" to thereby describe further embodiments that are not necessarily coextensive. For clarification, as used herein "comprising" is synonymous with "having," "including," "containing," or "characterized by," and is inclusive or open-ended and does not exclude additional, unrecited elements or method steps. As used herein, "consisting of" excludes any element, step, component, or ingredient not specified in the claim element. As used herein, "consisting essentially of" does not exclude materials or steps that do not materially affect the basic and novel characteristics of the claim (e.g., not affecting an active ingredient). In each instance herein any of the terms "comprising", "consisting essentially of" and "consisting of" may be replaced with either of the other two terms. The invention illustratively described herein suitably may be practiced in the absence of any element or elements, limitation or limitations which is not specifically disclosed herein.

The invention has been described with reference to various specific and preferred embodiments and techniques. It will be appreciated by one of ordinary skill in the art that compositions, methods, devices, device elements, materials, optional features, procedures and techniques other than those specifically described herein can be applied to the practice of the invention as broadly disclosed herein without resort to undue experimentation. All art-known functional equivalents of compositions, methods, devices, device elements, materials, procedures and techniques described herein; and portions thereof; are intended to be encompassed by this invention. Whenever a range is disclosed, all subranges and individual values are intended to be encompassed. This invention is not to be limited by the embodiments disclosed, including any shown in the drawings or exemplified in the specification, which are given by way of example or illustration and not of limitation. The scope of the invention shall be limited only by the claims.

### REFERENCES

U.S. Provisional Patent Application No. 60/516556 by Hergenrother et al., filed October 30, 2003; U.S. Provisional Patent Application No. 60/603246 by Hergenrother et al., filed August 20, 2004; U.S. 10/976,186 by Hergenrother et al., filed October 27, 2004.

US 6,762,045 Membrane derived caspase-3, compositions comprising the same and methods of use therefore; US 6,534,267 Polynucleotides encoding activators of caspases; US 6,403,765 Truncated Apaf-1 and methods of use thereof; US 6,303,329; 6,878,743 by Choong, et al. issued April 12, 2005; US 20040077542 by Wang, Xiaodong; et al., published April 22, 2004; US 20040180828 by Shi, Yigong, published September 16, 2004.
Slee EA et al., Benzyloxycarbonyl-Val-Ala-Asp (OMe) fluoromethylketone (Z-VAD.FMK) inhibits apoptosis by blocking the processing of CPP32, Biochem J. 1996 Apr 1;315 (Pt 1):21-4.
   1. Hanahan, D. & Weinberg, R. A. The hallmarks of cancer. Cell 100, 57-70 (2000).
   2. Okada, H. & Mak, T. W. Pathways of apoptotic and non-apoptotic death in tumour cells. Nature Rev. Cancer 4, 592-603 (2004).
   3. Roy, S. et al. Maintenance of caspase-3 proenzyme dormancy by an intrinsic "safety catch" regulatory tripeptide. Proc. Natl. Acad. Sci. 98, 6132-6137 (2001).
   4. Svingen, P. A. et al. Components of the cell death machine and drug sensitivity of the National Cancer Institute Cell Line Panel. Clin. Cancer Res. 10, 6807-6820 (2004).
   5. Lowe, S. W., Cepero, E. & Evan, G. Intrinsic tumor suppression. Nature 432, 307-315 (2004).
   6. Vogelstein, B. & Kinzler, K. W. Achilles' heel of cancer. Nature 412, 865-866 (2001).
   7. Traven, A., Huang, D. C. & Lithgow, T. Protein hijacking: key proteins held captive against their will. Cancer Cell 5, 107-108 (2004).
   8. Soengas, M. S. et al. Inactivation of the apoptosis effector Apaf-1 in malignant melanoma. Nature 409, 207-211 (2001).
   9. Wajant, H. Targeting the FLICE inhibitory protein (FLIP) in cancer therapy. Mol. Interv. 3, 124-127 (2003).
   10. Denicourt, C. & Dowdy, S. F. Targeting apoptotic pathways in cancer cells. Science 305, 1411-1413 (2004).
   11. Vassilev, L. T. et al. In vivo activation of the p53 pathway by small-molecule antagonists of MDM2. Science 303, 844-848 (2004).
   12. Degterev, A. et al. Identification of small-molecule inhibitors of interaction between the BH3 domain and Bcl-XL. Nature Cell Biol. 3, 173-182 (2001).
   13. Becattini, B. et al. Rational design and real time, in-cell detection of the proapoptotic activity of a novel compound targeting Bcl-XL. Chem. Biol. 11, 389-395 (2004).
   14. Wang, J.-L. et al. Structure-based discovery of an organic compound that binds Bcl-2 protein and induces apoptosis of tumor cells. Proc. Natl. Acad. Sci. 97, 7124-7129 (2000).
   15. Li, L. et al. A small molecule Smac mimic potentiates TRAIL- and TNFa-mediated cell death. Science 305, 1471-1474 (2004).
   16. Nguyen, J. T. & Wells, J. A. Direct activation of the apoptosis machinery as a mechanism to target cancer cells. Proc. Natl. Acad. Sci. U.S.A. 100, 7533-7538 (2003).
   17. Jiang, X. et al. Distincitive roles of PHAP proteins and prothymosin-α in a death regulatory pathway. Science 299, 223-226 (2003).
   18. Boatright, K. M. & Salvesen, G. S. Mechanisms of caspase activation. Curr. Opin. Cell. Biol. 15, 725-731 (2003).
   19. Nakagawara, A. et al. High levels of expression and nuclear localization of interleukin-1 β converting enzyme (ICE) and CPP32 in favorable human neuroblastomas. Cancer Res. 57, 4578-4584 (1997).
   20. Izban, K. F. et al. Characterization of the interleukin-1β-converting enzyme/Ced-3-family protease, caspase-3/CPP32, in Hodgkin's disease. Am. J. Pathol. 154, 1439-1447 (1999).
   21. Persad, R. et al. Overexpression of caspase-3 in hepatocellular carcinomas. Modern Patholo. 17, 861-867 (2004).
   22. Pop, C., Feeney, B., Tripathy, A. & Clark, A. C. Mutations in the procaspase-3 dimer interface affect the activity of the zymogen. Biochemistry 42, 12311-12320 (2003).
   23. Stennicke, H. R. et al. J. Biol. Chem. 273, 27084-27090 (1998).
   24. Denault, J.-B. & Salvesen, G. S. Human caspase-7 activity and regulation by its N-terminal peptide. J. Biol. Chem. 278, 34042-24050 (2003).
   25. Putt, K. S., Beilman, G. J. & Hergenrother, P. J. Direct quantitation of Poly(ADP-ribose) polymerase (PARP) activity as a means to distinguish necrotic and apoptotic death in cell and tissue samples. ChemBioChem 6, 53-55 (2005).
   26. Liang, Y., Nylander, K. D., Yan, C. & Schor, N. F. Role of caspase 3-dependent Bcl-2 cleavage in potentiation of apoptosis by Bcl-2. Mol. Pharmacol. 61, 142-149 (2002).
   27. Fujita, N., Nagahshi, A., Nagashima, K., Rokudai, S. & Tsuruo, T. Acceleration of apoptotic cell death after the cleavage of Bcl-XL protein by caspase-3-like proteases. Oncogene 17, 1295-1304 (1998).
   28. Earnshaw, W. C., Martins, L. M. & Kaufmann, S. H. Mammalian caspases: structure, activation, substrates, and functions during apoptosis. Annu. Rev. Biochem. 68, 383-424 (1999).
   29. Koty, P. P., Zhang, H. & Levitt, M. L. Antisense bcl-2 treatment increases programmed cell death in non-small cell lung cancer cell lines. Lung Cancer 23, 115-127 (1999).
National Center for Biotechnology Information (NCBI) Database of the National Library of Medicine / National Institutes of Health (NIH) website: http://www.ncbi.nlm.nih.gov/ using the Gene database to search for CASP3 (caspase 3, apoptosis-related cysteine protease [Homo sapiens] GeneID: 836 Locus tag: HGNC:1504; MIM: 600636 updated 15-May-2005. Other Aliases: HGNC:1504, APOPAIN, CPP32, CPP32B, SCA-1; Other Designations: Human procaspase3 coding sequence; PARP cleavage protease; SREBP cleavage activity 1; Yama; caspase 3; cysteine protease CPP32).
Hergenrother PJ. Obtaining and screening compound collections: a user's guide and a call to chemists. Curr Opin Chem Biol. 2006
Silverman SK, Hergenrother PJ. Combinatorial chemistry and molecular diversity Tools for molecular diversification and their applications in chemical biology. Curr Opin Chem Biol. 2006.
Goode DR, Sharma AK, Hergenrother PJ. Using peptidic inhibitors to systematically probe the S1' site of caspase-3 and caspase-7. Org Lett. 2005 Aug 4;7(16):3529-32. PMID: 16048334
Dothager RS, Putt KS, Allen BJ, Leslie BJ, Nesterenko V, Hergenrother PJ. Synthesis and identification of small molecules that potently induce apoptosis in melanoma cells through G1 cell cycle arrest. J Am Chem Soc. 2005 Jun 22;127(24):8686-96. PMID: 15954774
Putt KS, Hergenrother PJ. A nonradiometric, high-throughput assay for poly(ADP-ribose) glycohydrolase (PARG): application to inhibitor identification and evaluation. Anal Biochem. 2004 Oct 15;333(2):256-64. PMID: 15450800
Putt KS, Hergenrother PJ. An enzymatic assay for poly(ADP-ribose) polymerase-1 (PARP-1) via the chemical quantitation of NAD(+): application to the high-throughput screening of small molecules as potential inhibitors. Anal Biochem. 2004 Mar 1;326(1):78-86. PMID: 14769338
Nesterenko V, Putt KS, Hergenrother PJ. Identification from a combinatorial library of a small molecule that selectively induces apoptosis in cancer cells. J Am Chem Soc. 2003 Dec 3;125(48):14672-3. PMID: 14640619

## Claims

1. A compound of formula ZZ: 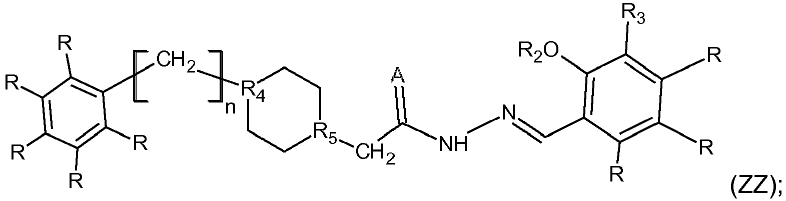 wherein n = 1 or 2; R, independently of other R, is hydrogen, halogen, allyl, or alkyl group having from 1 to 6 carbon atoms; R₂ = hydrogen, alkyl group having from 1 to 6 carbon atoms, ester; R₃ = hydrogen, halogen, alkyl, haloalkyl, allyl, alkenyl, or haloalkenyl; R₄ and R₅ are N; or R₄=N and R₅=C; or R₄ and R₅ = C; and A = oxygen or sulfur;
for use in the treatment of cancer, by administering to a patient in need thereof an effective amount of said compound.

2. A compound for use according to claim 1, wherein said compound is PAC-1:

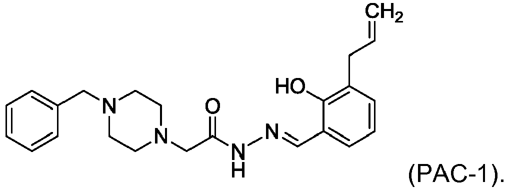

3. A pharmaceutical composition for use in treating cancer, comprising a compound according to claim 1 and a pharmaceutically acceptable carrier.

4. A pharmaceutical composition for use according to claim 3, wherein said pharmaceutical composition is for oral administration.

5. A compound for use according to claim 1, capable of modifying a procaspase-3 molecule in a cancer cell so as to selectively induce apoptosis in said cancer cell.

6. A compound for use according to claim 5, wherein said compound is PAC-1,

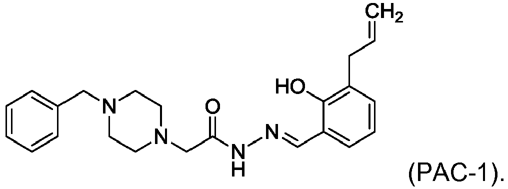

7. A compound for use according to claim 5, wherein the compound is Structure 5,

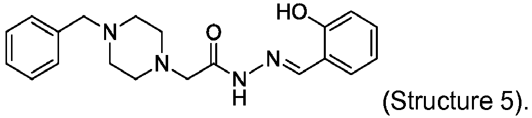

8. A compound for use according to claim 5, further comprising the step of assessing a procaspase-3 or caspase-3 parameter in a cancer cell; wherein said parameter is one or more of a semi-quantitative or quantitative amount, a functional amount, and an activity level of said procaspase-3 or caspase-3.

9. A method of direct *in vitro* screening for a compound capable of modifying a procaspase-3 molecule, comprising: (a) providing a test compound; (b) providing a purified procaspase-3; (c) exposing the test compound to the purified procaspase-3; (d) measuring a procaspase-3 activity following exposure to the test compound; (e) identifying a modifying compound by comparing a test activity upon the exposure to the test compound with an unmodified activity in the absence of exposure to the test compound; thereby screening for a compound capable of modifying a procaspase-3 molecule, the method further comprising comparing said modified activity or said unmodified activity with a reference activity; wherein said reference activity is due to exposure to a compound according to claim 1.

10. A method of in cellular screening for a compound capable of modifying a procaspase-3 molecule, comprising: (a) providing a test compound; (b) providing a cell, wherein the cell putatively expresses procaspase-3; (c) exposing the cell to the test compound; (d) measuring a cell parameter following exposure to the test compound; wherein said cell parameter comprises one or more of cell viability and apoptotic indicator; (e) identifying a modifying compound by comparing a tested cell parameter upon the exposure to the test compound with an unmodified cell parameter in the absence of exposure to the test compound; thereby screening for a compound capable of modifying a procaspase-3 molecule, further comprising comparing said modified activity or said unmodified activity with a reference activity; wherein said reference activity is due to exposure to a compound according to claim 1.

11. A compound for use according to claim 1, wherein the compound is a procaspase activator compound and the use comprises (a) identifying a potential susceptibility to treatment of a cancer cell with a procaspase activator compound; and (b) exposing said cancer cell to an effective amount of the procaspase activator compound.

12. A compound for use according to claim 11, wherein said procaspase activator compound is capable of activating procaspase-3, procaspase-7, or both procaspase-3 and procaspase-7.

13. A compound for use according to claim 1, wherein the compound is Structure 5:

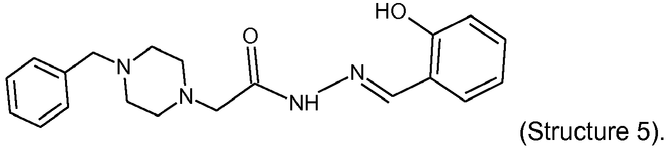

14. A compound for use according to claim 1, 2 or 13, wherein said administering is effected orally.

15. A compound having the formula ZZ2: 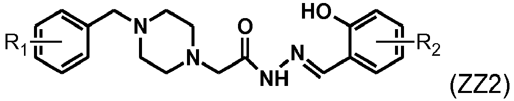 wherein R₁ and R₂ each independently is hydrogen, halogen, alkyl, allyl, haloalkyl, alkenyl, or haloalkenyl, for use in the treatment of cancer.

16. A compound for use according to claim 15, wherein R₁ and R₂ each independently is hydrogen, halogen, allyl, or alkyl group having from 1 to 6 carbon atoms.

17. A compound having the formula: 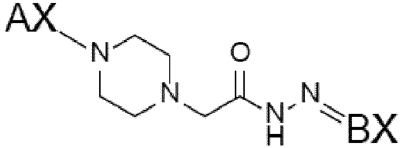 wherein AX is selected from the group consisting of: 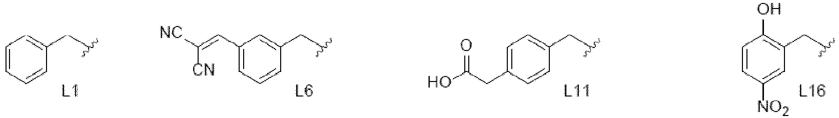 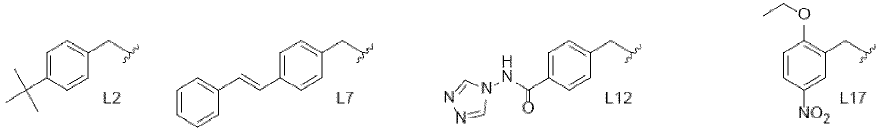 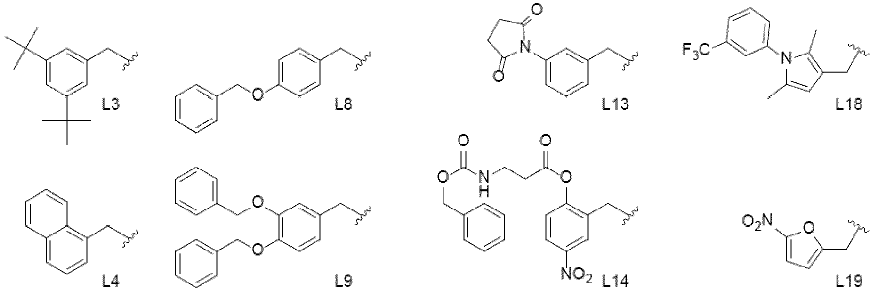 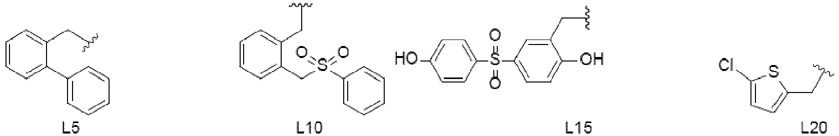 and BX is selected from the group consisting of:

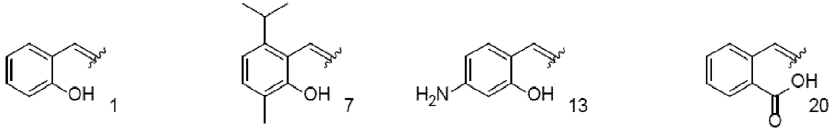

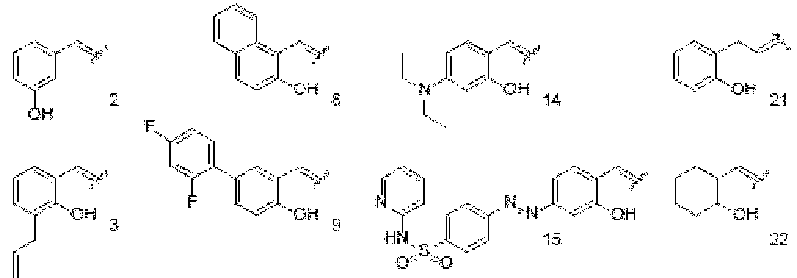

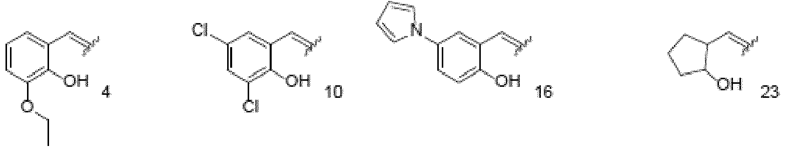

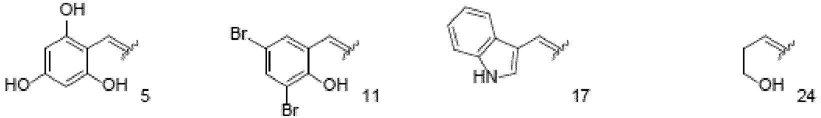

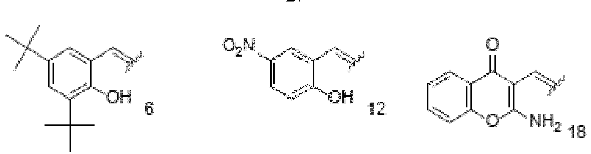

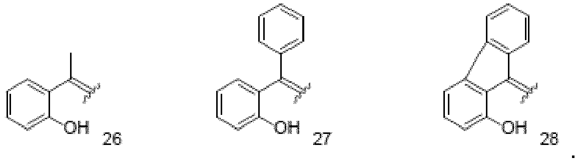

18. A compound selected from the group consisting of: formulae L01R06, L02R03, L02R06, L08R06, L09R03, L09R06, and L09R08, wherein the formulae are as follows: 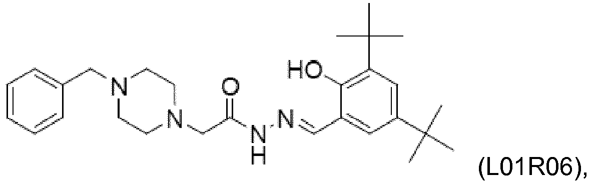 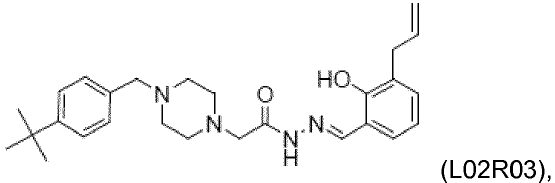 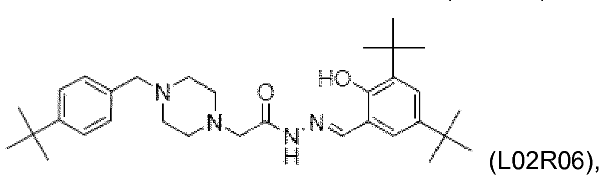 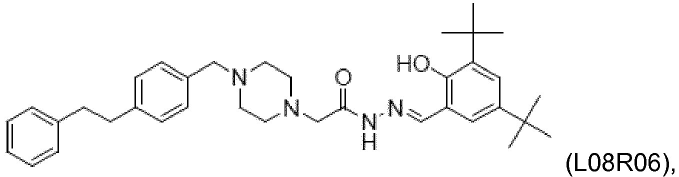 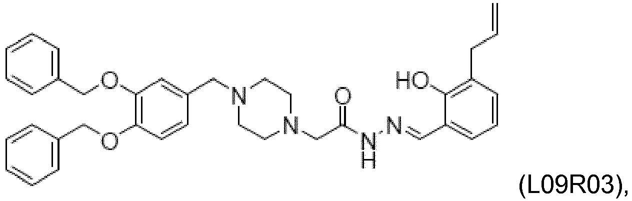 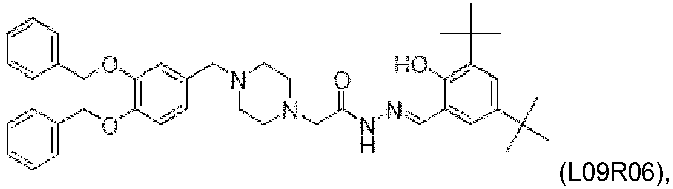 and

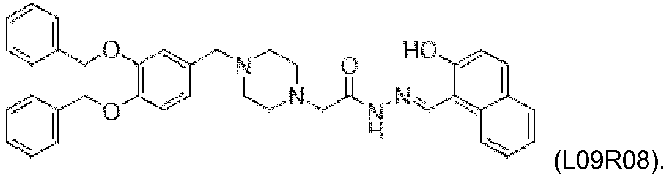

19. A compound according to claim 18, for use in treating cancer.

20. A compound for use according to claim 1, 5 or 19, wherein the cancer is lung cancer, breast cancer, renal cancer, melanoma, lymphoma, neuroblastoma or leukemia.

21. A compound capable of activating a procaspase-3 molecule for use in the treatment of cancer, by administering the compound to a cancer cell to thereby induce death in said cancer cell wherein the compound is a compound according to claim 1.

22. A compound for use according to claim 21 wherein the compound has structural formula ZZ2, 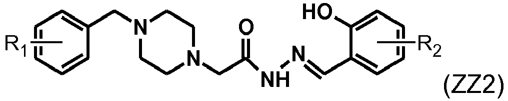 wherein R₁ and R₂ each independently is hydrogen, halogen, alkyl, allyl, haloalkyl, alkenyl, or haloalkenyl.

23. A compound for use according to claim 21 wherein the compound is selected from the group consisting of PAC-1 and Structure 5, 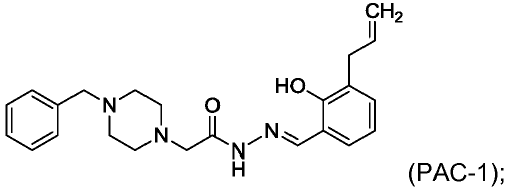 and

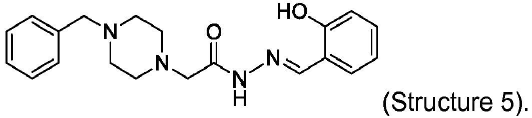

24. Use of a compound according to claim 1 in the manufacture of a medicament for the treatment of cancer.

## Patentansprüche

1. Verbindung der Formel ZZ: 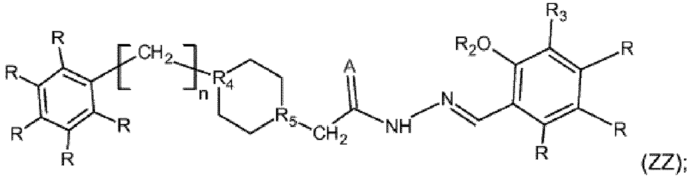 wobei n = 1 oder 2; R, unabhängig von anderen R, Wasserstoff, Halogen, Allyl oder Alkylgruppe mit 1 bis 6 Kohlenstoffatomen ist; R₂ = Wasserstoff, Alkylgruppe mit 1 bis 6 Kohlenstoffatomen, Ester; R₃ = Wasserstoff, Halogen, Alkyl, Halogenalkyl, Allyl, Alkenyl oder Halogenalkenyl; R₄ und R₅ N sind; oder R₄ = N und R₅ = C; oder R₄ und R₅ = C; und A = Sauerstoff oder Schwefel;
zur Verwendung in der Behandlung von Krebs, durch Verabreichen, an einen Patienten mit Bedarf daran, einer wirksamen Menge der Verbindung.

2. Verbindung zur Verwendung nach Anspruch 1, wobei die Verbindung PAC-1 ist:

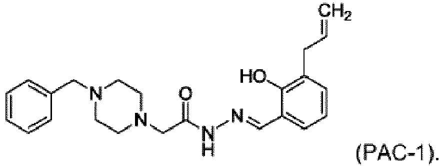

3. Pharmazeutische Zusammensetzung zur Verwendung bei Behandlung von Krebs, umfassend eine Verbindung nach Anspruch 1 und einen pharmazeutisch verträglichen Träger.

4. Pharmazeutische Zusammensetzung zur Verwendung nach Anspruch 3, wobei die pharmazeutische Zusammensetzung zur oralen Verabreichung ist.

5. Verbindung zur Verwendung nach Anspruch 1, mit der Fähigkeit zum Modifizieren eines Procaspase-3-Moleküls in einer Krebszelle, um in der Krebszelle Apoptose selektiv herbeizuführen.

6. Verbindung zur Verwendung nach Anspruch 5, wobei die Verbindung PAC-1 ist,

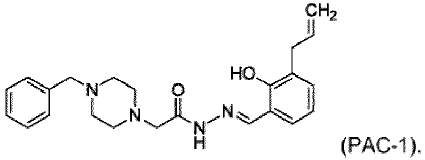

7. Verbindung zur Verwendung nach Anspruch 5, wobei die Verbindung Struktur 5 ist,

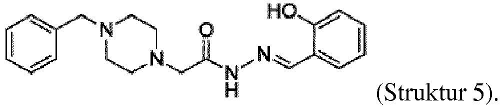

8. Verbindung zur Verwendung nach Anspruch 5, weiterhin umfassend den Schritt des Beurteilens eines Procaspase-3- oder Caspase-3-Parameters in einer Krebszelle; wobei der Parameter einer oder mehrere von einer halbquantitativen oder quantitativen Menge, einer funktionellen Menge und einem Aktivitätsniveau der Procaspase-3 oder Caspase-3 ist.

9. Verfahren zum direkten *In*-*vitro*-Screening auf eine Verbindung mit der Fähigkeit zum Modifizieren eines Procaspase-3-Moleküls, umfassend: (a) Bereitstellen einer Testverbindung; (b) Bereitstellen einer gereinigten Procaspase-3; (c) Aussetzen der Testverbindung gegenüber der gereinigten Procaspase-3; (d) Messen einer Procaspase-3-Aktivität im Anschluss an die Aussetzung gegenüber der Testverbindung; (e) Identifizieren einer modifizierenden Verbindung durch Vergleichen einer Testaktivität bei der Aussetzung gegenüber der Testverbindung mit einer unmodifizierten Aktivität bei Nichtvorhandensein einer Aussetzung gegenüber der Testverbindung; dadurch Screening auf eine Verbindung mit der Fähigkeit zum Modifizieren eines Procaspase-3-Moleküls, wobei das Verfahren weiterhin Vergleichen der modifizierten Aktivität oder der unmodifizierten Aktivität mit einer Referenzaktivität umfasst; wobei die Referenzaktivität auf Aussetzung gegenüber einer Verbindung nach Anspruch 1 zurückzuführen ist.

10. Verfahren zum zellulären Screening auf eine Verbindung mit der Fähigkeit zum Modifizieren eines Procaspase-3-Moleküls, umfassend: (a) Bereitstellen einer Testverbindung; (b) Bereitstellen einer Zelle, wobei die Zelle Procaspase-3 vermutlich exprimiert; (c) Aussetzen der Zelle gegenüber der Testverbindung; (d) Messen eines Zellparameters im Anschluss an die Aussetzung gegenüber der Testverbindung; wobei der Zellparameter eines oder mehrere von Zellviabilität und apoptotischem Indikator umfasst; (e) Identifizieren einer modifizierenden Verbindung durch Vergleichen eines getesteten Zellparameters bei der Aussetzung gegenüber der Testverbindung mit einem unmodifizierten Zellparameter bei Nichtvorhandensein einer Aussetzung gegenüber der Testverbindung; dadurch Screening auf eine Verbindung mit der Fähigkeit zum Modifizieren eines Procaspase-3-Moleküls, weiterhin umfassend Vergleichen der modifizierten Aktivität oder der unmodifizierten Aktivität mit einer Referenzaktivität; wobei die Referenzaktivität auf Aussetzung gegenüber einer Verbindung nach Anspruch 1 zurückzuführen ist.

11. Verbindung zur Verwendung nach Anspruch 1, wobei die Verbindung eine Procaspase-Aktivator-Verbindung ist und die Verwendung (a) Identifizieren einer potenziellen Empfänglichkeit für die Behandlung einer Krebszelle mit einer Procaspase-Aktivator-Verbindung; und (b) Aussetzen der Krebszelle gegenüber einer wirksamen Menge der Procaspase-Aktivator-Verbindung umfasst.

12. Verbindung zur Verwendung nach Anspruch 11, wobei die Procaspase-Aktivator-Verbindung zum Aktivieren von Procaspase-3, Procaspase-7, oder von sowohl Procaspase-3 als auch Procaspase-7 in der Lage ist.

13. Verbindung zur Verwendung nach Anspruch 1, wobei die Verbindung Struktur 5 ist:

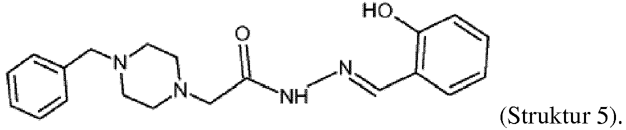

14. Verbindung zur Verwendung nach Anspruch 1, 2 oder 13, wobei das Verabreichen oral erfolgt.

15. Verbindung mit der Formel ZZ2: 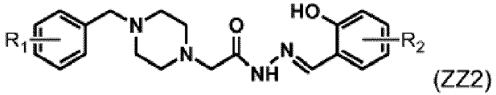 wobei R₁ und R₂ jeweils unabhängig Wasserstoff, Halogen, Alkyl, Allyl, Halogenalkyl, Alkenyl oder Halogenalkenyl sind, zur Verwendung in der Behandlung von Krebs.

16. Verbindung zur Verwendung nach Anspruch 15, wobei R₁ und R₂ jeweils unabhängig Wasserstoff, Halogen, Allyl oder Alkylgruppe mit 1 bis 6 Kohlenstoffatomen sind.

17. Verbindung mit der Formel: 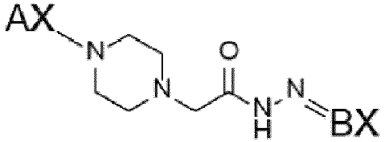 wobei AX aus der Gruppe ausgewählt ist, die besteht aus: 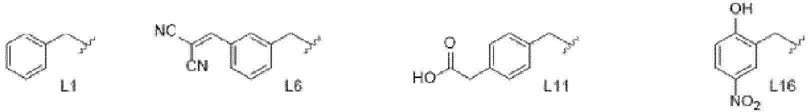 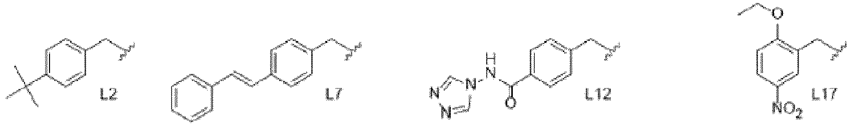 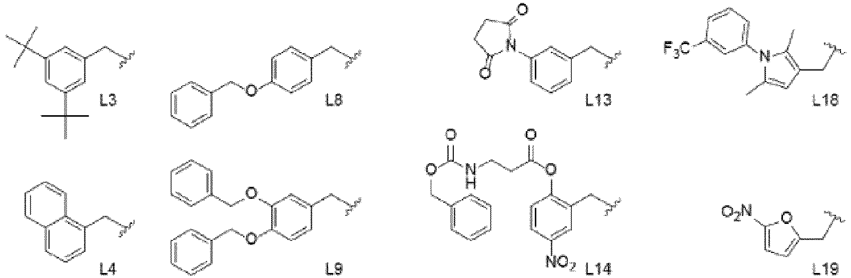 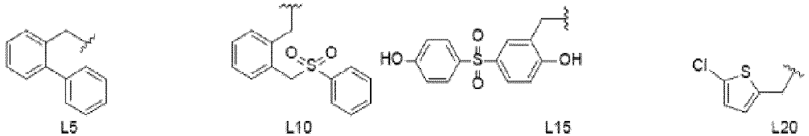 und BX aus der Gruppe ausgewählt ist, die besteht aus:

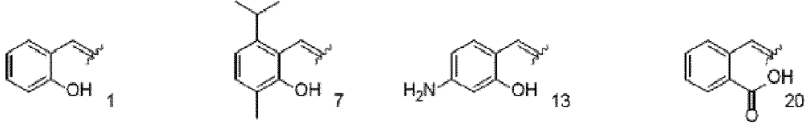

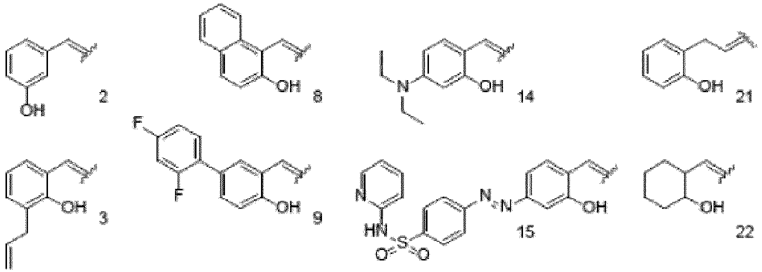

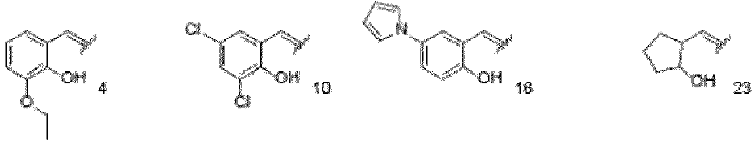

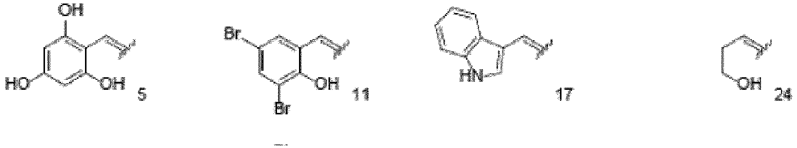

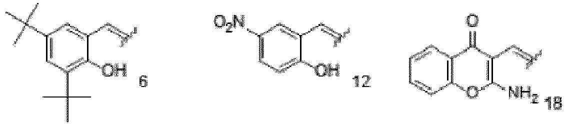

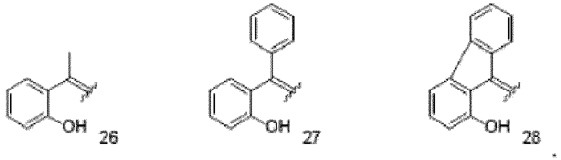

18. Verbindung, ausgewählt aus der Gruppe, bestehend aus: Formeln L01R06, L02R03, L02R06, L08R06, L09R03, L09R06 und L09R08, wobei die Formeln wie folgt sind: 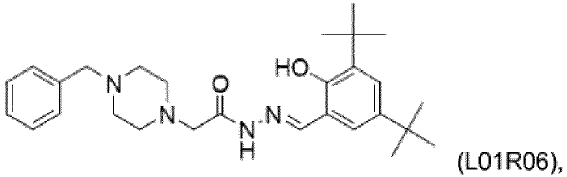 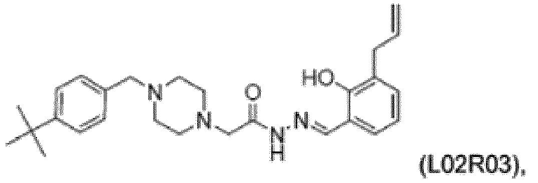 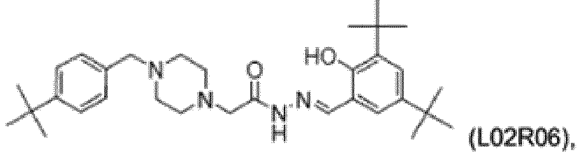 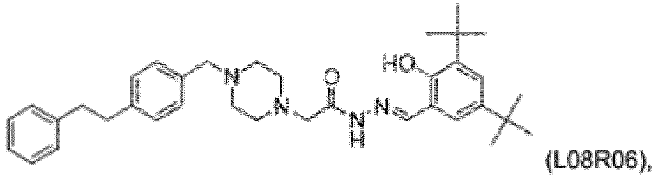 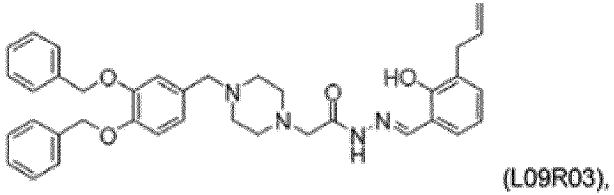 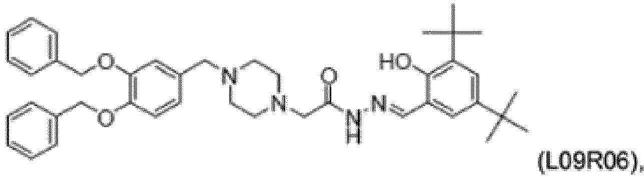 und

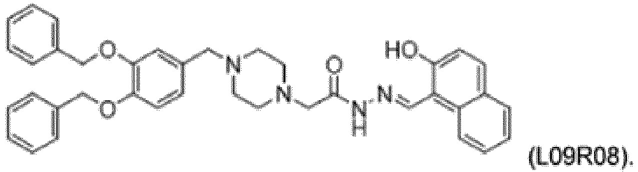

19. Verbindung nach Anspruch 18, zur Verwendung bei Behandlung von Krebs.

20. Verbindung zur Verwendung nach Anspruch 1, 5 oder 19, wobei der Krebs Lungenkrebs, Brustkrebs, Nierenkrebs, Melanom, Lymphom, Neuroblastom oder Leukämie ist.

21. Verbindung mit der Fähigkeit zum Aktivieren eines Procaspase-3-Moleküls zur Verwendung in der Behandlung von Krebs, durch Verabreichen der Verbindung an eine Krebszelle, um dadurch in der Krebszelle den Tod herbeizuführen, wobei die Verbindung eine Verbindung gemäß Anspruch 1 ist.

22. Verbindung zur Verwendung nach Anspruch 21, wobei die Verbindung Strukturformel ZZ2 hat, 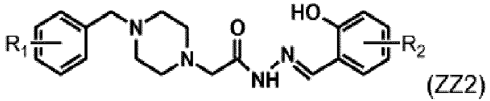 wobei R₁ und R₂ jeweils unabhängig Wasserstoff, Halogen, Alkyl, Allyl, Halogenalkyl, Alkenyl oder Halogenalkenyl sind.

23. Verbindung zur Verwendung nach Anspruch 21, wobei die Verbindung aus der Gruppe ausgewählt ist, die aus PAC-1 und Struktur 5 besteht, 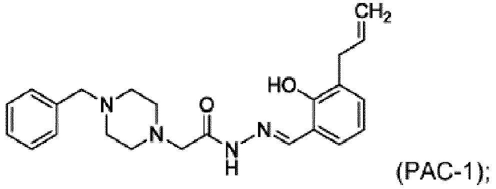 und

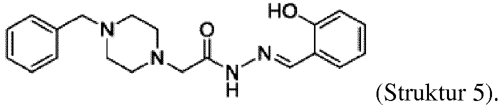

24. Verwendung einer Verbindung nach Anspruch 1 in der Herstellung eines Medikaments für die Behandlung von Krebs.

## Revendications

1. Composé de la formule ZZ: 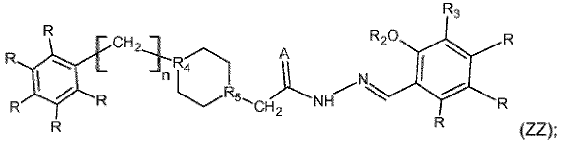 dans lequel n = 1 ou 2; R, indépendamment d'un autre R, est un hydrogène, un halogène, un allyle ou un groupe alkyle possédant de 1 à 6 atomes de carbone; R₂ = hydrogène, groupe alkyle possédant de 1 à 6 atomes de carbone, ester; R₃ = hydrogène, halogène, alkyle, haloalkyle, allyle, alcényle ou haloalcényle; R₄ et R₅ sont N; ou R₄ = N et R₅ = C; ou R₄ et R₅ = C; et A = oxygène ou soufre;
pour une utilisation dans le traitement d'un cancer, par l'administration à un patient en ayant besoin d'une quantité efficace dudit composé.

2. Composé pour une utilisation selon la revendication 1, où ledit composé est PAC 1:

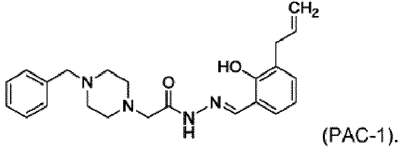

3. Composition pharmaceutique pour une utilisation dans le traitement d'un cancer, comprenant un composé selon la revendication 1 et un véhicule pharmaceutiquement acceptable.

4. Composition pharmaceutique pour une utilisation selon la revendication 3, où ladite composition pharmaceutique est pour une administration orale.

5. Composé pour une utilisation selon la revendication 1, capable de modifier une molécule de procaspase-3 dans une cellule cancéreuse de manière à induire sélectivement une apoptose dans ladite cellule cancéreuse.

6. Composé pour une utilisation selon la revendication 5, où ledit composé est PAC 1:

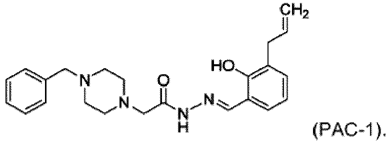

7. Composé pour une utilisation selon la revendication 5, où le composé est la Structure 5:

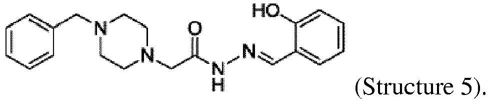

8. Composé pour une utilisation selon la revendication 5, comprenant en outre l'étape d'évaluation d'un paramètre de procaspase-3 ou de caspase-3 dans une cellule cancéreuse; où ledit paramètre est un ou plusieurs parmi une valeur semi-quantitative ou quantitative, une quantité fonctionnelle et un niveau d'activité de ladite procaspase-3 ou caspase-3.

9. Méthode de criblage *in vitro* direct d'un composé capable de modifier une molécule de procaspase-3, comprenant: (a) la fourniture d'un composé d'essai; (b) la fourniture d'une procaspase-3 purifiée; (c) l'exposition du composé d'essai à la procaspase-3 purifiée; (d) la mesure d'une activité de la procaspase-3 à la suite de l'exposition au composé d'essai; (e) l'identification d'un composé modifiant par la comparaison d'une activité d'essai lors de l'exposition au composé d'essai avec une activité non modifiée en l'absence de l'exposition au composé d'essai; criblant ainsi un composé capable de modifier une molécule de procaspase-3, la méthode comprenant en outre la comparaison de ladite activité modifiée ou de ladite activité non modifiée avec une activité de référence; dans laquelle l'activité de référence est due à une exposition à un composé selon la revendication 1.

10. Méthode de criblage dans une cellule d'un composé capable de modifier une molécule de procaspase-3, comprenant: (a) la fourniture d'un composé d'essai; (b) la fourniture d'une cellule, où la cellule exprime de façon présumée une procaspase-3; (c) l'exposition de la cellule au composé d'essai; (d) la mesure d'un paramètre de cellule à la suite de l'exposition au composé d'essai; où ledit paramètre de cellule comprend un ou plusieurs parmi la viabilité de la cellule et l'indicateur apoptotique; (e) l'identification d'un composé modifiant par la comparaison d'un paramètre de cellule testé lors de l'exposition au composé d'essai avec un paramètre de cellule non modifié en l'absence de l'exposition au composé d'essai; criblant ainsi un composé capable de modifier une molécule de procaspase-3, comprenant en outre la comparaison de ladite activité modifiée ou de ladite activité non modifiée avec une activité de référence; dans laquelle l'activité de référence est due à une exposition à un composé selon la revendication 1.

11. Composé pour une utilisation selon la revendication 1, où le composé est un composé activateur de procaspase et l'utilisation comprend (a) l'identification d'une sensibilité potentielle à un traitement d'une cellule cancéreuse avec un composé activateur de procaspase; et (b) l'exposition de ladite cellule cancéreuse à une quantité efficace du composé activateur de procaspase.

12. Composé pour une utilisation selon la revendication 11, où ledit composé activateur de procaspase est capable d'activer une procaspase-3, une procaspase-7 ou à la fois une procaspase-3 et une procaspase-7.

13. Composé pour une utilisation selon la revendication 1, où le composé est la Structure 5:

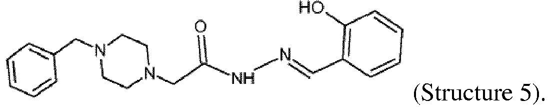

14. Composé pour une utilisation selon la revendication 1, 2 ou 13, où ladite administration est effectuée oralement.

15. Composé présentant la formule ZZ2: 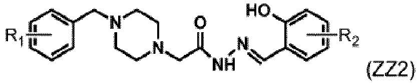 dans lequel R₁ et R₂ sont chacun indépendamment un hydrogène, un halogène, un alkyle, un allyle, un haloalkyle, un alcényle ou un haloalcényle, pour une utilisation dans le traitement d'un cancer.

16. Composé pour une utilisation selon la revendication 15, dans lequel R₁ et R₂ sont chacun indépendamment un hydrogène, un halogène, un allyle ou un groupe alkyle possédant de 1 à 6 atomes de carbone.

17. Composé présentant la formule: 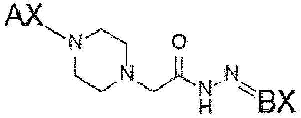 dans lequel AX est choisi dans le groupe constitué de: 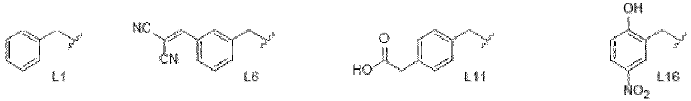 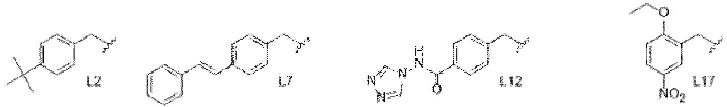 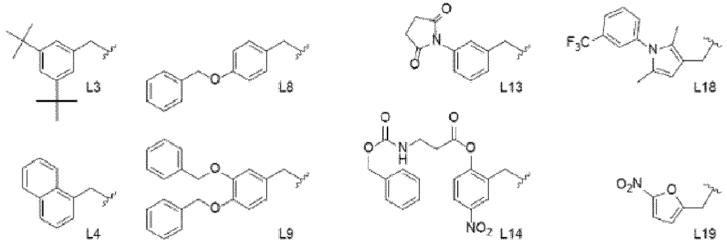 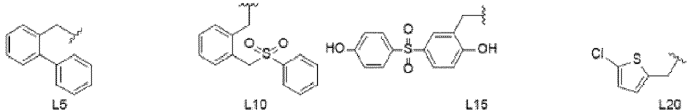 et BX est choisi dans le groupe constitué de:

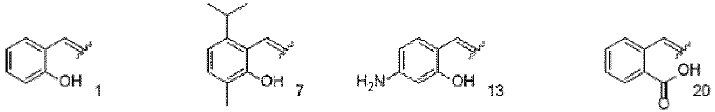

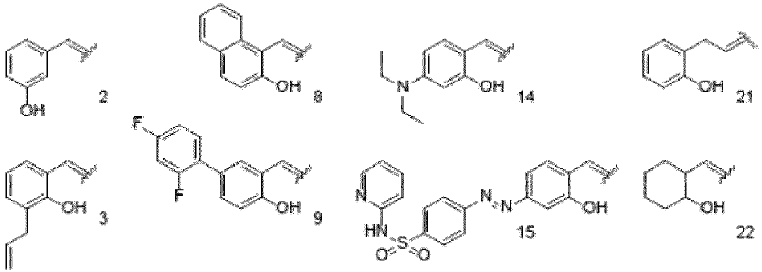

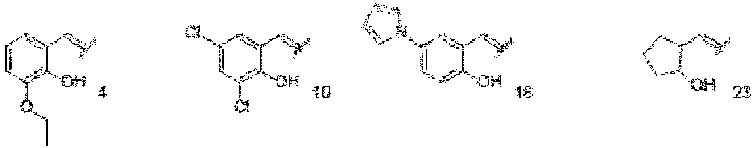

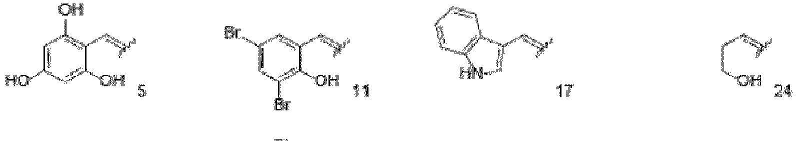

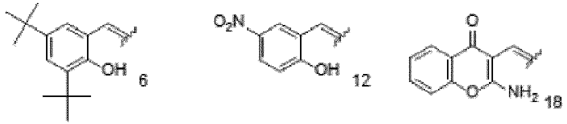

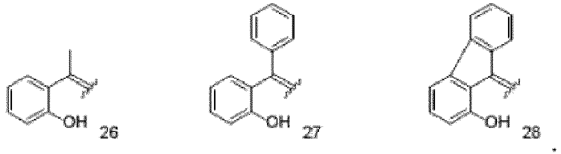

18. Composé choisi dans le groupe constitué de: formules L01R06, L02R03, L02R06, L08R06, L09R03, L09R06 et L09R08, où les formules sont comme suit:

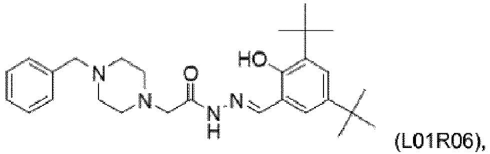

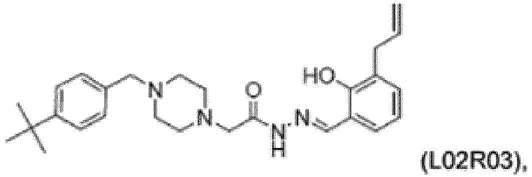

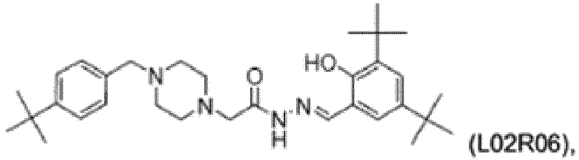

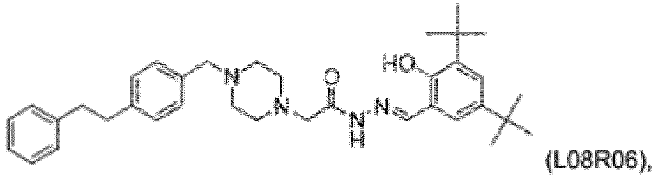

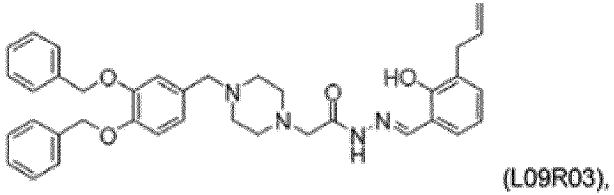

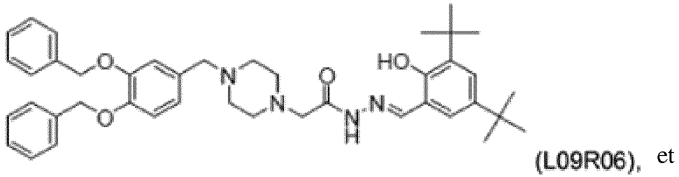

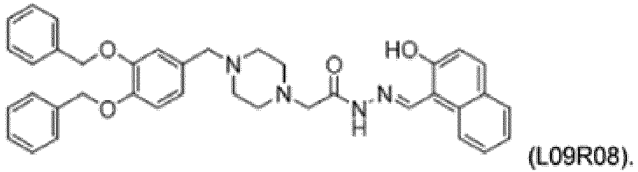

19. Composé selon la revendication 18, pour une utilisation dans le traitement d'un cancer.

20. Composé pour une utilisation selon la revendication 1, 5 ou 19, où le cancer est un cancer du poumon, un cancer du sein, un cancer rénal, un mélanome, un lymphome, un neuroblastome ou une leucémie.

21. Composé capable d'activer une molécule de procaspase-3 pour une utilisation dans le traitement d'un cancer, par l'administration du composé à une cellule cancéreuse pour induire ainsi la mort dans ladite cellule cancéreuse, où le composé est un composé selon la revendication 1.

22. Composé pour une utilisation selon la revendication 21, où le composé présente la formule structurale ZZ2: 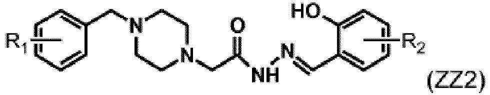 dans lequel R₁ et R₂ sont chacun indépendamment un hydrogène, un halogène, un alkyle, un allyle, un haloalkyle, un alcényle ou un haloalcényle.

23. Composé pour une utilisation selon la revendication 21, où le composé est choisi dans le groupe constitué de PAC-1 et la Structure 5:

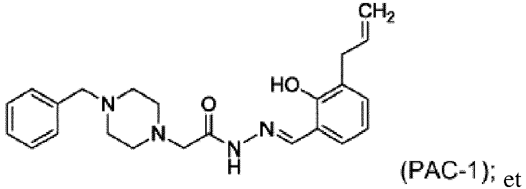

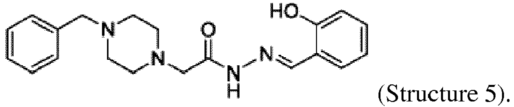

24. Utilisation d'un composé selon la revendication 1 dans la fabrication d'un médicament pour le traitement d'un cancer.
